# EUROPEAN PATENT APPLICATION

(11) **EP 2 138 482 A1**
(43) Date of publication of application: **30.12.2009**
(21) Application number: 08740535.3
(22) Date of filing: 17.04.2008
(51) Int. Cl.: C07D 217/24, A61K 31/472, A61K 31/517, A61K 31/5513, A61K 31/662, A61K 31/695, A61P 7/02, A61P 9/10, A61P 29/00, A61P 35/04, A61P 37/06, A61P 43/00, C07D 239/96, C07D 243/14, C07D 401/06, C07D 403/12, C07D 405/04, C07D 405/06, C07D 413/06, C07D 417/06

(54) **BICYCLIC HETEROCYCLIC COMPOUND**

(30) Priority: 19.04.2007 JP 2007109958
(71) Applicant: Astellas Pharma Inc., Tokyo 103-8411 (JP)
(72) Inventor: KOGA, Yuji, Tokyo 103-8411 (JP); OKUDA, Takao, Tokyo 103-8411 (JP); KAMIKUBO, Takashi, Tokyo 103-8411 (JP); KAGEYAMA, Michihito, Tokyo 103-8411 (JP); MORITOMO, Hiroyuki, Tokyo 103-8411 (JP)
(74) Representative: HOFFMANN EITLE
(86) International application number: PCT/JP2008/057465
(87) International publication number: WO 2008/133155

(57) **Abstract**

[Problem]

Provided is a compound, which exhibits a P2Y12 inhibitory action and is useful as a medical drug, particularly, as a platelet aggregation inhibitor.

[Means for solution]

The inventors have eagerly investigated P2Y12 inhibitors. As a result, the inventors have found that a bicyclic heterocyclic compound such as quinazolinedione, isoquinolone, and the like having an amino group substituted with lower alkyl, cycloalkyl, or lower alkylene-cycloalkyl at the specific position exhibits an excellent platelet aggregation inhibitory action, thereby completing the present invention. Since the compound of the invention exhibits excellent P2Y12 inhibitory action and platelet aggregation inhibitory action, it is useful as a platelet aggregation inhibitor.

## Description

### Technical Field

The present invention relates to a novel bicyclic heterocyclic compound useful as a medical drug, particularly, as a platelet aggregation inhibitor and a P2Y12 inhibitor, or a pharmaceutically acceptable salt thereof.

### Background Art

Platelets were discovered by Donne in 1842 and since then, platelets have long been regarded as one of the blood components necessary in hemostasis. At present, it is well known that platelets not only play the main role in hemostatic mechanism but also exhibit multiple functions relating to such areas as arteriosclerosis formation which attracts clinical attention; circulatory diseases including thrombotic diseases; cancer metastasis, inflammation, post-transplant rejection response, and immune response, etc.

For thrombotic diseases and ischemic diseases, in general, treatment has been carried out to restore the circulation of the blood by the use of medical agents or the application of physical methods. However, recently, it has been found that, after restoring the circulation of the blood, due to the disruption of vascular tissues including endothelial cells or the loss in the balance of fibrinolysis and clotting because of the medical agents, platelets are activated and the adhesion and cohesion of the platelets are accelerated, thereby leading to clinical problems. For example, it has been found that, when the recanalization is completed by applying a thrombolytic therapy using t-PA and the like, the fibrinolysis function and the clotting function are activated so that the balance of fibrinolysis and clotting throughout the body deteriorates. This leads to reocclusion which causes significant problems for clinical treatment (refer to Non-patent Document 1).

On the other hand, for treating diseases such as angina, cardiac infarction, and the like caused by coronary stenosis and aortic stenosis, PTCA therapy and the stent placement have rapidly become widespread and achieved a certain of progress. However, since these treatments may cause damages to the vascular tissues including endothelial cells, thereby leading to acute coronary occlusion as well as restenosis occurring during the chronic phase, problems arise. The platelets have played a critical role in various harmful thrombotic effects (such as reocclusion and the like) after carrying out a therapy for restoring the circulation of the blood. Therefore, it has been anticipated to have the efficacy of an anti-platelet agent. However, among the known anti-platelet agents, there is none which is approved that it has a sufficient effect.

As a prophylactic or therapeutic agent for these circulatory diseases, platelet aggregation inhibitors such as aspirin, cilostazol, prostaglandin I₂, prostaglandin E₁, ticlopidine, clopidogrel, dipyridamole, and the like have been used. In recent, there have been developed GPIIb/IIIa antagonists exhibiting a strong platelet aggregation inhibiting activity by inhibiting the last phase of the platelet aggregation. However, the use of the antagonists is limited as drip infusions for thrombosis during the acute phase (refer to Non-patent Document 2).

In recent, as for ticlopidine and clopidogrel used as anti-platelet agents, it has been found that the active metabolites thereof inhibit the fuction of P2Y12, which is a receptor for ADP, thereby exhibiting a platelet aggregation inhibitory action.

According to Patent Document 1, there is described that an isoquinolone derivative presented by a formula (A) exhibits a platelet aggregation inhibitory action and is useful as a platelet aggregation inhibitor. However, in the isoquinolone derivative represented by the formula (A), there is no substituent corresponding to R² in a compound of the present invention. 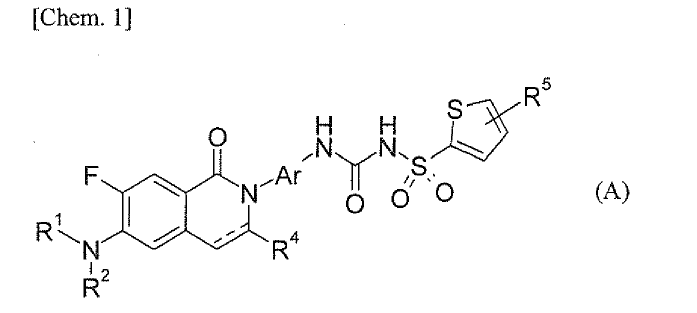 (See said official gazette for symbols in the formula)

According to Patent Document 2, there is described that a quinolone derivative presented by a formula (B) exhibits a P2Y12 inhibitory action and is useful as a platelet aggregation inhibitor. 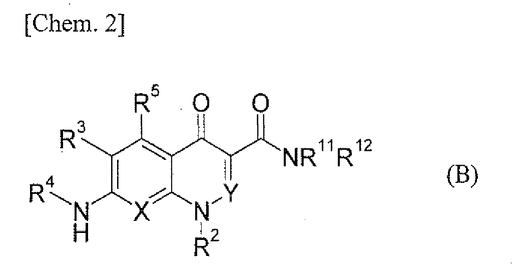 (See said official gazette for symbols in the formula)

According to Patent Document 3, there is described that a quinolone derivative presented by a formula (C) exhibits a P2Y12 inhibitory action and is useful as a platelet aggregation inhibitor. 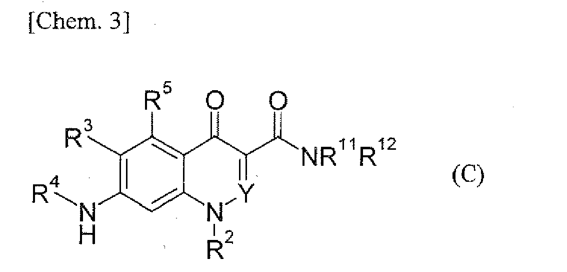 (See said official gazette for symbols in the formula)

According to Patent Document 4, there is described that a quinolone derivative presented by a formula (D) exhibits a P2Y12 inhibitory action and is useful as a platelet aggregation inhibitor. 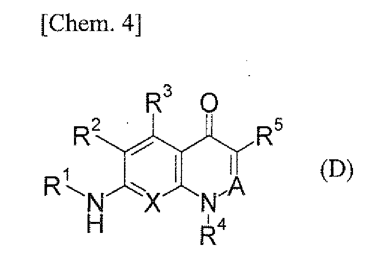 (See said official gazette for symbols in the formula)

According to Patent Document 5, there is described that the broad range of compounds shown as formulae (E-1) to (E-8) exhibit a platelet ADP receptor inhibitory action and are useful for prevention and treatment of diseases relating to cardiovascular diseases, particularly thrombosis. However, there is no specific disclosure of a compound of the invention. 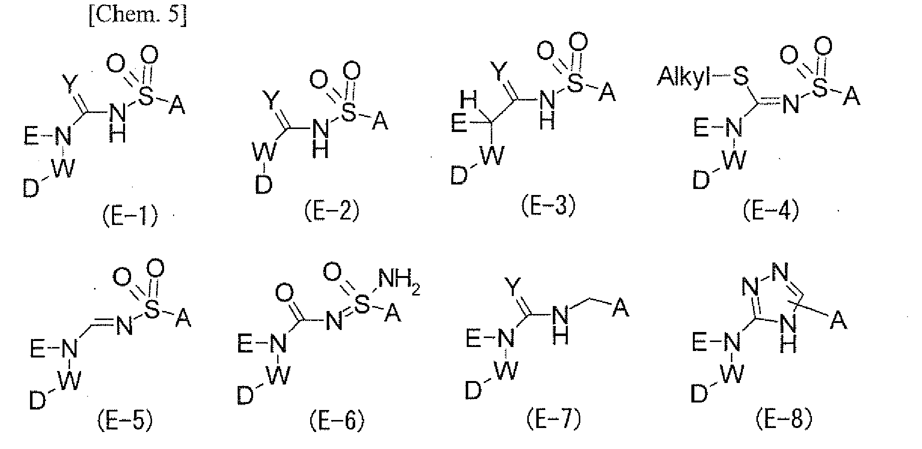 (wherein, the symbols indicate the following meanings: 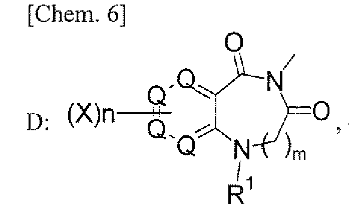 etc.
W: aryl, substituted aryl, heteroaryl, or substituted heteroaryl; as for the other symbols, refer to this publication)

According to Patent Document 6, there is described that a compound presented by a formula (F) exhibits a platelet ADP receptor inhibitory action and is useful for prevention and treatment of diseases relating to cardiovascular diseases, particularly thrombosis. However, there is no specific disclosure of a compound of the invention. 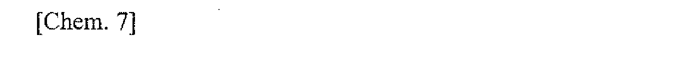 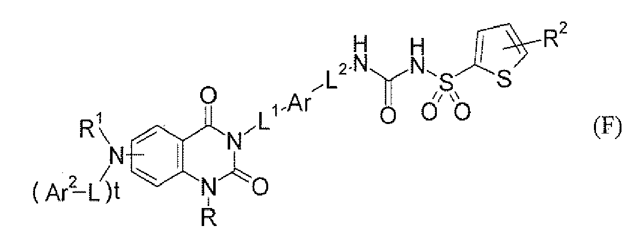 (See said official gazette for symbols in the formula)

According to Patent Document 7 published after the priority date of the present application, there is described that a compound presented by a formula (G) exhibits a platelet ADP receptor inhibitory action and is useful for prevention and treatment of diseases relating to cardiovascular diseases, particularly thrombosis. However, there is no substituent corresponding to R² in a compound of the invention. 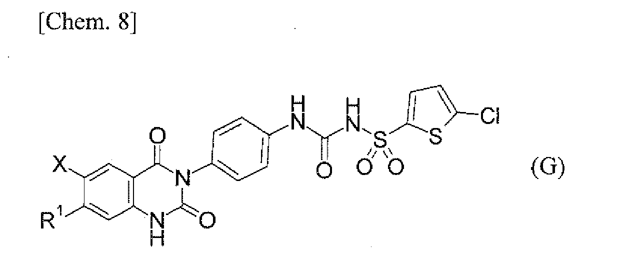 (See said official gazette for symbols in the formula)

According to Patent Document 8, there is described that a compound presented by a formula (H) exhibits an aldose reductase inhibitory action and a platelet aggregation inhibitory action. However, there is no specific disclosure of a compound of the invention. 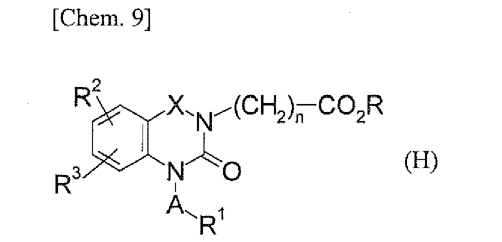 (See said official gazette for symbols in the formula)

According to Patent Document 9, there is described that a compound presented by a formula (J) exhibits a phosphatase inhibitory action and is useful for autoimmune diseases, proliferative diseases, and the like. However, there is no specific disclosure of a compound of the invention. In addition, there are no descriptions of a P2Y12 inhibitory action and a platelet aggregation inhibitory action. 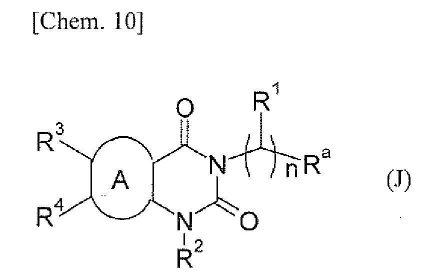 (See said official gazette for symbols in the formula)

According to Patent Document 10, there is described that a compound presented by a formula (K) exhibits a α4 integrin inhibitory action and is useful for inflammatory diseases, cardiovascular diseases, and the like. However, there are no descriptions of a P2Y12 inhibitory action and a platelet aggregation inhibitory action. 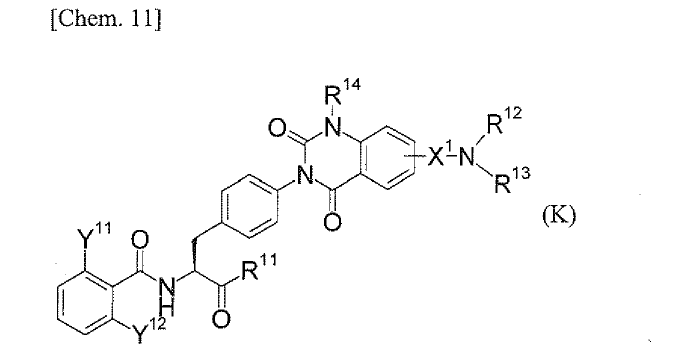 (See said official gazette for symbols in the formula)

According to Patent Document 11, there is described that a compound presented by a formula (L) exhibits a RNA polymerase inhibitory action and is useful for preventing or treating HCV infections. However, only one of the portions of the compound (H) corresponding to R³ and R⁴-NH- of the present application is substituted. In addition, there are no descriptions of a P2Y12 inhibitory action and a platelet aggregation inhibitory action.

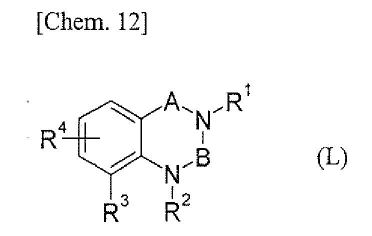

[Non-patent Document 1] 'Journal of the American College of Cardiology', 1988, Vol. 12, p. 616-623
[Non-patent Document 2] 'The Clinics' 2003, Vol. 52, p. 1516-1521
[Patent Document 1] Pamphlet of International Publication No. WO 2005/035520
[Patent Document 2] Pamphlet of International Publication No. WO 2005/009971
[Patent Document 3] Pamphlet of International Publication No. WO 2006/077851
[Patent Document 4] Pamphlet of International Publication No. WO 2007/105751
[Patent Document 5] Pamphlet of International Publication No. WO 2003/011872
[Patent Document 6] Pamphlet of International Publication No. WO 2005/032488
[Patent Document 7] Pamphlet of International Publication No. WO 2007/056219
[Patent Document 8] JP-A-03-181469
[Patent Document 9] Pamphlet of International Publication No. WO 2004/060878
[Patent Document 10] Pamphlet of International Publication No. WO 2005/061466
[Patent Document 11] Pamphlet of International Publication No. WO 2007/028789

### Disclosure of the invention

### Problem that the Invention is to solve

The present invention is to provide a compound, which exhibits a P2Y12 inhibitory action and is useful as a medical drug, particularly, as a platelet aggregation inhibitor. Means for Solving the Problem

The present inventors have made extensive studies to find an excellent platelet aggregation inhibitor. As a result, the inventors have found that a bicyclic heterocyclic compound such as quinazolinedione, isoquinolone, and the like having an amino group substituted with lower alkyl, cycloalkyl, or lower alkylene-cycloalkyl at the specific position exhibits an excellent platelet aggregation inhibitory action, thereby completing the present invention.
Thus, the present invention relates to a bicyclic heterocyclic compound presented by the formula (I) or a pharmaceutically acceptable salt thereof.
A bicyclic heterocyclic compound presented by the formula (I) or a pharmaceutically acceptable salt thereof: 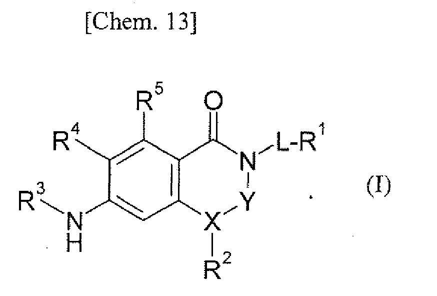 (wherein, symbols indicate the following meanings:
X: C(R⁶) or N;
Y: (i) CH(R⁷) when X is C(R⁶), and (ii) C(O) or *-C(O)-CH₂- when X is N, wherein * represents a bond to X;
R⁶ and R⁷ indicate H, or
R⁶ and R⁷ may form a bond together;
R¹: lower alkyl, halogeno-lower alkyl, lower alkylene-R¹⁰, lower alkenylene-R¹⁰, aryl, or a heterocyclic group, in which lower alkylene, lower alkenylene, aryl, and the heterocyclic group may be substituted;
L: a single bond, -O-, -N(R¹¹)-, -N(R¹¹)C(O)-*, or -N(R¹¹)C(O)O-*, wherein * represents a bond to R¹;
R¹⁰: -OR¹¹, -CN, -C(O)R¹¹, -CO₂R⁰, -CO₂-lowe alkylene-aryl, -C(O)N(R¹¹)₂, -C(O)N(R⁰)-S(O)₂-R¹¹, -C(O)N(R⁰)-OR⁰, -C(O)N(R⁰)O-heterocyclic group, -C(O)N(R⁰)N(R⁰)₂, -N(R¹¹)₂, -N(R¹¹)C(O)R¹¹, -N(R¹¹)-CO₂R⁰, -N(R⁰)C(O)CO₂R⁰, -N(R¹¹)-S(O)₂-R¹¹, -N(R¹¹)C(S)S-R⁰, -P(O)(OR⁰)₂, aryl, or a heterocyclic group, in which aryl and the heterocyclic group may be substituted;
R⁰: the same with or different from each other, and -H or lower alkyl;
R¹¹: the same with or different from each other, and -H, lower alkyl, halogeno-lower alkyl, lower alkenyl, cycloalkyl, cycloalkenyl, aryl, heterocyclic group, lower alkylene-OR⁰, lower alkylene-CO₂R⁰, lower alkylene-CO₂-lower alkylene-aryl, lower alkylene-aryl, lower alkylene-heterocyclic group, lower alkylene-OC(O)R⁰, lower alkylene-P(O)(OR⁰)₂, lower alkylene-O-lower alkylene-aryl, lower alkenylene-OR⁰, lower alkenylene-CO₂R⁰, lower alkenylene-aryl, lower alkenylene-heterocyclic group, or lower alkenylene-P(O)(OR⁰)₂, in which lower alkylene, lower alkenylene, cycloalkyl, cycloalkenyl, aryl, and heterocyclic group may be substituted;
R²: lower alkyl, cycloalkyl, cycloalkenyl, or a heterocyclic group;
R³: lower alkyl, cycloalkyl, or lower alkylene-cycloalkyl;
R⁴_{:} -H or halogen;
R⁵: -H, halogen, -OR⁰, -O-halogeno-lower alkyl, or -O-lower alkylene-aryl, wherein, N-(2,6-dichlorobenzoyl)-4-[7-(ethylamino)-1-methyl-2,4-dioxo-1,4-dihydroquinazolin-3(2H)-yl]-L-phenylalanine and 3-(3-chlorophenyl)-7-(isobutylamino)-1-methylquinazoline-2,4(1H,3H)-dione are excluded) (the same shall apply hereinafter).

The present invention also relates to a pharmaceutical composition comprising as an active ingredient a bicyclic heterocyclic compound presented by the formula (I) or a pharmaceutically acceptable salt thereof, especially, a P2Y12 inhibitor and/or a platelet aggregation inhibitor.
Further, the present invention relates to use of a bicyclic heterocyclic compound presented by the formula (I) or a pharmaceutically acceptable salt thereof for the manufacture of a P2Y12 inhibitor and/or a platelet aggregation inhibitor and a method for treating cardiovascular diseases which closely relate to thrombogenesis by platelet aggregation.
That is:
(1) A pharmaceutical composition comprising the compound of the formula (I) or a pharmaceutically acceptable salt thereof, and a pharmaceutically acceptable carrier.
(2) The pharmaceutical composition according to (1), which is a platelet aggregation inhibitor.
(3) The pharmaceutical composition according to (1), which is a P2Y12 inhibitor.
(4) Use of the compound described in the general formula (I) or a pharmaceutically acceptable salt thereof for the manufacture of a platelet aggregation inhibitor or a P2Y12 inhibitor.

### Effect of the Invention

Since a compound of the present invention exhibits an excellent P2Y12 inhibitory action, it is useful as a medical drug, particularly, as a platelet aggregation inhibitor.

### Best Mode for Carrying Out the Invention

Hereinbelow, the present invention will be described in detail.
Preferred example of 'lower alkyl' in the present Specification includes linear or branched alkyl having 1 to 6 (hereinafter, abbreviated as C₁₋₆) of carbon atoms, particularly, a group such as methyl, ethyl, n-propyl, isopropyl, n-butyl, isobutyl, sec-butyl, tert-butyl, n-pentyl, 3-pentyl, n-hexyl, or the like. C₁₋₅ alkyl is more preferable, and methyl, ethyl, n-propyl, isopropyl, tert-butyl, or 3-pentyl is further preferable.

Preferred example of 'lower alkenyl' includes linear or branched C₂₋₆ alkenyl, particularly, a group such as vinyl, propenyl, butenyl, pentenyl, 1-methylvinyl, 1-methyl-2-propenyl, 1,3-butadienyl, 1,3-pentadienyl, or the like. C₂₋₄ alkenyl is more preferable, and vinyl, propenyl, butenyl, 1-methylvinyl, or 1-methyl-2-propenyl is further preferable.

Preferred example of 'lower alkylene' includes linear or branched C₁₋₆ alkylene, particularly, a group such as methylene, ethylene, trimethylene, tetramethylene, pentamethylene, hexamethylene, propylene, methylmethylene, ethylethylene, 1,2-dimethylethylene, 1,1,2,2-tetramethylethylene, or the like. C₂₋₄ alkylene is more preferable, and methylene, ethylene, trimethylene, tetramethylene, methylmethylene, propylene, or 1,2-dimethylethylene is further preferable.

Preferred example of 'lower alkenylene' includes linear or branched C₂₋₆ alkenylene, particularly, a group such as vinylene, ethylidene, propenylene, butenylene, pentenylene, hexenylene, 1,3-butadienylene, 1,3-pentadienylene, or the like. C₂₋₄ alkenylene is more preferable, and vinylene, ethylidene, propenylene, or butenylene is further preferable.

The 'halogen' indicate F, Cl, Br, or I.
The 'halogeno-lower alkyl' is C₁₋₆ alkyl substituted with one or more of halogens; preferably lower alkyl substituted with 1 to 5 halogens; more preferably fluoromethyl, difluoromethyl, trifluoromethyl, trifluoroethyl, or pentafluoroethyl; and further preferably trifluoromethyl.

The 'cycloalkyl' is a C₃₋₁₀ saturated cyclic hydrocarbon group which may include bridges. In particular, it is a group such as cyclopropyl, cyclobutyl, cyclopentyl, cyclohexyl, cycloheptyl, cyclooctyl, adamantly, or the like. C₃₋₈ cycloalkyl is preferable, and cyclopropyl, cyclobutyl, cyclopentyl, cyclohexyl, or cycloheptyl is more preferable.

The 'cycloalkenyl,' is C₃₋₁₅ cycloalkenyl, which may include bridges and contains a cyclic group condensed with benzene ring at a double-bonded site. In particular, it is a group such as cyclopentenyl, cyclopentadienyl, cyclohexenyl, cyclohexadienyl, 1-tetrahydronaphthyl, 1-indenyl, 9-fluorenyl, or the like. C₅₋₁₀ cycloalkenyl is preferable, and cyclopentenyl or cyclohexenyl is more preferable.

The 'aryl' is a C₆₋₁₄ monocyclic to tricyclic aromatic hydrocarbon group which contains a cyclic group condensed with C₅₋₈ cycloalkene at a double-bonded site. In particular, it is a group such as phenyl, naphthyl, 5-tetrahydronaphthyl, 4-indenyl, 1-fluorenyl, or the like; more preferably phenyl or naphthyl; and further preferably phenyl.

The 'heterocyclic' group is a 3 to 15-membered, preferably 5 to 10-membered, monocyclic to tricyclic heterocyclic group containing 1 to 4 heteroatoms selected from oxygen, sulfur, and nitrogen; and contains a saturated ring, an aromatic ring, and a partially hydrogenated cyclic group. A cyclic atom such as sulfur or nitrogen may be oxidized to form an oxide or a dioxide. In particular, the heterocyclic group is a group such as pyridyl, pyrazinyl, pyrimidinyl, pyridazinyl, imidazolyl, benzoimidazolyl, benzofuranyl, benzothienyl, benzothiadiazolyl, benzothiazolyl, benzoisothiazolyl, benzooxazolyl, benzoisooxazolyl, pyrrolyl, pyrrolidinyl, thienyl, furyl, dioxazolyl, dioxoranyl, triazinyl, triazolyl, tetrazolyl, thiazolyl, thiadiazolyl, oxadiazolyl, pyrazolyl, pyrazolidinyl, isothiazolyl, oxazolyl, isooxazolyl, quinolyl, isoquinolyl, tetrahydroquinolyl, tetrahydroisoquinolyl, quinazolinyl, quinoxalinyl, phthalazinyl, piperidyl, piperazinyl, azepanyl, diazepanyl, tetraydhrofuranyl, tetrahydropyranyl, morpholinyl, methylenedioxyphenyl, ethylenedioxyphenyl, trithianyl, indolyl, isoindolyl, indolinyl, indazolyl, tetrahydrobenzoimidazolyl, chromanyl, chromonyl, benzoimidazolonyl, or the like. The heterocyclic group is preferably a 5 to 10-membered monocyclic to bicyclic heterocyclic group, and more preferably pyrrolyl, imidazolyl, triazolyl, tetrazolyl, furyl, oxazolyl, oxadiazolyl, thienyl, thiazolyl, pyridyl, benzofuranyl, benzothienyl, quinolyl, pyrrolidinyl, piperidinyl, piperazinyl, or morpholinyl.

The meaning of 'may be substituted' is 'no substitution' or 'having 1 to 5 substituents which are the same with or different from each other'. The meaning of 'is substituted' is 'having 1 to 5 substituents which are the same with or different from each other'. In addition, in case of having a plurality of substituents, the substituents may be the same with or different from each other.

The substituents for the 'lower alkylene' and the 'lower alkenylene', which may be substituted, according to R¹; and the 'lower alkylene' and the 'lower alkenylene', which may be substituted, according to R¹¹ are preferably groups selected from halogens, -OR⁰, and -CO₂R⁰.

The substituents for the 'aryl', which may be substituted, according to R¹; the 'aryl', which may be substituted, according to R¹⁰; and the 'aryl', which may be substituted, according to R¹¹ are preferably groups selected from the following G¹ group.
G¹ group: halogen, lower alkyl, halogeno-lower alkyl, -OR⁰, -O-halogeno-lower alkyl, -CO₂R⁰, -O-lower alkylene-CO₂R⁰, lower alkylene-CO₂R⁰, and lower alkenylene-CO₂R⁰.

The substituents for the 'heterocyclic group', which may be substituted, according to R¹; the 'heterocyclic group', which may be substituted, according to R¹⁰; and the 'heterocyclic group', which may be substituted, according to R¹¹ are preferably groups selected from the following G² group.
G² group: halogen, lower alkyl, halogeno-lower alkyl, -OR⁰, -O-halogeno-lower alkyl, -CO₂R⁰, -O-lower alkylene-CO₂R lower alkylene-CO₂R⁰, lower alkenylene-CO₂R⁰, -SR⁰, oxo, and thioxo.

The substituents for the 'cycloalkyl' and the 'cycloalkenyl', which may be substituted, according to R¹¹ are preferably groups selected from halogens, -OR⁰, and-CO₂R⁰.

Preferred embodiments of the present invention will be described below.
(a) Preferred example of R¹ includes -(lower alkylene which may be substituted with -OR⁰)-OR⁰, lower alkenylene-OR⁰, lower aikylene-CO₂R⁰, lower alkenylene-CO₂R⁰, lower alkylene-N(R⁰)-lower alkylene-OR⁰, lower alkylene-N(R⁰)-lower alkylene-CO₂R⁰, lower alkylene-N(lower alkylene-OR⁰)-lower alkylene-CO₂R⁰, lower alkylene-C(O)N(R⁰)-lower alkylene-OR⁰, lower alkylene-C(O)N(R⁰)-lower alkylcne-CO₂R⁰, or lower alkylene-(heterocyclic group substituted with -CO₂R⁰). More preferred example thereof includes lower alkylene-CO₂R⁰ lower alkenylene-CO₂R⁰, lower alkylene-N(R⁰)-lower alkylene-CO₂R⁰, lower alkylene-N(lower alkylene-OR⁰)-lower alkylene-CO₂R⁰, lower alkylene-C(O)N(R⁰)-lower alkylene-CO₂R⁰, or lower alkylene-(heterocyclic group substituted with -CO₂R⁰). Further preferred example thereof includes lower alkylene-CO₂R⁰ or lower alkylene-N(R⁰)-lower alkylene-CO₂R⁰. The most preferred example thereof includes lower alkylene-CO₂H.
(b) Preferred example of L includes a single bond, -O-, or -NH-.
(c) Preferred example of X includes N.
(d) Preferred example of Y includes C(O).
(e) Preferred example of R² includes lower alkyl or cycloalkyl. Isopropyl, 3-pentyl, or cyclopentyl is more preferable; and 3-pentyl or cyclopentyl is further preferable.
(f) Preferred example of R³ includes cycloalkyl or lower alkylene-cycloalkyl. Cyclohexyl or cyclopropylmethyl is more preferable; and cyclohexyl is further preferable.
(g) Preferred example of R⁴ includes -F.
(h) Preferred example of R⁵ includes -H.
As the other preferred embodiments, a compound composed of each preferred group described in the above (a) to (h) is preferable.

In addition, the other preferred embodiments of the compound of the present invention shown as the general formula (I) will be described below.
(1) The compound according to the formula (I) in which X is N, and Y is C(O).
(2) The compound according to (1) in which R³ is cycloalkyl or lower alkylene-cycloalkyl.
(3) The compound according to (2) in which R⁴ is -F.
(4) The compound according to (3) in which R⁵ is -H.
(5) The compound according to (4) in which R² is lower alkyl or cycloalkyl.
(6) The compound according to (5) in which L is a single bond, -O-, or -NH-.
(7) The compound according to (6) in which R¹ is lower alkylene-CO₂R⁰, lower alkenylene-CO₂R⁰, lower alkylene-N(R⁰)-lower alkylene-CO₂R⁰, lower alkylene-N(lower alkylene-OR⁰)-lower alkylene-CO₂R⁰, lower alkylene-C(O)N(R⁰)-lower alkylene-CO₂R⁰, or lower alkylene-(heterocyclic group substituted with -CO₂R⁰).
(8) The compound according to the formula (1) in which X is C(R⁶), and Y is CH(R⁷).
(9) The compound according to the formula (I) in which X is N, and Y is *-C(O)-CH₂- (wherein, * represents a bond to X).
(10) The compound according to the formula (I) selected from a group composed of 4-[7-(cyclnhexylamino)-1-cyclopentyl-6-fluoro-2,4-dioxo-1,4-dihydroquinazolin-3(2H)-yl]butanoic acid; 4-[7-(cyclohexylamino)-1-cyclopentyl-6-fluoro-2,4-dioxo-1,4-dihydroquinazolin-3(2H)-yl]-2-methylbutanoic acid; 4-{[7-(cyclohexylamino)-1-cyclopentyl-6-fluoro-2,4-dioxo-1,4-dihydroquinazolin-3(2H)-yl]amino}butanoic acid; 4-{[7-(cyclohexylamino)-1-cyclopentyl-6-fluoro-2,4-dioxo-1,4-dihydroquinazolin-3(2H)-yl]oxy}butanoic acid; [{2-[7-(cyclohexylamino)-1-cyclopentyl-6-fluoro-2,4-dioxo-1,4-dihydroquinazolin-3(2H)-yl]ethyl}(2-methoxyethyl)amino]acetic acid; 4-({1-cyclopentyl-7-[(cyclopropylmethyl)amino]-6-fluoro-2,4-dioxo-1,4-dihydroquinazolin-3(2H)-yl}amino)butanoic acid; 4-{[7-(cyclohexylamino)-6-fluoro-1-isopropyl-2,4-dioxo-1,4-dihydroquinazolin-3(2H)-yl]amino}butanoic acid; 5-({1-cyclopentyl-7-[(cyclopropylmethyl)amino]-6-fluoro-2,4-dioxo-1,4-dihydroquinazolin-3(2H)-yl}amino)pentanoic acid; 1-{2-[7-(cyclohexylamino)-1-cyclopentyl-6-fluoro-2,4-dioxo-1,4-dihydroquinazolin-3(2H)-yl]ethyl}piperidine-3-carboxylic acid; (2E)-4-[7-(cyclohexylamino)-1-cyclopentyl-6-fluoro-2,4-dioxo-1,4-dihydroquinazolin-3(2H)-yl]-2-butenoic acid; and {[7-(cyclohexylamino)-1-cyclopentyl-6-fluoro-2,4-dioxo-1,4-dihydroquinazolin-3(2H)-yl]oxy}acetic acid, or a pharmaceutically acceptable salt thereof.

According to the type of the substituents, tautomers or geometrical isomers of the compound of the invention may exist. In the present specification, only one form of the isomers of the compound is described. However, a compound separated from the isomers or a mixed isomer, as well as the isomers, is included in the invention.
In addition, the compound (I) may contain asymmetrical carbon atoms and axial asymmetry. Therefore, optical isomers such as (R) form and (S) form based on the compound may exist. Both a compound mixed with these optical isomers and a compound isolated from these isomers is included in the invention.
Moreover, a pharmacologically acceptable prodrug of the compound (I) is included in the invention. A pharmacologically acceptable prodrug represents a compound having a group capable of being converted into the amino group, OH, CO₂H, and the like of the invention by solvolysis or under physiological conditions. Examples of the group which forms the prodrug include groups described in Prog. Med., 5,2157 to 2161 (1985) and 'Pharmaceutical Research and Development', Drug Design, Hirogawa Publishing Company, Vol. 7,163 to 198 (1990).

According to the type of the substituent, the compound of the invention may form an acid addition salt or a salt with base. Only when the resulting salt is a pharmaceutically acceptable salt, it is included in the invention. Specific examples of the salt include acid addition salts with an inorganic acid such as hydrochloric acid, hydrobromic acid, hydroiodic acid, sulfuric acid, nitric acid, phosphoric acid, or the like, and an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethansulfonic acid, p-toluenesulfonic acid, aspartic acid, glutamic acid, or the like; salts with an inorganic base such as sodium, potassium, magnesium, calcium, aluminum, or the like, and an organic base such as methylamine, ethylamine, ethanolamine, lysine, ornithine, or the like; ammonium salts; and the like.
In addition, various hydrates, solvates, and substances having crystal polymorphism of the compound of the invention and a pharmaceutically acceptable salt thereof are included in the invention. Moreover, a compound labeled with a radioactive isotope or a nonradioactive isotope is included in the invention.

### (Production Method)

A compound of the invention and a pharmaceutically acceptable salt thereof may be produced by using the characteristics based on the type of a basic structure or substituents thereof and applying various known synthesis methods. At this time, according to the type of a functional group, it may technically be effective in production when the functional group is replaced with an appropriate protective group (a group capable of being readily converted into the functional group) at the stage of a starting material or an intermediate. Examples of the functional group include an amino group, a hydroxyl group, a carboxyl group, and the like. Examples of the protective group include protective groups described in 'Protective Groups in Organic Synthesis (3rd edition, 1999)' written by Greene and Wuts. It is preferable that these groups are appropriately selected for a use according to the reaction conditions. According to the methods, when a reaction is carried out by introducing the protective group and then the protective group is removed according to need, the desired compound may be obtained.
The prodrug of the compound (I) may be produced by introducing the specific group at the stage of a starting material or an intermediate in the same manner as the above protective group or by carrying out a reaction using the resulting compound (I).
The reaction may be carried out by a person skilled in the art applying the known methods such as normal esterification, amidation, dehydration, and the like.
The typical method for producing the compound of the invention will be described below. However, the production method of the invention is not limited to the following examples.

### (Production Process 1)

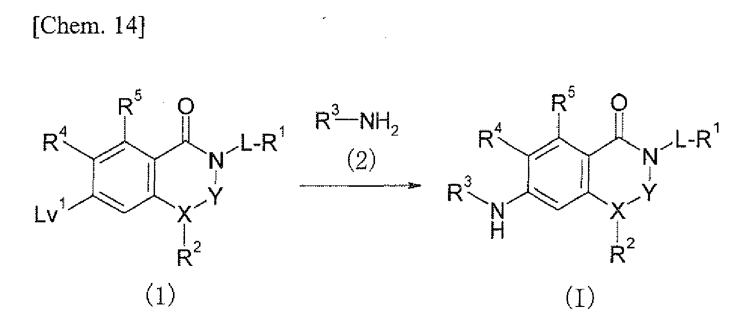

(wherein, Lv¹ represents an elimination group. The same shall be applied hereinafter.)
This production process includes making a compound (1) undergo a reaction with a compound (2) to obtain the compound (I) of the present invention. Herein, Lv¹ is an elimination group and for example, halogens, methanesulfonyloxy, p-toluenesulfonyloxy, and the like may be exemplified.
As for the reaction, the same amount of the compound (1) and the compound (2) or an excess amount of one of the compounds is used for the reaction. The reaction is carried out in an inert solvent or without a solvent under a cooled condition to heated by reflux, preferably at the temperature of 0°C to 100°C, and stirred generally for 0.1 hours to 5 days. Herein, as the solvent, it is not particularly limited but examples of the solvent include aromatic hydrocarbons such as benzene, toluene, xylene, and the like; ethers such as diethyl ether, tetrahydrofuran (THF), dioxane, dimethoxyethane, and the like; halogenated hydrocarbons such as dichloromethane, 1,2-dichloroethane, chloroform, and the like; N,N-dimethylformamide (DMF); N,N-dimethylacetamide (DMA); N-methylpyrrolidine-2-one (NMP); dimethyl sulfoxide (DMSO); ethyl acetate; acetonitrile; or mixtures thereof. When the reaction is carried out in the presence of an organic base such as triethylamine, N,N-diisopropylethylamine, N-methylmorpholine, or the like, or an inorganic base such as potassium carbonate, sodium carbonate, or potassium hydroxide, or the like, it may be advantageous to smoothly proceed the reaction.

### (Production Process 2)

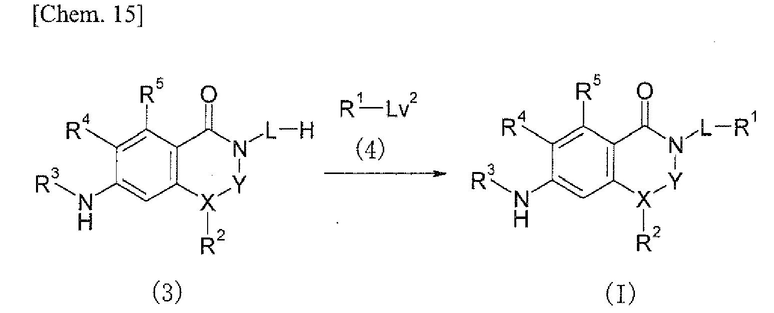

(wherein, Lv² represents an elimination group or -OH. The same shall be applied hereinafter)
This production process includes making a compound (3) undergo a reaction with a compound (4) to obtain the compound (I) of the present invention. Herein, Lv² is an elimination group and for example, halogens, methanesulfonyloxy, p-toluenesulfonyloxy, and the like may be exemplified.
As for the reaction, when Lv² is an elimination group, the same amount of the compound (3) and the compound (4) or an excess amount of one of the compounds is used for the reaction. The reaction is carried out in an inert solvent or without a solvent, under a cooled condition to heated by reflux, preferably at the temperature of 0°C to 100°C, and stirred generally for 0.1 hours to 5 days. Herein, as the solvent, it is not particularly limited but examples of the solvent include aromatic hydrocarbons, ethers, halogenated hydrocarbons, DMF, DMA, NMP, DMSO, ethyl acetate, acetonitrile, or mixtures thereof. When the reaction is carried out in the presence of an organic base such as triethylamine, N,N-diisopropylethylamine, N-methylmorpholine, or the like, or an inorganic base such as potassium carbonate, sodium carbonate, or potassium hydroxide, or the like, it may be advantageous to smoothly proceed the reaction.
In addition, in accordance with a compound, the compound (4) in which Lv² is - OH may be used to obtain the compound (I) of the invention by a Mitsunobu reaction or the modified method thereof. For example, by using an activating agent prepared from azodicarboxylic acid derivatives such as diethyl azodicarboxylate and the like, and phosphine reagents such as triphenylphosphine and the like, the reaction can be carried out in solvents such as ethers, halogenated hydrocarbons, and the like under a cooled condition, at room temperature to under a heated condition.

### (Production Process 3)

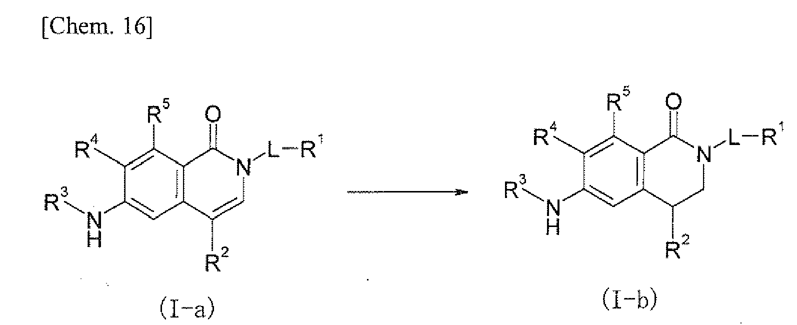

This production process includes hydrogenating a compound (I-a) of the invention to obtain a compound (I-b) of the invention.
The reaction is carried out under a hydrogen atmosphere the compound (I-a), in an inert solvent for a reaction, is stirred in the presence of metal catalysts generally for 1 hour to 5 days. In general, this reaction is carried out under a cooled condition to a heated condition, and preferably at room temperature. Herein, examples of the solvent are not particularly limited but include alcohols such as methanol, ethanol, 2-propanol, and the like; ethers; water; ethyl acetate; DMF; and the like. As the metal catalysts, palladium catalysts such as palladium carbon, palladium black, palladium hydroxide, and the like; platinum catalysts such as a platinum plate, platinum oxide, and the like; and nickel catalysts such as Raney nickel and the like are preferably used. In accordance with a compound, it may be advantageous to carry out the reaction in the presence of acids such as acetic acid, hydrochloric acid, and the like.

### (Production Process 4)

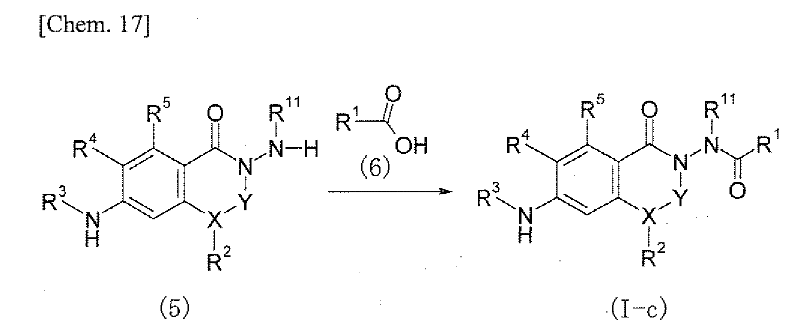

This production process includes making a compound (5) undergo a reaction with a compound (6) or the reactive derivative thereof to perform amidation and obtain a compound (I-c) of the present invention.
For the amidation reaction, a method which includes using a condensation agent such as carbonyldiimidazole (CDI), 1-ethyl-3-(3-dimethylaminopropyl)carbodiimide hydrochloride (WSC·HCl), dicyclohexylcarbodiimide, diphenylphosphoryl azide, diethylphosphoryl cyanide, and the like; a method which includes using isobutyl chloroformate, ethyl chloroformate, and the like to obtain the compound via a mixed anhydride; and a method which includes using thionyl chloride, phosphorus oxychloride, or the like to obtain the compound via an acid halide are suitably applied. The reaction conditions may be suitably selected in accordance with the reactive derivative and the condensation agent to be used, and the reaction may be generally carried out in an inert solvent for a reaction, such as, halogenated hydrocarbons, aromatic hydrocarbons, ethers, pyridine, DMF, DMSO, and the like, under a cooled condition, a cooled condition to room temperature, and room temperature to a heated condition. In accordance with the reactions, it may be advantageous to carry out the reaction in the presence of an organic base (triethylamine, diisopropylethylamine, N-methylmorpholine, pyridine, 4-(N,N-dimethylamino)pyridine, or the like is suitably used) or a metallic base (potassium carbonate, cesium carbonate, or the like is suitably used).

### (Production Process 6)

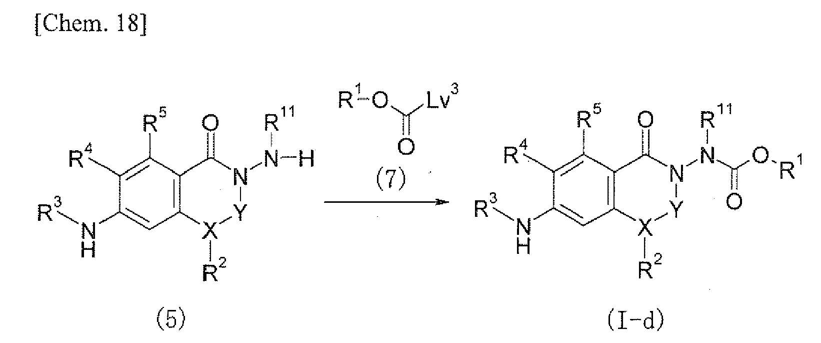

(wherein, Lv³ represents an elimination group such as halogens and the like. The same shall be applied hereinafter)
This production process includes making the compound (5) to undergo a reaction with a compound (7) to obtain a compound (I-d) of the present invention.
The reaction is carried out in an inert solvent for a reaction, such as, halogenated hydrocarbons, aromatic hydrocarbons, ethers, pyridine, DMF, DMSO, or the like, under a cooled condition, a cooled condition to room temperature, and room temperature to a heated condition. In accordance with the reactions, it may be advantageous to carry out the reaction in the presence of an organic base (triethylamine, diisopropylethylamine, N-methylmorpholine, pyridine, 4-(N,N-dimethylamino)pyridine, or the like is suitably used) or a metallic base (potassium carbonate, cesium carbonate, or the like is suitably used).

### Production Process 6: The Other Production Processes

A number of the compounds represented by the formula (I) may be produced by using the compound of the invention obtained as mentioned above and by arbitrarily combining processes which the person skilled in the art can generally employ, such as, the known amidation, hydrolysis, a Homer-Emmons reaction, a Wittig reaction, oxidation, reduction, alkylation, and the like. For example, the following reactions may be employed.

### Production Process 6-1

When carboxylic acid and an amine compound is amidated, it is possible to produce an amide compound.
The reaction can be carried out in the same manner as in Production Process 4.

### Production Process 6-2

When a compound containing an ester group is hydrolyzed, it is possible to produce a compound containing a carboxyl group. For example, the reaction can be carried out in an inert solvent for a reaction, such as aromatic hydrocarbons, ethers, halogenated hydrocarbons, alcohols, DMF, DMSO, pyridine, water, and the like, in the presence of an acid such as mineral acids, for example, sulfuric acid, hydrochloric acid, hydrobromic acid, and the like, and organic acids, for example, formic acid, acetic acid, and the like; or in the presence of a base such as lithium hydroxide, sodium hydroxide, potassium hydroxide, potassium carbonate, sodium carbonate, cesium carbonate, ammonia, or the like, under a cooled condition to a heated condition.

### Production Process 6-3

When a compound containing an aldehyde and a ketone is subjected to a Homer-Emmons reaction or a Wittig reaction, it is possible to convert an oxo group to an alkylidene group.
For the Homer-Emmons reaction or the Wittig reaction, a method which the person skilled in the art generally uses may be employed. For example, in the presence of a Horner-Emmons reagent or a Wittig reagent, the reaction can be carried out in a solvent such as aromatic hydrocarbons, ethers, halogenated hydrocarbons, DMF, DMA, NMP, DMSO, acetonitrile, or the like under a cooled condition to a heated condition. In accordance with the type of the Homer-Emmons reagent or the Wittig reagent, it is preferable to carry out the reaction in the presence of a base such as potassium carbonate, tert-butoxy potassium, sodium hydride, alkyl lithium such as n-butyl lithium, and the like.

### (Starting Compound Synthesis)

Starting compounds used for the production of the compound (I) of the invention can be synthesized by applying the following methods, the known methods, or the modified methods thereof.

### (Starting Material Synthesis 1)

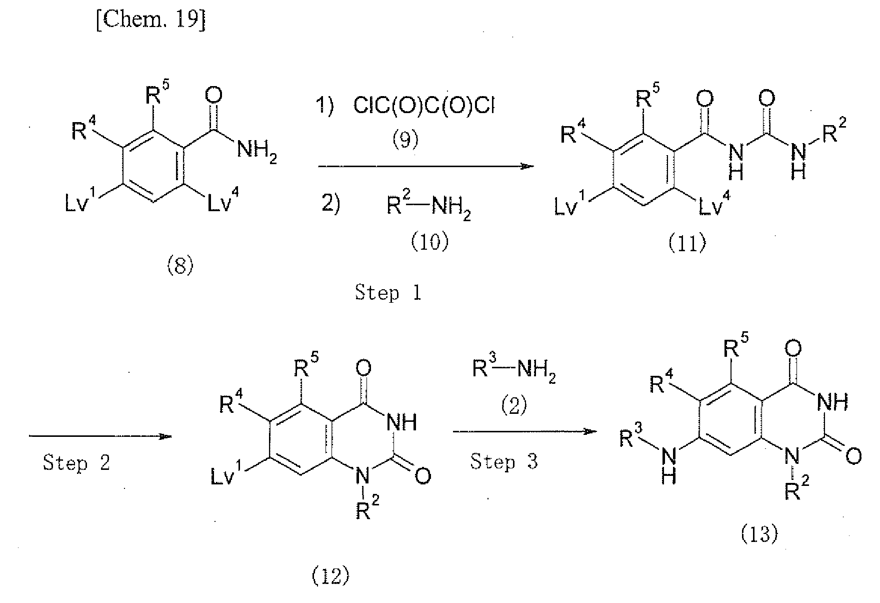

(wherein, Lv⁴ represents an elimination group, for example, halogens such as fluorine, chlorine, and the like. The same will be applied hereinafter)

### Step 1:

This step includes making acylisocyanate, which is obtained by reacting a compound (8) to oxalyl chloride (9), undergo a reaction with a compound (10) to obtain a compound (11).
The reaction between the compound (8) and oxalyl chloride (9) can be carried out by using the same amount or an excess amount of one of the compounds in a solvent such as ethers, halogenated hydrocarbons, or the like under ice cooling, at room temperature to under a heated condition. The resulting acylisocyanate may be isolated or not be isolated to be used for a reaction with the compound (10).
The reaction between the resulting acylisocyanate and the compound (10) can be carried out by using the same amount or an excess amount of one of the compounds in a solvent such as ethers, halogenated hydrocarbons, aromatic hydrocarbons, or the like under a cooled condition, at room temperature to under a heated condition.

### Step 2:

This step includes cyclizing a compound (11) within a molecule to obtain a compound (12).
In the presence of a base such as sodium hydride, potassium bis(trimethylsilyl)amide, and the like, the reaction is carried out in an inert solvent or without a solvent under a cooled condition to heated by reflux, preferably at the temperature of 0°C to 100°C, and stirred generally for 0.1 hours to 5 days. Herein, as the solvent, it is not particularly limited but examples of the solvent include aromatic hydrocarbons, ethers, halogenated hydrocarbons, DMF, and the like.

### Step 3:

This step includes making a compound (12) undergo a reaction with the compound (2) to obtain a compound (13).
The reaction can be carried out in the same manner as in Production Process 1.

### (Starting Material Synthesis 2)

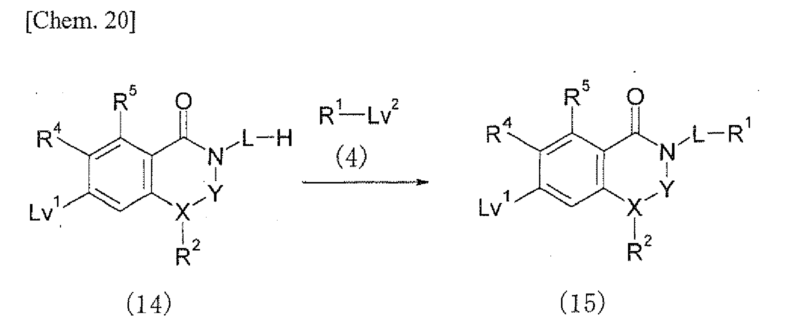

This process includes making a compound (14) undergo a reaction with the compound (4) to obtain a compound (15).
The reaction can be carried out in the same manner as in Production Process 2.

### (Starting Material Synthesis 3)

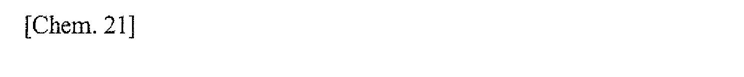

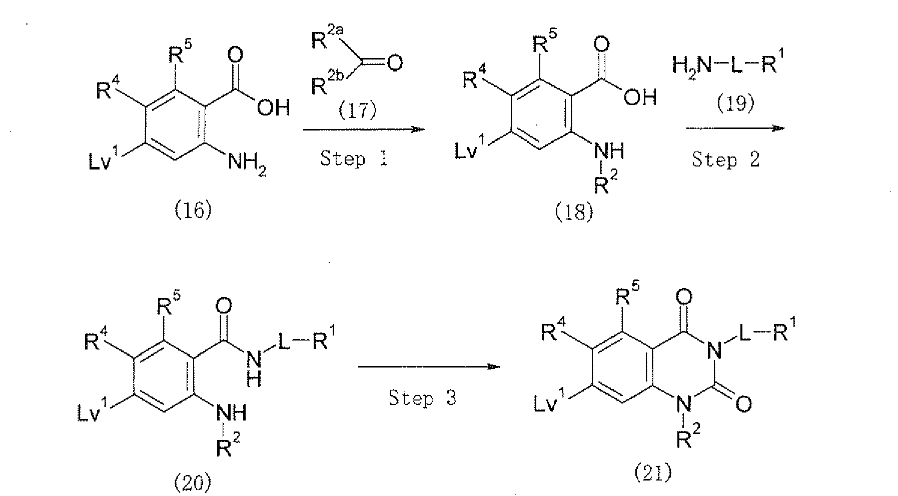

(wherein, R^{2a} and R^{2b} indicate remnants of an aldehyde or a ketone corresponding to R². The same will be applied hereinafter)

### Step 1:

This step includes reductively alkylating a compound (16) and an aldehyde or a ketone compound (17) corresponding to R² to obtain a compound (18).
By using the same amount of the compound (16) and the compound (17) or an excess amount of one of the compounds and in the presence of a reducing agent, the reaction is carried out in an inert solvent for a reaction and from -45°C to heated by reflux, preferably up to 0°C to room temperature, and stirred for generally 0.1 hours to 5 days. Herein, as the solvent, it is not particularly limited but examples of the solvent include alcohols, ethers, or the mixture thereof. Examples of the reducing agent include sodium cyanoborohydride, sodium triacetoxyborohydride, sodium borohydride, and the like. It may be preferable to carry out the reaction in the presence of a dehydrating agent such as molecular sieves and the like, or an acid such as acetic acid, hydrochloric acid, titanium (IV) isopropoxide complex, and the like. In accordance with the reaction, in the case where it is possible to stably isolate an imine body formed as an intermediate in a reaction system, it is permissible to separately carry out a reductive reaction after obtaining the imine body.

### Step 2:

This step includes amidating a compound (18) and a compound (19) to obtain a compound (20).
The reaction can be carried out in the same manner as in Production Process 4.

### Step 3:

This step includes making the compound (20) undergo a reaction with a carbonylating agent for cyclization to obtain a compound (21).
By using the same amount of the compound (20) and the carbonylating agent such as phosgene, triphosgene, CDI, ethyl chloroformate, and the like or an excess amount of one of the compounds, the reaction can be carried out in a solvent such as ethers, halogenated hydrocarbons, DMF, and the like at room temperature to under a heated condition. In accordance with the reaction, it may be advantageous to carry out the reaction in the presence of an organic base (triethylamine, diisopropylethylamine, N-methylmorpholine, pyridine, or the like is suitably used) or a metallic base (sodium hydride, potassium carbonate, cesium carbonate, or the like is suitably used).

### (Starting Material Synthesis 4)

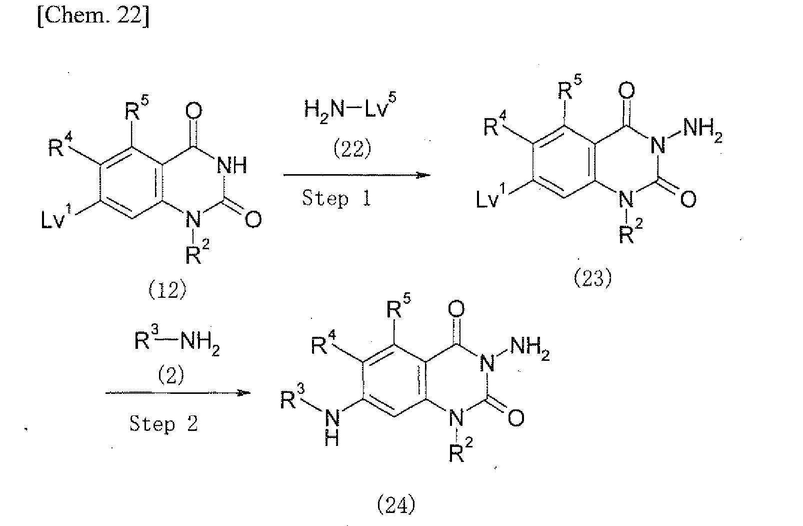

(wherein, Lv⁵ represents an elimination group such as 2,4-dinitrophenoxy and the like. The same will be applied hereinafter)

### Step 1:

This step includes making the compound (12) undergo a reaction with a compound (22) to obtain a compound (23).
By using the same amount of the compound (12) and the compound (22) or an excess amount of one of the compounds and in the presence of a base such as sodium hydride, potassium carbonate, and the like, the reaction can be carried out in a solvent such as ethers, DMF, DMA, NMP, and the like at room temperature to under a heated condition.

### Step 2:

This step includes making the compound (23) undergo a nucleophilic substitution reaction with the compound (2) to obtain a compound (24).
The reaction can be carried out in the same manner as in Production Process 1.

### (Starting Material Synthesis 5)

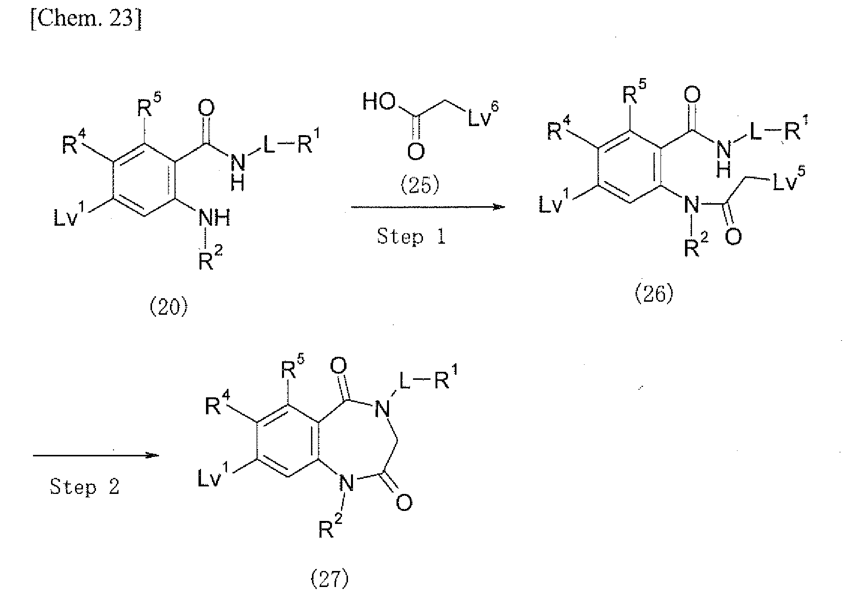

(wherein, Lv⁶ represents an elimination group such as chlorine, bromine, and the like. The same will be applied hereinafter)

### Step 1:

This step includes amidating the compound (20) and a compound (25) to obtain a compound (26).
The amidation can be carried out in the same manner as in Production Process 4.

### Step 2:

This step includes cyclizing the compound (26) within a molecule to obtain a compound (27) of the invention.
The reaction can be carried out by subjecting the compound (26) to the reaction in the presence of a base such as sodium hydride and the like in an inert solvent for a reaction, such as aromatic hydrocarbons, ethers, halogenated hydrocarbons, DMF, and the like, under a cooled condition to a heated condition under reflux.

### (Starting Material Synthesis 6)

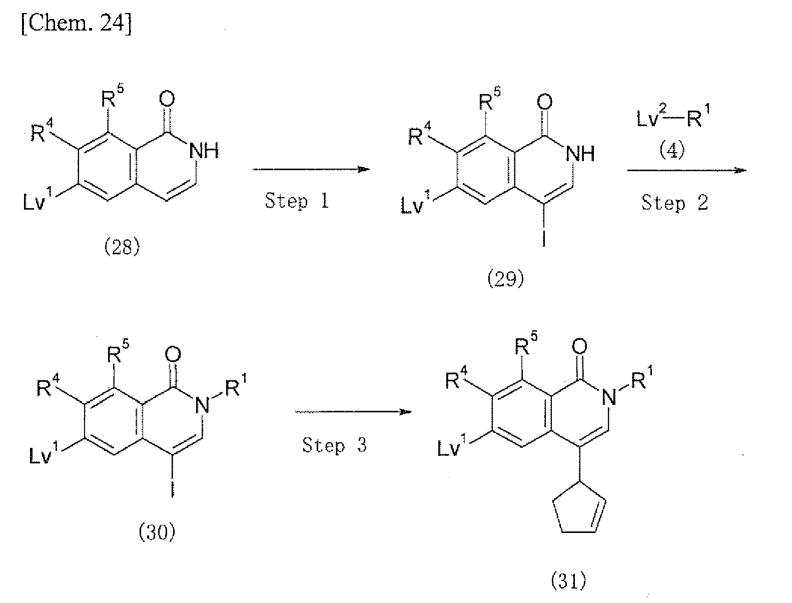

### Step 1:

This step includes making a compound (28) undergo a reaction with an iodizing agent to obtain a compound (29).
By using the same amount of the compound (28) and the iodizing agent such as iodine, iodine monochloride, N-iodosuccinimide, and the like or an excess amount of one of the compounds, the reaction can be carried out in a solvent such as aromatic hydrocarbons, halogenated hydrocarbons, pyridine, and the like at room temperature to under a heated condition.

### Step 2:

This step includes making the compound (29) undergo a reaction with the compound (4) to obtain a compound (30).
The reaction can be carried out in the same manner as in Production Process 2.

### Step 3:

This step includes making the compound (30) undergo a reaction with cyclopentene to obtain a compound (31).
By using the same amount of the compound (30) and cyclopentene or an excess amount of one of the compounds, the reaction can be carried out in an inert solvent for a reaction in the presence of a base and a palladium catalyst at room temperature to under a heated condition under reflux. Herein, examples of the solvent are not particularly limited but include aromatic hydrocarbons, ethers, halogenated hydrocarbons, DMF, and the like. As the base, potassium carbonate, potassium acetate, and the like are preferable. As the palladium catalyst, tetrakis(triphenylphosphine)palladium, palladium acetate, and the like are preferable.

The compound of the invention is isolated as a free compound or a pharmaceutically acceptable salt thereof, a hydrate, a solvate, or a substance having crystal polymorphism, and then purified. A pharmaceutically acceptable salt of the compound (1) of the invention can be produced according to the processes included in a common salt productive reaction.
The isolation and the purification of the compound are carried out by applying common chemical operations such as extraction, fractionated crystallization, various differential chromatographies, and the like.
It is possible to separate the various isomers by selecting the suitable starting compounds or using the difference in physicochemical properties between the isomers. For example, it is possible to lead an optical isomer to a stereochemically pure isomer according to the general optical resolution methods (for example, fractionated crystallization which leads to an optically-active diastereomeric salt with a base or an acid, chromatography in which a chiral column is used, and the like). In addition, it is possible to produce the isomer from the suitably optically-active starting compound.

Pharmacological activities of the compound of the invention have been confirmed by the following tests.

### Test Method (1) Human Platelet Aggregation Inhibitory Activity Measurement Test

Using a syringe in which 1/10 vol of a 3.8% sodium citrate solution is contained, blood was collected from a healthy volunteer (adult male). The blood was subjected to a centrifugation at 160 × g for 10 minutes and a supernatant platelet rich plasma (PRP) was separated. The remaining blood of which the PRP has been collected was subjected to a centrifugation at 1,800 × g for 10 minutes and a platelet poor plasma (PPP) was separated. The number of the platelets in the PRP was measured using an automatic blood cell counter (MEK-6258, Nihon Kohden Corporation). After that, the PPP was added to the PRP to adjust the number of the platelets to 3 × 10⁸ /ml, thereby using the platelets for the following tests. As an ADP, which is a platelet aggregating agent, a product manufactured by MC Medical Inc. was used. The platelet aggregation was measured using a platelet aggregometer (MCM Hematracer 212; MC medical Inc). That is, 80 µl of the PRP having the number of the platelets of 3 x 10⁸ /ml and 10 µl of a test compound solution or solvent (10% DMSO or 10% DMSO-9% hydroxypropyl-β-cyclodextrin-4.5% d-mannitol) were incubated at 37°C for 1 minute and then 10 µl of ADP (50 µM) was added to the mixture to cause platelet aggregation, thereby recording a variation in transmitted light for 5 minutes. An inhibition rate was calculated using an area under the platelet aggregation curve as an index. The result when the compound of the invention has 10 µM (final concentration) is shown in Table 1. In addition, Ex represents a number of the compounds of Examples.

**[Table 1]**

| Ex | % Inhibition |
|---|---|
| 24 | 89 |
| 42 | 75 |
| 65 | 86 |
| 69 | 50 |
| 77 | 88 |
| 114 | 91 |
| 155 | 93 |

### Test Method (2) Substitution Test for the binding between Human P2Y12 and 2-methylthio-ADP(2-MeS-ADP)

In a 10 cm petri dish, C6-15 cells were spread to be 1 x 10⁶ of cells by using a DMEM culture medium and cultured for 1 day. After that, genes of plasmids such as 8 µg of pEF-BOS-dhfr-human P2Y12 and 0.8 µg ofpEF-BOS-neo (Nucleic Åcid Res., 18, 5322, 1990) were introduced to the cells using a transfection reagent (LipofectAMINE 2000; manufactured by GIBCO BRL Inc).
After 24 hours since the above-mentioned gene introduction operation had been completed, the cells to which the genes were introduced were recovered and suspended in a DMEM culture medium containing 0.6 mg/ml of G418 (manufactured by GIBCO BRL Inc). After that, the cells were serially diluted and spread on a 10 cm petri dish. Colonies appeared after 2 weeks later were individually collected and used in the following tests as P2Y12 protein-expressing C6-15 cells (WO 02/36631, Mol. Pharmacol., 60, 432, 2001).
The P2Y12 protein-expressing C6-15 cells were cultured and then the cells were recovered. The cells were washed with PBS, suspended in 20 mM Tris-HCl (pH 7.4) containing 5 mmol/l EDTA and Complete^{™} (manufactured by Boehringer Mannheim) which is a protease inhibitor cocktail set, and homogenized by Polytron. The cells were subjected to an ultracentrifugation and a precipitate is suspended in 50 mM Tris-HCl (pH 7.4) containing 1 mM of EDTA, 100 mM NaCl, and Complete^{™} to perform membrane fraction.
To 100 µl of the P2Y12 protein-expressing C6-15 cell membrane fraction (100 µg/ml) produced as mentioned above, 1.5 µl of a test compound solution and 50 µl of 0.75 nM [³³P]-2-MeS-ADP (2100 Ci/ mmol, manufactured by PerkinElmer Inc) were added. The mixture was incubated in 50 mM Tris-HCl (pH 7.4) containing 100 mM NaCl and 50 mM MgCI₂ at room temperature for 1 hour, and then recovered by cell harvest on a glass filter. Microscintillators were added to the glass filter and the radioactive activity was measured by a liquid scintillation counter. In addition, simultaneously, the radioactivity in the case where only a solvent is added to the above-mentioned test and in the case where 1.5 µl of 250 µM ADP is added thereto were measured respectively as the total binding amount and the nonspecific binding amount. The total binding amount and the nonspecific binding amount were set respectively as the inhibition rate of 0% and the inhibition rate of 100% to calculate the inhibition rate (%) of the test compound. The result when the compound of the invention has 30 nM (final concentration) is shown in Table 2.

**[Table 2]**

| Ex | % Inhibition |
|---|---|
| 24 | 60 |
| 42 | 56 |
| 114 | 71 |
| 155 | 72 |
| 157 | 67 |

### Test Method (3) Rat Platelet Aggregation Inhibition Test and Measurement of Test Compound Concentration in Plasma

An aqueous sodium hydroxide solution and the same amount of a 1% aqueous methyl cellulose solution were added to the compound of the invention to prepare a 0.5% aqueous methyl cellulose solution or a suspension solution. This preparation solution was orally administered to a male SD rat (5 to 7 weeks old) which has been fasted over 12 hours using a sonde at a dose of 30 mg/kg. After 2 hours later since the administration of the compound, using a syringe in which 1/10 vol of a 3.8% sodium citrate solution is contained, a blood was collected. In the same manner as in Test Method (1), a PPP and a PRP having the number of the platelets as 3 x 10⁸ /ml were prepared. 90 µl of the PRP having the number of the platelets of 3 x 10⁸ /ml was incubated at 37°C for 1 minute and then 10 µl of ADP (50 µM) was added thereto to cause platelet aggregation, thereby recording a variation in transmitted light for 5 minutes. An inhibition rate was calculated using an area under the platelet aggregation curve as an index.
The PPP prepared as mentioned above was used to measure the concentration of the PPP in the plasma. In order to form a standard curve, a PPP of a SD rat to which the compound is not administered has been separated and the compounds of the invention consecutively diluted (final concentration of 30 µM to 0.0003 µM: suitably selected according to the compounds) by such PPP are also prepared. To 100 µl of PPP of the rat to which the compound of the invention is administered and PPP which contains the diluted compounds of the invention, distilled water (equal amount) and 5% trichloro acetic acid were added, thereby mixing them. The mixtures were placed under ice cooling for 10 minutes and subjected to a centrifugation operation to recover supernatants. To the supernatants was added 2M Tris base (3 µl), and they were mixed to neutralize the supernatants. 50 µl of the P2Y12 protein-expressing C6-15 cell membrane fraction (200 µg/ ml) and 50 µl of the PPP (according to the compounds, the PPP diluted with 50 mM Tris-HCl (pH 7.4) in which 100 mM NaCl and 50 mM MgCl₂ are contained was used) treated with trichloro acetic acid were mixed. After that, 50 µl of 0.75 nM [33P]-2-MeS-ADP (2100 Ci/ mmol, manufactured by PerkinElmer Inc.) was added to the mixture. The mixture was incubated in 50 mM Tris-HCl (pH 7.4) containing 100 mM NaCl and 50 mM MgCl₂ at room temperature for 1 hour, and then recovered by cell harvest on a glass filter. Microscintillators were added to the glass filter and the radioactive activity was measured by a liquid scintillation counter. A binding inhibition curve calculated based on the measurement results from the PPP containing the consecutively diluted compound of the invention was set as a standard curve, and then based on the result from the PPP-derived from the rat to which the compound of the invention is administered, the concentration of the compound of the invention in the PPP was converted.
The results are shown in Table 3. As a result from evaluation according to the above-mentioned method, it has been cleared that the compound of the invention exhibits an excellent platelet aggregation inhibitory activity when it is orally administered and also exhibits satisfactory disposition.

**[Table 3]**

| Ex | % Inhibition |
|---|---|
| 65 | 56 |
| 114 | 90 |

As results from each of the above-mentioned tests, it has been found that the compound of the invention exhibits excellent P2Y12 inhibitory action, platelet aggregation inhibitory action, and disposition. Therefore, the compound of the invention is useful as a prophylactic and/or therapeutic agent for circulatory diseases closely related to formation of blood clot due to platelet aggregation, for example, ischemic diseases such as reocclusion and restenosis, which are subsequent to unstable angina, acute cardiac infarction and the secondary prevention thereof, hepatic artery bypass surgery, and a PTCA method or the stent placement surgery, acceleration of dissolution of blood clot in hepatic artery, and prevention of reocclusion; cerebral vascular disorders such as transient cerebral ischemic attack (TIA) cerebral infarction, subarachnoid hemorrhage (vasospasm), and the like; peripheral arterial diseases such as chronic arterial occlusive disease and the like; etc. and as an adjunctive agent at the time of cardiac surgery or vascular surgery.

It is possible to prepare a drug preparation containing the compound (I) of the present invention and 1 or 2 or more kinds of the salts thereof as effective components according to the methods generally applied using a carrier for a medical agent, excipient, and the like which have been generally used in this field.
For the administration, it may be the oral administration by using a tablet, a pill, a capsule, a granuled agent, a powdered agent, a liquid agent, and the like; or the parenteral administration by using an injection agent for intra-articular, intravenous, intramuscular, and the like, suppository, eye-drops, ophthalmic ointments, transdermal solutions, ointments, adhesive skin patches, transmucosal solutions, transmucosal patches, inhalers, and the like.
As a solid composition for the oral administration according to the invention, a tablet, a powdered agent, a granule agent, and the like are used. In these solid compositions, one or two or more kinds of the effective components are mixed with at least one kind of inert excipient, for example, lactose, mannitol, glucose, hydroxypropyl cellulose, microcrystalline cellulose, starch, polyvinyl pyrollidone, and/or magnesium aluminometasilicate. According to the general methods, it is permissible that the composition may contain inert additives, for example, a lubricant such as magnesium stearate, a disintegrating agent such as sodium carboxymethyl starch and the like, a stabilizing agent, and a solubilizing agent. If necessary, it is permissible that the tablet and the pill are coated with sugar or films made of substance soluble in the stomach or the intestines.
Examples of a liquid composition for the oral administration include a pharmaceutically acceptable opalizers, solutions, suspension agents, syrups, elixirs, or the generally used inert diluents, for example, purified water or ethanol. The liquid composition may contain adjuvants such as a solubilizing agent, a wetting agent, and a suspension agent, sweetener, flavor, aromatic, and antiseptic agents as well as inert diluents.
Examples of an injection agent for the parenteral administration include aseptic aqueous or nonaqueous solutions, suspention agents, or opalizers. Examples of the aqueous solutions include distilled water for injection or physiological saline. Examples of the nonaqueous solutions include propylene glycol, polyethylene glycol, vegetable oil such as olive oil, alcohols such as ethanol, polysorbate 80 (Japanese Pharmacopoeia), or the like. These compositions may further contain a tonicity agent, an antiseptic agent, a wetting agent, an emulsifying agent, a dispersant, a stabilizing agent, or a solubilizing agent. These are sterilized by, for example, filtration through a bacteri-holding filter, combination with disinfecting agents, or irridation. In addition, it is permissible that a sterile solid composition is produced by using these additives and before using them, they are dissolved or suspended in sterile water or sterile solvents for injection and then used.
Examples of an agent for external use include ointments, plasters, cream, jelly, patches, sprays, lotions, eye-drops, ophthalmic ointments, and the like. The generally used ointment base, lotion base, aqueous or nonaqueous liquid, suspension agents, emulsion, and the like are included. Examples of the ointment base or lotion base include polyethylene glycol, propylene glycol, white petrolatum, white beeswax, polyoxyethylene-hydrogenated castor oil, glyceryl monostearate, stearyl alcohol, cetyl alcohol, lauromacrogol, sorbitan sesquioleate, and the like.
As the transmucosal products such as inhalers, transnasal products, solid, liquid, or semisolid products have been used and it is possible to produce the products according to the known methods. For example, it is permissible appropriately to add the known excipients, pH adjusting agents, an antiseptic agent, surfactants, lubricant, a stabilizing agent, a thickening agent, and the like. For the administration, a device for appropriate inhalation or insufflations may be used. For example, the known devices such as a measured administration inhalation device and the like or a sprayer may be used to administer compounds as the compound itself, the prescribed powdered mixture, or a solution or a suspension solution in which the compound is combined with a pharmaceutically acceptable carrier. For the dried powder inhaler, it may be a disposable inhaler or an inhaler capable of once or multiple administrations. In addition, dried powders or a powder-containing capsule may be used. On the other hand, the inhaler may be a suitable ejection product, for example, a pressurized aerosol spray type in which the most appropriate gas such as chlorofluoroalkane, hydrofluoroalkane, or carbon dioxide is used, etc.

In the case of oral administration, the daily dose is generally from about 0.001 to 100 mg/kg, preferably from 0.1 to 30 mg/kg, and more preferably 0.1 to 10 mg/kg per body weight, administered in one portion or in 2 to 4 divided portions. In the case of the intravenous administration, the daily dose is suitably administered from around 0.0001 to 10 mg/kg per body weight, once a day or two or more times a day. In addition, in the case of the transmucosal agents, at a dose of from around 0.001 to 100 mg/kg per body weight is administered once a day or two or more times a day. The dose is appropriately decided in response to the individual case taking the symptoms, the ages, the genders, and the like into consideration.

The compound of the present invention may be used in combination with various therapeutic or prophylactic agents for the diseases for which the above-mentioned compound of the invention are considered to be effective. The combined preparation may be administered simultaneously, or may be separately administered in succession or at desired intervals. The preparations to be co-administered may be a blend, or may be prepared individually.

### Examples

The production processes of the compound (I) of the invention will be described below in detail on the basis of Examples. However, the compound of the invention is not limited to the compounds described in the following Examples. The production processes of a starting compound are represented in Production Examples.

As for Production Examples, Examples, and Tables described below, the following abbreviations will be used.
PEx: Production Examples, Ex: Examples, No: number of compounds, MS: m/z value of mass analysis (EI: EI-MS; FAB: FAB-MS; ESI: ESI-MS; + described following to an ionization method represents a positive ion, and - represents a negative ion; and when there is no particular limitation, in the case of EI, it represents (M)⁺, in the case of FAB+ and ESI+, it represents (M+H)⁺, and in the case of FAB- and ESI-, it represents (M-H)⁻), NMRI: δ(ppm) of ¹H NMR in DMSO-d₆, Syn: Production Method (the number represents that similar to the compounds of Examples having the number as Example number, it was produced using the corresponding starting materials; and when P is described in front of the number, the number represents that similar to the compounds of Production Examples having the number as Production Example, it wasproduced using the corresponding starting materials). When the number is plural, it represent that it was produced by carrying out the reaction similarly in this order. In addition, HCl in structural formulae represents hydrochloride and TFA represents trifluoro acetate (the number in front of an acid component represents the molar ratio of the acid component, for example, 2 HCl represents dihydrochloride). In addition, DBU represents 1,8-diazabicyclo[5.4.0]-7-undecene.

### Production Example 1

To a dichloromethane (9 ml) solution of 2,4,5-trifluorobenzamide (430 mg) was added oxalyl dichloride (0.3 ml) at 0°C, followed by stirring at 45°C for 4 hours. The solvent was evaporated under reduced pressure, and dioxane (8.6 ml) and cyclopentylamine (0.3 ml) were added to the resulting residues and the mixture was stirred at room temperature for 12 hours. Water was added to the resulting reaction liquid, and the liquid was extracted with ethyl acetate, followed by washing with saturated brine. The liquid was dried over anhydrous sodium sulfate and the solvent was evaporated under reduced pressure to obtain residues. The residues were washed with a mixed solvent of ethyl acetate-hexane to obtain N-[(cyclopentylamino)carbonyl]-2,4,5-trifluoroberzamide (370 mg).

### Production Example 2

To a THF (220 ml) suspension of N-[(cyclopentylamino)carbonyl]-2,4,5-trifluorobenzamide (14.76 g) was added dropwise a toluene solution (217 ml) of 0.5 M potassium bis(trimethylsilyl)amide at -20°C. The temperature was raised to room temperature. After that, 1,4,7,10,13,16-hexaoxacyclooctadecane (2.75 g) was added to the mixture and the mixture was stirred at 100°C for 8 hours. The resulting reaction liquid was added to a mixed solution of a 10% aqueous citrate solution (150 ml) and 1M hydrochloric acid (150 ml) under ice cooling and the liquid was extracted with ethyl acetate, followed by washing with water and saturated brine in this order. The liquid was dried over anhydrous sodium sulfate and the solvent was evaporated under reduced pressure to obtain residues. The residues were purified by silica gel column chromatography to obtain 9.19 g of 1-cyclopentyl-6,7-difluoroquinazoline-2,4(1H,3H)-dione.

### Production Example 3

To a DMF (5 ml) solution of 1-cyclopentyl-6,7-difluoroquinazoline-2,4(1H,3H)-dione (320 mg) were added potassium carbonate (200 mg) and ethyl bromoacetate (0.15 ml), followed by stirring at 60°C for 12 hours. Water was added to the resulting reaction liquid and insoluble materials were collected by filtration to obtain ethyl (1-cyclopentyl-6,7-difluoro-2,4-dioxo-1,4-dihydroquinazolin-3(2H)-yl)acetate (270 mg).

### Production Example 4

To a DMSO (2 ml) solution of 1-cyclopentyl-6,7-difluoroquinazoline-2,4(1H,3H)-dione (100 mg) was added cyclohexylamine (0.13 ml), followed by stirring at 100°C for 12 hours. Water was added to the resulting reaction liquid and insoluble materials were collected by filtration to obtain 7-(cyclohexylamino)-1-cyclopentyl-6-fluoroquinazoline-2,4(1H,3H)-dione (110 mg).

### Production Example 5

To a toluene (8 ml) solution of ethyl 4-(1-cyclopentyl-5,7-difluoro-2,4-dioxo-1,4-dihydroquinazolin-3(2H)-yl)butanoate (400 mg) were added benzyl alcohol (0.24 ml) and potassium carbonate (320 mg), followed by stirring at 100°C for 12 hours. Water was added to the resulting liquid, and the liquid was extracted with ethyl acetate, followed by washing with saturated brine. The liquid was dried over anhydrous sodium sulfate and the solvent was evaporated under reduced pressure to obtain residues. The residues were purified by silica gel column chromatography to obtain ethyl 4-[5-(benzyloxy)-1-cyclopentyl-7-fluoro-2,4,-dioxo-1,4-dihydroquinazolin-3(2H)-yl]butanoate (470 mg).

### Production Example 6

To a THF (40 ml) solution of 1-cyclopentyl-6,7-difluoroquinazoline-2,4(1H,3H)-dione (1.5 g) was added 320 mg of 55% sodium hydride under ice cooling, followed by stirring at room temperature for 30 minutes. O-(2,4-dinitrophenyl)hydroxylamine (1.5 g) was added to the mixture and the mixture was heated under reflux for 3 hours. After that, O-(2,4-dinitrophenyl)hydroxylamine (1.5 g) was added to the mixture and the mixture was heated under reflux for 3 hours. Ethyl acetate was added to the reaction liquid and the liquid was washed with a saturated aqueous ammonium chloride solution, water and saturated brine in this order. The liquid was dried over anhydrous sodium sulfate and the solvent was evaporated under reduced pressure to obtain residues. The residues were purified by silica gel column chromatography to obtain 3-amino-1-cyclopenyl-6,7-difluoroquinazoline-2,4,(1H,3H)-dione (700 mg).

### Production Example 7

To an acetic acid (360 ml)suspension of 2-amino-4,5-difluorobenzoicacid (10.0 g) was added cyclopentanone (20.5 ml), followed by stirring for 4 days. Sodium triacetoxyborohydride (24.6 g) and acetic acid (40 ml) were added to the reaction liquid and the liquid was stirred for 4.5 hours. Water (50 ml) was added to the liquid and the solvent was evaporated under reduced pressure until the amount of the liquid to be around 1/3. Water (300 ml) was added to the liquid and insoluble materials were collected by filtration to obtain 2-(cyclopentylamino)-4,5-difluorobenzoic acid (6.24 g).

### Production Example 8

To a DMF (15 ml) solution of 2-(cyclopentylamino)-4,5-difluorobenzoic acid (1.00 g) and ethyl 4-(aminooxy)butanoate (930 mg) were added N-[3-(dimethylamino)propyl]-N'-ethylcarbodiimide hydrochloride (1.21 g) and 1-hydroxybenzotriazol (846 mg), followed by stirring for 3 days. The solvent was evaporated under reduced pressure and a 10% citrate solution was added to the mixture. The liquid was extracted with ethyl acetate, followed by washing with water and saturated brine. The liquid was dried over anhydrous sodium sulfate and the solvent was evaporated under reduced pressure to obtain residues. The residues were purified by column chromatography to obtain ethyl 4-({[2-(cyclopentylamino)-4,5-difluorobenzoyl]amino}oxy)butanoate (1.34 g).

### Production Example 9

To a dichloromethane (30 ml) solution of ethyl 4-({[2-(cyclopentylaimino)-4,5-difluorobenzoyl]amino}oxy)butanoate (1.29 g) were added a water (16 ml) solution of potassium carbonate (972 mg) and a dichloromethane (10 ml) solution of triphosgene (526 mg), followed by stirring overnight. A saturated aqueous ammonium chloride solution was added to the reaction liquid and the liquid was extracted with chloroform and the liquid was dried over anhydrous sodium sulfate. The solvent was evaporated under reduced pressure to obtain residues. The residues were purified by column chromatography to obtain ethyl 4-[(1-cyclopentyl-6,7-difluoro-2,4-dioxo-1,4-dihydroquinazolin-3(2H)-yl)oxy]butanoate (1.06 g).

### Production Example 10

To a toluene (50 ml) solution of ethyl 4-{[2-(cyclopentylamino)-4,5-difluorobenzoyl]amino}butanoate (2.0 g) was added chloroacetyl chloride (0.67 ml) at room temperature, followed by stirring at 60°C for 8 hours. The solution was cooled down to room temperature. After that, a saturated aqueous sodium hydrogen carbonate solution was added to the reaction liquid and the liquid was extracted with ethyl acetate, followed by washing with saturated brine. The liquid was dried over anhydrous sodium sulfate and the solvent was evaporated under reduced pressure to obtain residues. The residues were purified by silica gel column chromatography to obtain ethyl 4-({2-[(chloroacetyl)(cyclopentyl)amino]-4,5-difluorobenzoyl}amino}butanoate (2.2 g).

### Production Example 11

To a dioxane (30 ml) solution of ethyl 4-({2-[(chloroacetyl)(cyclopentyl)amino]-4,5-difluorobenzoyl}amino}butanoate (2.15 g) was added 55% sodium hydride (262 mg) under ice cooling, followed by stirring at 60°C for 3 days. Under ice cooling, a saturated aqueous ammonium chloride solution was added to the reaction liquid and the liquid was extracted with ethyl acetate, followed by washing with saturated brine. The liquid was dried over anhydrous sodium sulfate and the solvent was evaporated under reduced pressure to obtain residues. The residues were purified by silica gel column chromatography to obtain ethyl 4-(1-cyclopentyl-7,8-difluoro-2,5-dioxo-1,2,3,5-tetrahydro-4H-1,4-benzodiazepin-4-yl)butanoate (1.27 g).

### Production Example 12

To a pyridine (10 ml) solution of 6,7-difluoroisoquinoline-1(2H)-one (500 mg) was added iodine (1.4 g) at room temperature, followed by stirring at 48°C for 6 hours. After cooling the solution, a saturated aqueous sodium hydrogen carbonate solution and a 10% aqueous sodium thiosulfate solution were added to the reaction liquid and the liquid was extracted with ethyl acetate, followed by washing with saturated brine. The liquid was dried over anhydrous sodium sulfate and the solvent was evaporated under reduced pressure to obtain 6,7-difluoro-4-iodoisoquinoline-1(2H)-one (659 mg).

### Production Example 13

To a DMF (5 ml) solution of ethyl (6,7-difluoro-4-iodo-1-oxoisoquinolin-2(1H)-yl)acetate (200 mg) were added potassium acetate (150 mg), tetrabutylammonium chloride (142 mg), cyclopentene (0.45 ml) and palladium acetate (57 mg) at room temperature, followed by stirring at 80°C for overnight in a sealed tube. After cooling the mixture, water and ethyl acetate were added to the reaction liquid and insoluble materials were removed through celite, thereby carrying out a liquid-separating operation. An organic layer was washed with saturated brine and dried over anhydrous sodium sulfate. After that, filtration was carried out and the solvent was evaporated under reduced pressure. The resulting residues were purified by silica gel column chromatography to obtain ethyl (4-cyclopent-2-en-1-yl-6,7-difluoro-1-oxoisoquinolin-2(1H)-yl)acetate (162 mg).

### Production Example 14

To a THF (30 ml) suspension of 6,7-difluoro-4-iodoisoquinoline-1(2H)-one (1.6 g) were added a THF (5 ml) solution af DBU (2.8 ml) and a THF (5 ml) solution of [2-(chloromethoxy)ethyl](trimethyl)silane (2.3 ml) at -78°C, followed by stirring for 2 hours and 40 minutes. 1M of hydrochloric acid was added to the reaction liquid and the liquid was extracted with ethyl acetate, followed by washing with water and saturated brine. The liquid was dried over anhydrous sodium sulfate and the solvent was evaporated under reduced pressure. The resulting solution was purified by silica gel column chromatography to obtain 6,7-difluoro-4-iodo-2-{[2-(trimethylsilyl)ethoxy]methyl}isoquinoline-1(2H)-one (1.0 g).

### Production Example 15

To a THF (7 ml) solution of 4-cyclopent-2-en-1-yl-6,7-difluoro-2-{[2-(trimethylsilyl)ethoxy]methyl}isoquinoline-1(2H)-one (1.0 g) was added tetrabutylammonium fluoride hydrate (4.8 g), followed by stirring for 12 hours. The solvent was evaporated under reduced pressure and the residues were purified by silica gel column chromatography to obtain 4-cyclopent-2-en-1-yl-6,7-difluoroisoquinoline-1(2H)-one (400 mg).

### Production Example 16

Methyl (2E,4R)-4-[(tert-butoxycarbonyl)amino]pent-2-enoate (2.76 g) was dissolved in chloroform (15 ml). Trifluoroacetic acid (15 ml) was added to the mixture at room temperature and the mixture was stirred for 4 hours. The reaction liquid was concentrated under reduced pressure, and ethyl acetate and diethyl ether were added to the resulting residues. The resulting insoluble materials were collected by filtration to obtain methyl (2E,4R)-4-aminopent-2-enoate trifluoroacetate (2.93 g).

### Production Example 17

To a toluene (6 ml) solution of tert-butyl [(2-hydroxyethyl)(phenyl)amino]acetate (380 mg) were added p-toluenesulfonyl chloride (320 mg), triethylamine (0.25 ml) and trimethylamine hydrochloride (15 mg), followed by stirring overnight. In addition, p-toluenesulfonyl chloride (144 mg) and trimethylamine (0.13 ml) were added to the liquid and the liquid was stirred overnight. Water was added to the resulting reaction liquid and the solution was extracted with ethyl acetate, followed by washing with saturated brine. The liquid was dried over anhydrous sodium sulfate and the solvent was evaporated under reduced pressure. The resulting residues were purified by silica gel column chromatography to obtain tert-butyl [(2-{[(4-methylphenyl)sulfonyl]oxy}ethyl)(phenyl)amino]acetate (460 mg).

In the same manner as in the methods in Production Examples 1 to 17 and Examples described below, compounds of Production Examples 18 to 51 shown in Tables described below were produced. Structures, production methods, and physicochemical data of the compounds of Production Examples are shown in Tables 4 to 11.

### Example 1

To a THF (6.2 ml) and ethanol (3.1 ml) mixed solution of ethyl [7-(cyclohexylamino)-1-cyclopentyl-6-fluoro-2,4-dioxo-1,4-dihydroquinazolin-3(2H)-yl]acetate (310 mg) was added a 1M aqueous sodium hydroxide solution (2.1 ml), and the mixed solution was stirred at room temperature for 12 hours. To the resulting reaction liquid were added a 1M aqueous hydrochloric acid solution (2.1 ml) and water. The insoluble materials were collected by filtration to obtain [7-(cyclohexylamino)-1-cyclopentyl-6-fluoro-2,4-dioxo-1,4-dihydroquinazolin-3(2H)-yl]acetate (265 mg).

### Examples 2 and 3

To a ethyl acetate (3 ml) and ethanol (3 ml) mixed solution of ethyl [6-(cyclohexylamino)-4-cyclopent-2-en-1-yl-7-fluoro-1-oxoisoquinoline-2(1H)-yl]acetate (90 mg) was added 10% palladium-carbon (15 mg), and the solution was stirred over night at room temperature under a hydrogen atmosphere. The solution was filtered through celite and the solvent was evaporated under reduced pressure. The resulting residues were purified by silica gel column chromatography to obtain ethyl [6-(cyclohexylamino)-4-cyclopentyl-7-fluoro-1-oxoisoquinoline-2(1H)-yl]acetate (Example 2) (48 mg) and ethyl [6-(cyclohexylamino)-4-cyclopentyl-7-fluoro-1-oxo-3,4-dihydroisoquinoline-2(1H)-yl]acetate (Example 3) (35 mg) respectively.

### Example 4

To a dichloromethane (2 ml) solution of 7-(cycolohexylamino)-1-cyclopentyl-6-fluoroquinazoline-2,4,(1H,3H)-dione (100 mg)were added triphenylphosphine (150 mg), (2,2-dimethyl-1,3-dioxolan-4-yl)methanol (0.07 ml) and a 2.2 M diethyl azodicarboxylate toluene solution (0.26 m), and the mixture was stirred for 12 hours. The solvent was evaporated under reduced pressure, and the resulting residues were purified by silica gel column chromatography to obtain 7-(cyclohexylamino)-1-cyclopentyl-3-[(2,2-dimethyl-1,3-dioxolan-4-yl)methyl]-6-fluoroquinazoline-2,4 (1H,3H)-dione (50 mg).

### Example 5

To 7-(cyclohexylamino)-1-cyclopentyl-3-[(2,2-dimethyl-1,3-dioxolan-4-yl)methyl]-6-fluoroquinazoline-2,4,(1H,3H)-dione (50 mg) was added a 90% aqueous acetic acid solution (1.2 ml) and the mixture was stirred at 90°C for 12 hours. After cooling the mixture to room temperature, water was added to the mixture. The insoluble materials were collected by filtration to obtain 7-(cyclohexylamino)-1-cyclopentyl-3-[(2,3-dihydroxylpropyl)-6-fluoroquinazoline-2,4(1H,3H)-dione (25 mg).

### Example 6

To a dichloromethane (2 ml) solution of diethyl {[7-(cyclohexylamino)-1-cyclopentyl-6-fluoro-2,4-dioxo-1,4-dihydroquinazolin-3(2H)-yl]methyl}}phosphonate (80 mg) was added bromotrimethylsilane (0.1 ml), and the mixture was stirred for 12 hours. To the mixture were added DMF (2 ml) and bromotrimethylsilane (0.1 ml), and the mixture was stirred at 60°C for 12 hours. To the resulting reaction liquid was added methanol and the solvent was evaporated under reduced pressure. To the resulting residues were added ethyl acetate and a saturated aqueous sodium hydrogen carbonate solution to carry out a liquid-separating operation. To a water layer were added 1M hydrochloric acid and water and the insoluble materials were collected by filtration to obtain {[7-(cyclohexylamino)-1-cyclopentyl-6-fluoro-2,4-dioxo-1,4-dihydroquinazolin-3(2H)-yl]methyl}}phosphonate (31 mg). [0091]

### Example 7

To a DMF (4 ml) solution of 4-[7-(cyclohexylamino)-I-cyclopentyl-6-fluoro-2,4-dioxo-1,4-dihydroquinazolin-3(2H)-yl]acetate (200 mg) was added 1,1'-carbonyldiimidazole (120 mg), and the mixture was stirred overnight. To the resulting reaction liquid was added water and the insoluble materials were collected by filtration. To the resulting solid were added THF (3 ml), water (2 ml) and sodium borohydride (30 mg), and the mixture was stirred for 3 hours. The solvent was evaporated under reduced pressure. To the reaction liquid was poured water, and the insoluble materials were collected by filtration to obtain 7-(cyclohexylamino)-1-cyclopentyl-6-fluoro-3-(4-hydroxyethyl)quinazoline-2,4(1H,3H)-dione (87 mg).

### Example 8

To a DMSO (2 ml) solution of 7-(cyclohexylamino)-1-cyclopentyl-6-fluoro-3-(4-hydroxybutyl)quinazoline-2,4(1H,3H)-dione (55 mg) were added a sulfur trioxidepyridine complex (63 mg) and triethylamine (0.1 ml), and the mixture was stirred for 12 hours. To the resulting reaction liquid was added water, and the insoluble materials were collected by filtration to obtain 4-[7-(cyclohexylamino)-1-cyclopentyl-6-fluoro-2,4-dioxo-1,4-dihydroquinazolin-3(2H)-yl]butanal (50 mg).

### Example 9

To a DMF (1 ml) solution of 4-[7-(cyclohexylamino)-1-cyclopentyl-6-fluoro-2,4-dioxo-1,4-dihydroquinazolin-3(2H)-yl]butanal (50 mg) were added potassium carbonate (50 mg) and ethyl (diethoxyphosphoryl)acetate (0.07 ml), the mixture was stirred at 60°C for 12 hours. To the resulting reaction liquid was added water, and the insoluble materials were collected by filtration to obtain ethyl (2E)-6-[7-(cyclohexylamino)-1-cyclopentyl-6-fluoro-2,4-dioxo-1,4-dihydroquinazolin-3(2H)-yl]hex-2-enoate (100 mg).

### Example 10

To a DMF (2 ml) solution of ethyl 2-(diethoxyphosphoryl)propanoate(0.09 ml) was added 55% sodium hydride (17 mg) under ice cooling, and the mixture was stirred for 30 minutes. To the resulting reaction liquid was added [7-(cyclohexylamino)-1-cyclopentyl-6-fluoro-2,4-dioxo-1,4-dihydroquinazolin-3(2H)-yl]acetaldehyde (100 mg), and the mixture was stirred at room temperature for 12 hours. To the resulting liquid was added water, and the insoluble materials were collected by filtration and then purified by silica gel column chromatography to obtain ethyl (2E)-4-[7-(cyclohexylamino)-1-cyclopentyl-6-fluoro-2,4-dioxo-1,4-dihydroquinazolin-3(2H)-yl]-2-methylbut-2-enoate (11mg).

### Example 11

To tert-butyl {2-[7-(cyclohexylamino)-1-cyclopentyl-6-fluoro-2,4-dioxo-1,4-dihydroquinazolin-3(2H)-yl]ethyl}carbamate (200 mg) was added 4 M hydrogen chloride ethyl acetate solution (2 ml), and the mixture was stirred for 12 hours. To the resulting reaction liquid was added a saturated aqueous sodium hydrogen carbonate solution, and the mixture was extracted with ethyl acetate, followed by washing with saturated brine. The resulting mixture was dried over anhydrous sodium sulfate and the solvent was evaporated under reduced pressure to obtain residues. The residues were washed with a mixed solvent of ethyl acetate-hexane to obtain 3-(2-aminoethyl)-7-(cyclohexylamino)-1-cyclopentyl-6-fluoroquinazolin-2,4(1H,3H)-dione (160 mg).

### Example 12

To a DMF (4.0 ml) solution of [7-(cyclohexylamino)-1-cyclopentyl-6-fluoro-2,4-dioxo-1,4-dihydroquinazolin-3(2H)-yl]acetic acid (200 mg) was added 1,1'-carbonyldiimidazole (120 mg), and the mixture was stirred for 12 hours. To the resulting reaction liquid was added cold water and the insoluble materials were collected by filtration. The resulting insoluble materials were dissolved in dioxane (4 ml) and then DBU (0.085 ml) and 3-(aminosulfonyl)propyl acetate (100 mg) were added, and the mixture was stirred at 60°C for 12 hours. To the resulting reaction liquid was added water and the liquid was extracted with ethyl acetate, followed by washing with saturated brine. The resulting liquid was dried over anhydrous sodium sulfate and the solvent was evaporated under reduced pressure to obtain residues. The residues were purified by silica gel column chromatography to obtain 3-[({2-[7-(cyclohexylamino)-1-cyclopentyl-6-fluoro-2,4-dioxo-1,4-dihydroquinazolin-3(2H)-yl]acetyl}amino)sulfonyl]propyl acetate (240 mg).

### Example 13

To an ethanol (2 ml) solution of 4-[7-(cyclohexylamino)-1-eyelopentyl-6-fluoro-2,4-dioxo-1,4-dihydroquinazolin-3(2H)-yl]-N-(tetrahdro-2H-pyran-2-yloxy)butanamide (100 mg) was added p-toluenesulfonic acid hydrate (40 mg), and the mixture was stirred for 12 hours. To the resulting reaction liquid was added water and the insoluble materials were collected by filtration to obtain 4-[7-(cyclohexylamino)-1-cyclopentyl-6-fluoro-2,4-dioxo-1,4-dihydroquinzolin-3(2H)-yl]-N-hydroxybutanamide (73 mg).

### Example 14

To a DMF (5 ml) solution of (2E)-4-[7-(cyclohexylamino)-1-cyclopentyl-6-fluoro-2,4-dioxo-1,4-dihydroquinazolin-3(2H)-yl]-2-butenoic acid (120 mg) was added 1,1'-carbonyldiimidazole (60 mg) under ice cooling, and the mixture was stirred at the same temperature for 2 hours. At the same temperature, to the mixture was added a hydrazine hydrate (60 µl), and the mixture was stirred at room temperature for 2 hours. To the resulting liquid were added water and a saturated aqueous sodium hydrogen carbonate solution, and the liquid was extracted with ethyl acetate, followed by washing with saturated brine. The resulting liquid was dried over anhydrous sodium sulfate and the solvent was evaporated under reduced pressure to obtain residues. The residues were purified by silica gel column chromatography to obtain (2E)-4-[7-(cyclohexylamino)-1-cyclopentyl-6-fluoro-2,4-dioxo-1,4-dihydroquinazolin-3(2H)-yl]but-2-enohydrazide (60 mg).

### Example 15

To a ethanol (4 ml) solution of (2E)-4-[7-(cyclohexylamino)-1-cyclopentyl-6-fluoro-2,4-dioxo-1,4-dihydroquinazolin-3(2H)-yl]but-2-enohydrazide (60 mg) were added carbon disulfide (30 µl) and potassium hydroxide (10 mg) under ice cooling, and the mixture was stirred under ice cooling for 30 minutes and at room temperature for 1 hour and was heated under reflux for 6 hours. To the resulting reaction liquid was added water and the liquid was washed with diethyl ether. To a water layer was added 1M hydrochloric acid under ice cooling and the water layer was extracted with ethyl acetate, followed by washing with saturated brine. The resulting mixture was dried over anhydrous sodium sulfate and the solvent was evaporated under reduced pressure to obtain residues. The residues were purified by silica gel column chromatography to obtain 7-(cyclohexylamino)-1-cyclopentyl-6-fluoro-3-[(2E)-3-(5-thioxo-4,5-dihydro-1,3,4-oxadiazol-2-yl)prop-2-en-1-yl]quinazoline-2,4(1H,3H)-dione (58 mg).

### Example 16

To a 2-propanol (4 ml) solution of ethyl (2E)-4-[7-(cyclohexylamino)-1-cyclopentyl-6-fluoro-2,4-dioxo-1,4-dihydroquinazolin-3(2H)-yl]but-2-enoate (200 mg) were added bis(2,4-pentanedionate)manganese(II) (2.2 mg) and phenylsilane (0.1 ml), and the mixture was stirred under oxygen atmosphere at 60°C for 12 hours. The resulting reaction liquid was cooled down to room temperature and subjected to celite filtration, and the solvent was evaporated under reduced pressure. The resulting residues were purified by silica gel column chromatography to obtain ethyl 4-[7-(cyclohexylamino)-1-cyclopentyl-6-fluoro-2,4-dioxo-1,4-dihydroquinazolin-3(2H)-yl]-2-hydroxybutanoate (120 mg).

### Example 17

To a DMF (1.25 ml) solution of ethyl 4-[7-(cyclohexylamino)-1-cyclopentyl-6-fluoro-2,4-dioxo-1,4-dihydroquinazolin-3(2H)-yl]-2-hydroxybutanoate (50 mg) were added silver oxide (I) (120 mg) and iodomethane (0.03 ml), and the mixture was stirred for 12 hours. The insoluble materials were collected by filtration and the solvent was evaporated under reduced pressure. The resulting residues were purified by silica gel column chromatography to obtain ethyl 4-[7-(cyclohexylamino)-1-cyclopentyl-6-fluoro-2,4-dioxo-1,4-dihydroquinazolin-3(2H)-yl]-2-methoxybutanoate (18 mg).

### Example 18

To a dioxane (10 ml) solution of 4-[7-(cyclohexylamino)-1-cyclopentyl-6-fluoro-2,4-dioxo-1,4-dihydroquinazolin-3(2H)-yl]butanenitrile (80 mg) was added tributyltin azide (0.25 ml), and the mixture was heated under reflux for 12 hours. To the reaction liquid was added 1M aqueous sodium hydroxide solution, and the liquid was washed with diethyl ether. To a water layer was added 1M hydrochloric acid, and the water layer was extracted with chloroform, followed by washing with saturated brine. The resulting mixture was dried over anhydrous sodium sulfate and the solvent was evaporated under reduced pressure to obtain residues. The residues were purified by silica gel column chromatography and washed using diethyl ether to obtain 7-(cyclohexylamino)-1-cyclopentyl-6-fluoro-3-[3-(1H-tetrazole-5-yl)propyl]quinazoline-2,4(1H,3H)-dione (55 mg).

### Example 19

To an acetone (8 ml) solution of 7-(cyclohexylamino)-1-cyclopentyl-3-(1,3-diaxolan-2-ylmethyl)-6-fluoroquinazoline-2,4(1H,3H)-dione (340 mg) an acetone solution (8 ml) was added 1M hydrochloric acid (0.8 ml), and the mixture was stirred at 60°C for 12 hours. To the resulting reaction liquid was added water and the insoluble materials were collected by filtration to obtain [7-(cyclohexylamino)-1-cyclopentyl-6-fluoro-2,4-dioxo-1,4-dihydroquinazolin-3(2H)-yl]-acetaldehyde (110 mg).

### Example 20

To a DMF (4 ml) solution of 55% sodium hydride (50 mg) a DMF solution (4 ml) was added benzyl alcohol (1 ml), and the mixture was stirred for 30 minutes. To the resulting reaction liquid was added ethyl (2E)-4-[7-(cyclohexylamino)-1-cyclopentyl-6-fluoro-2,4-dioxo-1,4-dihydroquinazolin-3(2H)-yl]but-2-enoate (200 mg), and the mixture was stirred for 12 hours. The resulting reaction liquid was neutralized by 1M aqueous hydrochloric acid solution and 1M aqueous sodium hydroxide solution was added to the liquid and stirred for one night. To the resulting reaction liquid were added 1M hydrochloric acid and water, and the liquid was extracted with ethyl acetate, followed by washing with saturated brine. The liquid was dried over anhydrous sodium sulfate and the solvent was evaporated under reduced pressure to obtain residues. The residues were washed with a mixed solvent of ethyl acetate-hexane to obtain 3-(benzyloxy)-4-[7-(cyclohexylamino)-1-cyclopentyl-6-fluoro-2,4-dioxo-1,4-dihydroquinazolin-3(2H)-yl]butanoic acid (150 mg).

### Example 21

To an ethyl 2(S)-2-hydroxypropanoate (3.0 ml) solution of 7-(cyclohexylamino)-1-cyclopentyl-5-fluoro-3-(2-hydroxyethyl)quinazoline-2,4(1H,3H)-dione (145 mg) was added tosilic acid hydrate (100 mg), and the mixture was stirred at 120°C for 12 hours. To the resulting reaction liquid was added a saturated aqueous sodium hydrogen carbonate solution, and the liquid was extracted with chloroform, followed by washing with saturated brine. The liquid was dried over anhydrous sodium sulfate and the solvent was evaporated under reduced pressure to obtain residues. The residues were purified by silica gel column chromatography to obtain ethyl 2-{2-[7-(cyclohexylamino)-1-cyclopentyl-6-fluoro-6,4-dioxo-1,4-dihydroquinazolin-3(2H)-yl]ethoxy}propanoic acid. To the resulting compound were added ethanol (1.0 ml), THF (2.0 ml), and a 1M aqueous sodium hydroxide solution (1 ml), and the mixture was stirred at room temperature over night. To the resulting reaction liquid were added water and 1M hydrochloric acid, and the liquid was extracted with chloroform, followed by washing with saturated brine. The liquid was dried over anhydrous sodium sulfate and the solvent was evaporated under reduced pressure to obtain residues. The residues were purified by silica gel column chromatography to obtain 2-{2-[7-(cyclohexylamino)-1-eyclopentyl-6-fluoro-2,4-dioxo-1,4-dihydroquinazolin-3(2H)-yl]ethoxy}prapanoic acid (44 mg).

### Example 22

To a pyridine (2.0 ml) solution of ethyl ({2-[7-(cyclohexylamino)-1-cyclopentyl-6-fluoro-2,4-dioxo-1,4-dihydroquinazolin-3(2H)-yl]ethyl}amino)acetate (100 mg) was added acetic anhydride (30 µl), and the mixture was stirred at room temperature for 12 hours. To the resulting reaction liquid was added 1M hydrochloric acid, and the liquid was extracted with ethyl acetate, followed by washing with saturated brine. The liquid was dried over anhydrous sodium sulfate and the solvent was evaporated under reduced pressure to obtain ethyl (acetyl{2-[7-(cyclohexylamino)-1-cyclopentyl-6-fluoro-2,4-dioxo-1,4-dihydroquinazolin-3(2H)-yl]ethyl}amino)acetate (80 mg).

### Example 23

To a THF (3.0 ml) solution of 3-(2-aminoethyl)-7-(cyclohexylamino)-1-cyclopentyl-6-fluoroquinazoline-2,4(1H,3H)-dione (150 mg) were added ethyl chloro(oxo)acetate (50 µl) and triethylamine (70 µl), and the mixture was stirred at room temperature for 12 hours. To the resulting reaction liquid was added water, and the insoluble materials were collected by filtration to obtain ethyl ({2-[7-(cyclohexylamino)-1-cyclopentyl-6-fluoro-2,4-dioxo-1,4-dihydroquinazolin-3(2H)-yl]ethyl}amino)(oxo)acetate (160 mg).

### Example 24

To a DMSO (7.8 ml) solution of tert-butyl [(1-cyclopentyl-6,7-difluoro-2,4-dioxo-1,4-dihydroquinazolin-3(2H)-yl)oxy]acetate (310 mg) was added cyclohexylamine (0.5 ml), and the mixture was stirred at 80°C overnight. To the resulting reaction liquid was added an aqueous citrate solution and the liquid was extracted with ethyl acetate, washed with water and saturated brine, and then dried over anhydrous sodium sulfate. The solvent was evaporated under reduced pressure. The resulting residues were purified by silica gel column chromatography to obtain tert-butyl {[7-(cyclohexylamino)-1-cyclopentyl-6-fluoro-2,4-dioxo-1,4-dihydroquinazolin-3(2H)-yl]oxy} acetate (140 mg). To a dichloromethane solution (4 ml) of the resulting compound (130 mg) was added dropwise trifluoroacetic acid (4 ml) under ice cooling, and the mixture was stirred at room temperature for 2 hours. The solvent was evaporated under reduced pressure. After that, to the resulting liquid were added chloroform, a saturated aqueous sodium hydrogen carbonate solution and an aqueous citrate solution, and the liquid was extracted with chloroform, followed by washing with saturated brine. The liquid was dried over anhydrous sodium sulfate. After that, the solvent was evaporated under reduced pressure. The resulting residues were washed with diisopropyl ether to obtain {[7-(cyclohexylamino)-1-cyclopetyl-6-fluoro-2,4-dioxo-1,4-dihydroquinazolin-3(2H)-yl]oxy}acetic acid (116 mg).

### Example 25

To a THF (6.0 ml) solution of ethyl ({2-[7-(cyclohexylamino)-1-cyclopentyl-6-fluoro-2,4-dioxo-1,4-dihydroquinazolin-3(2H)-yl]ethyl}amino)acetate (292 mg) were added potassium carbonate (120 mg) and methyl iodide (0.05 ml), and the mixture was stirred at room temperature for 12 hours. To the resulting reaction liquid was added water, and the liquid was extracted with ethyl acetate, followed by washing with saturated brine. The liquid was dried over anhydrous sodium sulfate. After that, the solvent was evaporated under reduced pressure. The resulting residues were purified by silica gel column chromatography to obtain ethyl [{2-[7-(cyclohexylamino)-1-cyclopentyl-6-fluoro-2,4-dioxo-1,4-dihydroquinazolin-3(2H)-yl]ethyl}(methyl)amino]acetate. To the resulting compound was added 4M hydrogen chloride ethyl acetate solution, and the mixture was stirred at room temperature for 12 hours. To the resulting liquid was added hexane, and the insoluble materials were collected by filtration to obtain ethyl [{2-[7-(cyclohexylamino)-1-cyclopentyl-6-fluoro-2,4-dioxo-1,4-dihydroquinazolin-3(2H)-yl]ethyl}(methyl)amino]acetate hydrochloride (228 mg).

### Example 26

To an ethyl acetate (15 ml) solution of ethyl (2E)-4-{[7-(cyclohexylamino)-1-cyclopentyl-6-fluoro-2,4-dioxo-1,4-dihydroquinazolin-3(2H)-yl]amino}but-2-enoate (348 mg) was added 10% palladium-carbon (80 mg), and the mixture was stirred under hydrogen atmosphere at room temperature for 2 hours. The mixture was filtered through celite and the solvent was evaporated under reduced pressure. The resulting residues were purified by silica gel column chromatography and then washed with a mixed solvent of ethyl acetate-diisopropyl ether to obtain ethyl (2E)-4-{[7-(cyclohexylamino)-1-cyclopentyl-6-fluoro-2,4-dioxo-1,4-dihydroquinazolin-3(2H)-yl]amino}butanoate (264 mg).

### Example 27

To a THF (4 ml) and ethyl acetate (4 ml) mixed solution of benzyl ethyl 2,2'({[7-(cyclohexylamino)-1-cyclopentyl-6-fluoro-2,4-dioxo-1,4-dihydroquinazolin-3(2H)-yl]acetyl}imino)diacetate (200mg) was added 10% palladium-carbon (50 mg), and the mixture was stirred under hydrogen atmosphere at room temperature overnight. The mixture was filtered through celite and the solvent was evaporated under reduced pressure. To the resulting residues was added ethyl acetate and the insoluble materials were collected by filtration to obtain [{[7-(cyclohexylamino)-1-cyclopentyl-6-fluoro-2,4-dioxo-1,4-dihydroquinazolin-3(2H)-yl]acetyl}(2-ethoxy-2-oxoethyl)amino]acetic acid (100 mg).

### Example 28

To a THF (1.0 ml) solution of 3-(2-aminoethyl)-7-(cyc1ohexylamino)-1-cyclopentyl-6-fluoroquinazolinc-2,4(1H,3H)-dione (50 mg) were added 1H-1,2,4-triazole-3-sulfonyl chloride (25 mg) and triethylamine (25 µl) at room temperature, and the mixture was stirred for 12 hours. To the resulting reaction liquid was added water, and the insoluble materials were collected by filtration to obtain N-{2-[7-(cyclohexylamino)-1-cyclopentyl-6-fluoro-2,4-dioxo-1,4-dihydroquinazolin-3(2H)-yl]ethyl}-1H-1,2,4-triazole-3-sulfonamide (58 mg).

### Example 29

To a DMF (1.0 ml) solution of ethyl ({2-[7-(cyclohexylamino)-1-cyclopentyl-6-fluoro-2,4-dioxo-1,4-dihydroquinazolin-3(2H)-yl]ethyl}amino)acetate (50 mg) were added carbon disulfide (10 µl) and potassium carbonate (22 mg), and the mixture was stirred at room temperature for 12 hour. To the resulting reaction liquid was added iodomethane (10 µl), and the mixture was stirred at room temperature for 2 hours. To the liquid was added water and the insoluble materials were collected by filtration to obtain ethyl ({2-[7-(cyclohexylamino)-1-cyclopentyl-6-fluoro-2,4-dioxo-1,4-dihydroquinazolin-3(2H)-yl]ethyl}[(methylsulfanyl)carbothioyl]amino)acetate (50 mg).

### Example 30

To a pyridine (5 ml) solution of 3-amino-7-(cyclohexylamino)-1-cyclopentyl-6-fluoroquinazoline-2,4(1H,3H)-dione (152 mg) was added dropwise ethyl 4-chloro-4-oxobutanoate (65 µl) under ice cooling, and the mixture was stirred under ice cooling for 1 hour and at room temperature for 3 hours. Under ice cooling, ethyl 4-chloro-4-oxobutanoate (100 µl) was added dropwise to the resulting mixture and the mixture was stirred under ice cooling for 30 minutes and at room temperature for 1 hour. The solvent was evaporated under reduced pressure. To the resulting mixture was added a saturated aqueous ammonium chloride solution and the mixture was extracted with ethyl acetate, followed by washing with the saturated aqueous ammonium chloride solution, water and saturated brine in this order. The mixture was dried over anhydrous sodium sulfate. After that, the solvent was evaporated under reduced pressure. The resulting residues were purified by silica gel column chromatography to obtain ethyl 4-{[7-(cyclohexylamino)-1-cyclopentyl-6-fluoro-2,4-dioxo-1,4-dihydroquinazolin-3(2H)-yl]amino}-4-oxobutanoate (199 mg).

### Example 31

A pyridine (20 ml)suspension of 3-amino-7-(cyclohexylamino)-1-cyclopentyl-6-fluoroquinazoline-2,4(1H,3H)-dione (1.01 g) was added dropwise a dichloromethane (9 ml) solution of benzyl chloroformate (600 µl) under ice cooling, and the mixture was stirred under ice cooling for 45 minutes and at room temperature for 45 minutes. Under ice cooling, to the mixture was added dropwise a dichloromethane (6 ml)solution of benzyl chloroformate (600 µl), and the mixture was stirred under ice cooling for 30 minutes. Under ice cooling, to the mixture was added dropwise a dichloromethane (4 ml) solution of benzyl chloroformate (400 µl) and the mixture was stirred under ice cooling for 30 minutes. The solvent was evaporated under reduced pressure. To the resulting residues was added a 10% aqueous citrate solution. The insoluble materials were collected by filtration, washed with water and hexane, and dried to obtain a powder (1.53 g). To a THF (18.8 ml) suspension liquid of the resulting powder (936 mg) was added a 1M aqueous sodium hydroxide solution, and the mixture was stirred at room temperature for 3.5 hours. To the mixture were added water (18.8 ml), 1M hydrochloric acid (6.0 ml) and water (18.8 ml) in this order, and then the mixture was stirred for 30 minutes. The insoluble materials were collected by filtration, washed with water and hexane in this order, and then dried to obtain benzyl [7-(cyclohexylamino)-1-cyclopentyl-6-fluoro-2,4-dioxo-1,4-dihydroquinazolin-3(2H)-yl]carbamate (842 mg).

### Example 32

To a DMF (6 ml) suspension of benzyl [7-(cyclohexylamino)-6-fluoro-1-opropyl-2,4-dioxo-1,4-dihydroquinazol-3(2H)-yl]carbamate (263 mg) and potassium carbonate (310 mg) were added ethyl 5-bromopentanoate (110 µl) and benzyltriethylammonium chloride (31 mg), and the mixture was stirred at room temperature overnight. To the resulting reaction liquid was added water, the liquid was extracted with ethyl acetate, washed with water and saturated brine in this order, and then dried over anhydrous sodium sulfate. The solvent was evaporated under reduced pressure. The resulting residues were purified by silica gel column chromatography to obtain ethyl 5-{[(benzyloxy)carbonyl][7-(cyclohexylamino)-6-fluoro-1-isopropyl-2,4-dioxo-1,4-dihydroquinazolin-3(2H)-yl]amino}pentanoate (273 mg).

### Example 33

To a ethanol (8 ml) solution of ethyl 5-{[(benzyloxy)carbonyl][7-(cyclohexylamino)-6-fluoro-1-isopropyl-2,4-dioxo-1,4-dihydroquinazolin-3(2H)-yl]amino}pentanoate (266 mg) was added 10% palladium-carbon (66 mg), and the mixture was stirred under hydrogen atmosphere for 2.5 hours. The mixture was filtered through celite and the solvent of the filtrate was evaporated under reduced pressure to obtain ethyl 5- {[7-(cyclohexylamino)-6-fluoro-1-isopropyl-2,4-dioxo-1,4-dihydroquinazlin-3(2H)-yl]amino}pentanoate (215 mg).

### Example 34

To tert-butyl 3-[{2-[7-(cyclohexylamino)-1-cyclopentyl-6-fluoro-2,4-dioxo-1,4-dihydroquinazolin-3(2H)-yl]ethyl}(methyl)amino]propanoate (100 mg) was added a 4M hydrogen chloride ethyl acetate solution (1 ml), and the mixture was stirred at room temperature overnight. The insoluble materials were collected by filtration to obtain 3-[{2-[7-(cyclohexylamino)-1-cyclopentyl-6-fluoxo-2,4-dioxo-1,4-dihydroquinazolin-3(2H)-yl]ethyl}(methyl)amino]propanoic acid hydrochloride (16 mg).

### Example 35

To a dichloromethane (1 ml) solution of ethyl ((2-[7-(cyclohexylamino)-1-cyclopentyl-6-fluoro-2,4-dioxo-1,4-dihydroquinazolin-3(2H)-yl]ethyl}amino)acetate (50 mg) were added triethylamine (20 µl) and methyl chloroformate (10 µl), and the mixture was stirred overnight. To the resulting reaction liquid was added water, and the liquid was extracted with chloroform, washed with a saturated aqueous sodium hydrogen carbonate solution, and then dried over anhydrous sodium sulfate. The solvent was evaporated under reduced pressure to obtain ethyl [{2-[7-(cyclohexylamino)-1-cyclopentyl-6-fluoro-2,4-dioxo-1,4-dihydroquinazolin-3(2H)-yl]ethyl}(methoxycarbonyl)amino]acetate (54 mg)

### Example 36

To tert-butyl {2-[7-(cyclohexylamino)-6-fluoro-1-isopropyl-2,4-dioxo-1,4-dihydroquinazolin-3(2H)-yl]ethyl}carbamate (1.5 g) was added a 4M hydrogen chloride ethyl acetate solution (30 ml), and the mixture was stirred overnight. To the resulting reaction liquid was added hexane (50 ml), and the insoluble materials were collected by filtration to obtain 3-(2-aminoethyl)-7-(cyclohexylamino)-6-fluoro-1-isopropylquinazolin-2,4(1H,3H)-dione dihydrochloride (1.2 g).

In the same manner as in the methods in Examples 1 to 36 and the above-mentioned Production Examples, compounds of Examples 37 to 316 shown in Tables described below were produced using the respectively corresponding starting materials. Structures of each of the compounds of Examples are shown in Tables 12 to 62, and physicochemical data and the production methods thereof are shown in Tables 63 to 72.

In addition, in Tables 73 to 75, structures of the other compounds of the invention are shown. It is possible to easily synthesize these compounds according to the methods described in the above-mentioned Production Methods and Examples, the method obvious to the person skilled in the art, or the modified methods thereof.

**[Table 4]**

| PEx | Syn | Structure | Data |
|---|---|---|---|
| 12 | P12 | 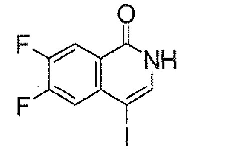 | FAB+: 308 |
| 18 | P3 | 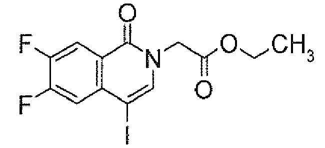 | FAB+: 394 |
| 13 | P13 | 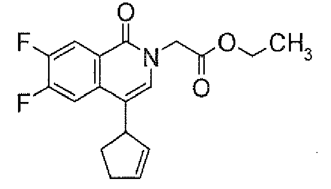 | FAB+: 334 |
| 1 | P1 | 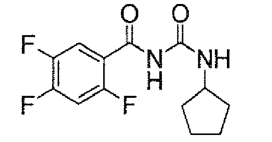 | FAB+: 287 |
| 2 | P2 | 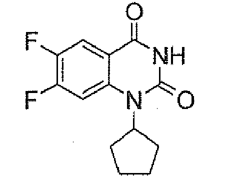 | FAB+: 267 |
| 3 | P3 | 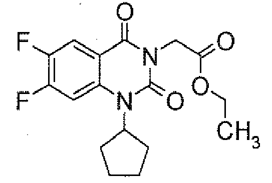 | FAB+: 353 |
| 19 | P3 | 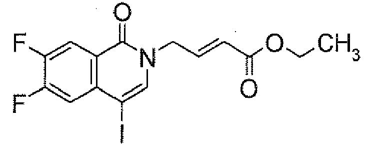 | FAB+: 420 |

**[Table 5]**

| | | | |
|---|---|---|---|
| 20 | P13 | 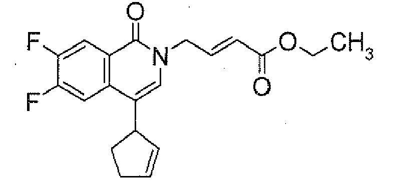 | FAB+: 360 |
| 21 | P3 | 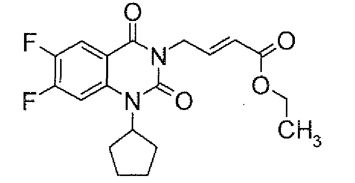 | FAB+: 379 |
| 22 | 26 | 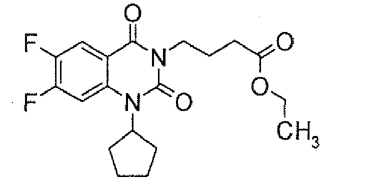 | FAB+: 381 |
| 7 | P7 | 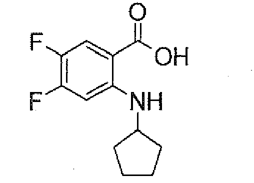 | EI: 241 |
| 23 | P2 | 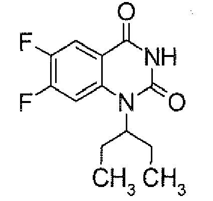 | ESI+: 269 |
| 14 | P14 | 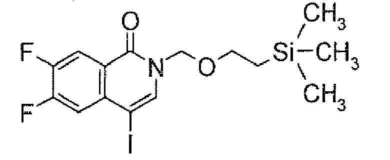 | FAB+: 438 |
| 24 | P1 | 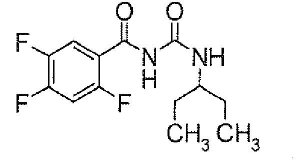 | FAB+: 289 |

**[Table 6]**

| | | | |
|---|---|---|---|
| 25 | P3 | 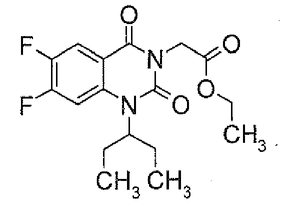 | FAB+: 355 |
| 26 | P1 | 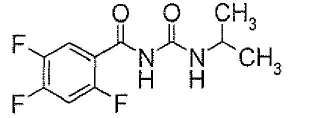 | FAB+: 261 |
| 27 | P2 | 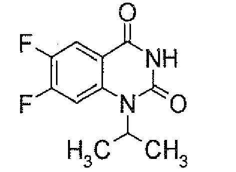 | ESI+: 241 |
| 28 | P3 | 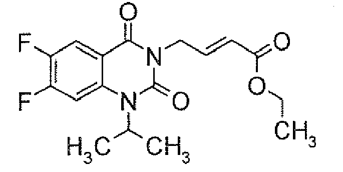 | FAB+: 353 |
| 29 | P1 | 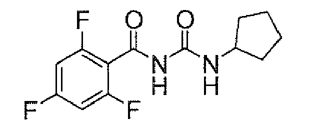 | FAB+: 287 |
| 30 | 26 | 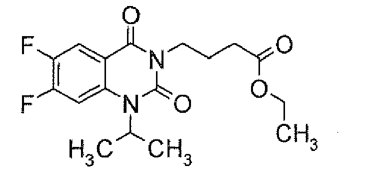 | FAB+: 355 |
| 31 | P2 | 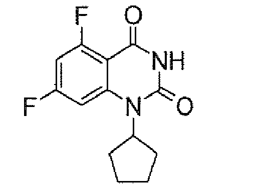 | ESI+: 267 |
| 32 | P1 | 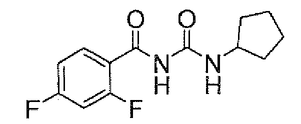 | FAB+: 269 |

**[Table 7]**

| | | | |
|---|---|---|---|
| 33 | P2 | 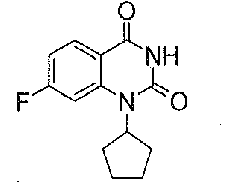 | FAB+: 249 |
| 34 | P3 | 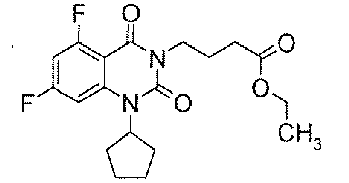 | FAB+: 381 |
| 35 | P13 | 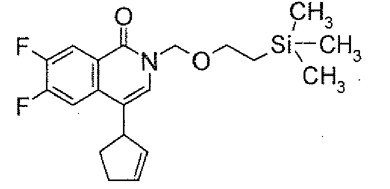 | FAB+: 378 |
| 15 | P15 | 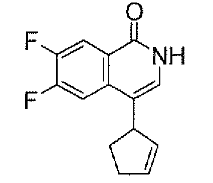 | EI: 247 |
| 36 | P3 | 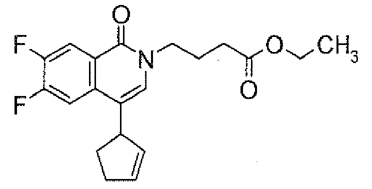 | FAB+: 362 |
| 37 | P8 | 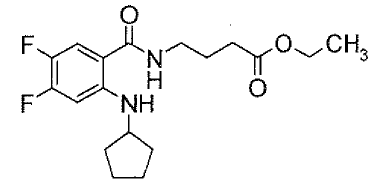 | FAB+: 355 |

**[Table 8]**

| | | | |
|---|---|---|---|
| 5 | P5 | 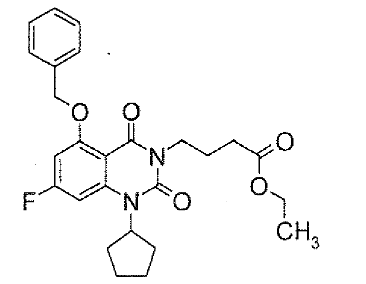 | FAB+: 469 |
| 38 | P1 | 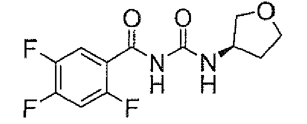 | FAB+: 289 |
| 10 | P10 | 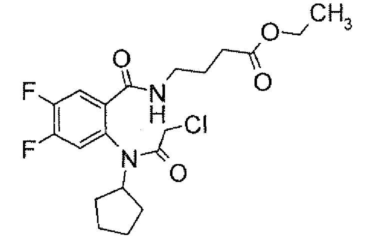 | FAB+: 431 |
| 11 | P11 | 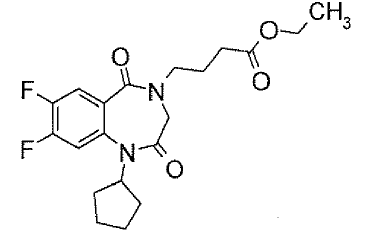 | FAB+: 395 |
| 39 | P2 | 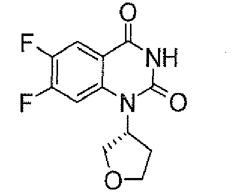 | ESI+: 269 |
| 8 | P8 | 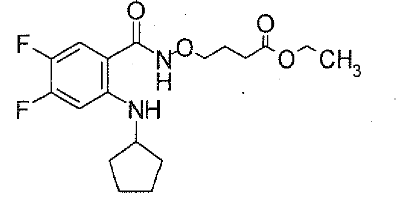 | FAB+: 371 |

**[Table 9]**

| | | | |
|---|---|---|---|
| 6 | P6 | 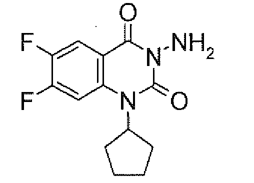 | EI: 281 |
| 40 | P4 | 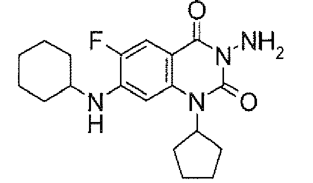 | FAB:+ 361 |
| 9 | P9 | 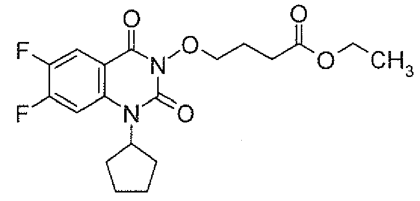 | ESI+: 397 |
| 4 | P4 | 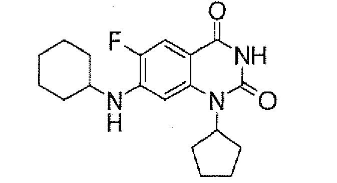 | ESI+: 346 |
| 41 | P4 | 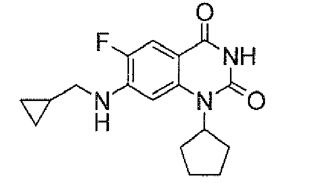 | FAB+: 318 |
| 42 | P4 | 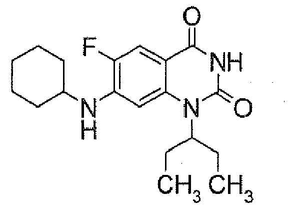 | Fay+: 348 |

**[Table 10]**

| | | | |
|---|---|---|---|
| 43 | P4 | 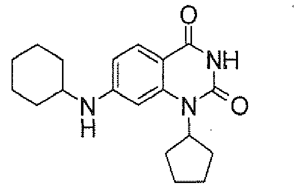 | FAB+: 328 |
| 44 | P4 | 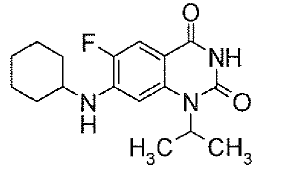 | FAB+: 320 |
| 45 | P4 | 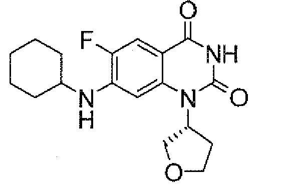 | FAB+: 348 |
| 46 | P4 | 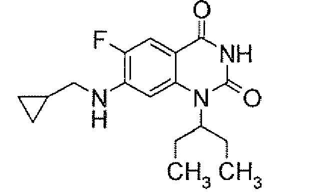 | FAB+: 320 |
| 47 | P16 | 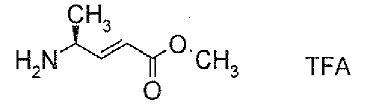 | FAB+: 130 |
| 16 | P16 | 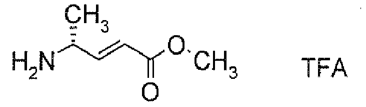 | FAB+: 130 |
| 48 | P6 | 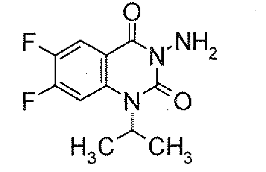 | EI: 255 |

**[Table 11]**

| | | | |
|---|---|---|---|
| 49 | P4 | 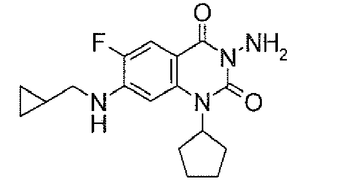 | ESI+: 333 |
| 50 | P4 | 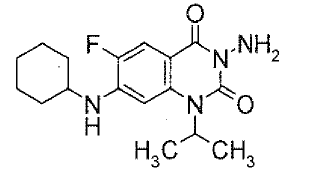 | ESI+: 335 |
| 51 | P3 | 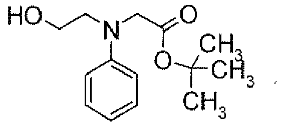 | ESI+: 252 |
| 17 | P17 | 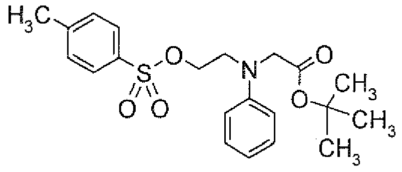 | FAB-: 404 |

**[Table 12]**

| Ex | Structure |
|---|---|
| 37 | 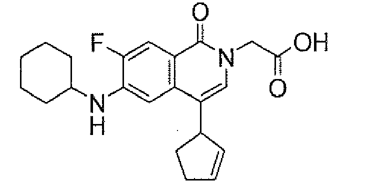 |
| 38 | 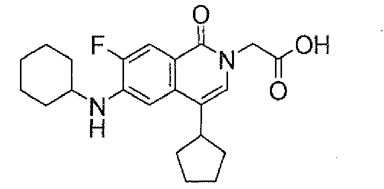 |
| 39 | 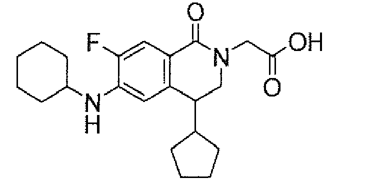 |
| 1 | 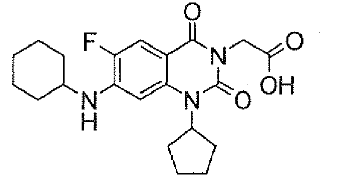 |
| 40 | 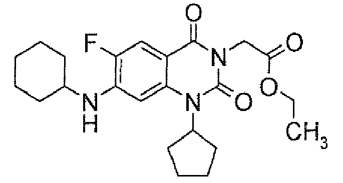 |
| 41 | 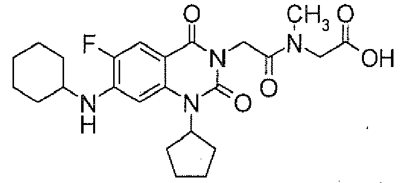 |

**[Table 13]**

| | |
|---|---|
| 42 | 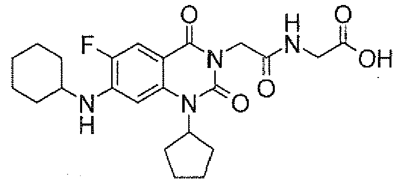 |
| 43 | 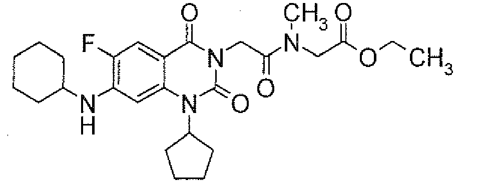 |
| 44 | 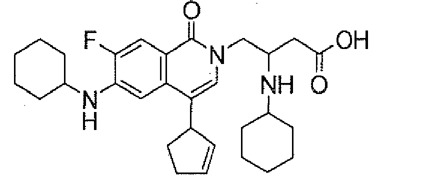 |
| 45 | 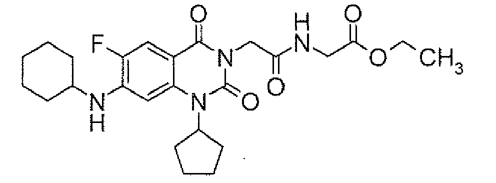 |
| 46 | 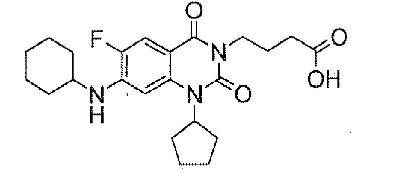 |
| 47 | 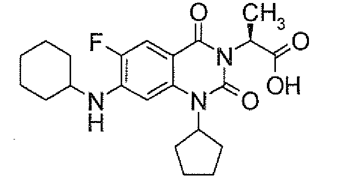 |
| 48 | 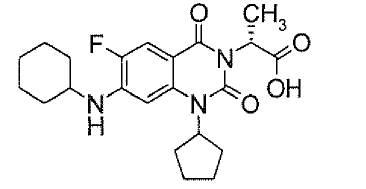 |

**[Table 14]**

| | |
|---|---|
| 49 | 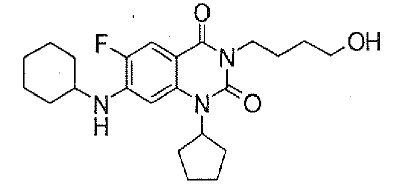 |
| 7 | 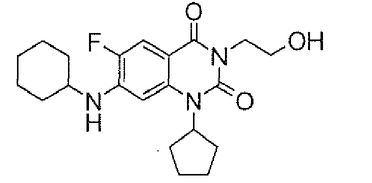 |
| 50 | 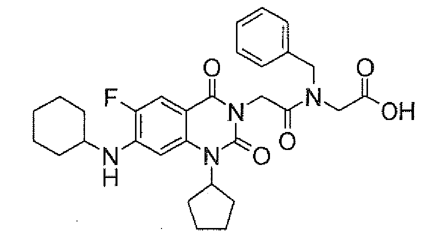 |
| 51 | 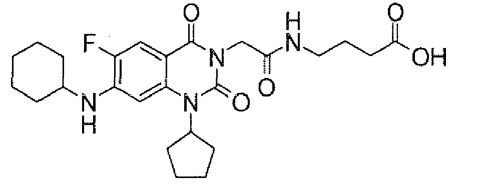 |
| 52 | 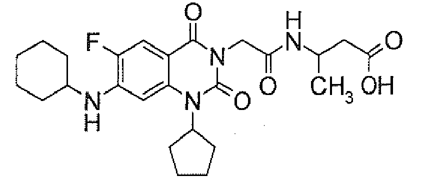 |
| 53 | 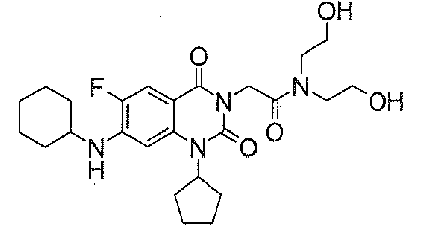 |

**[Table 15]**

| | |
|---|---|
| 54 | 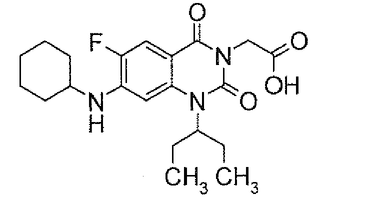 |
| 55 | 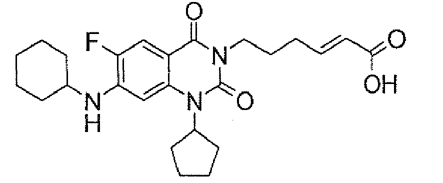 |
| 6 | 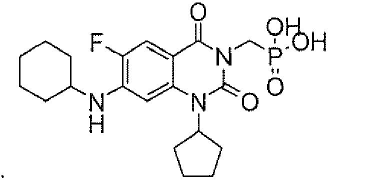 |
| 5 | 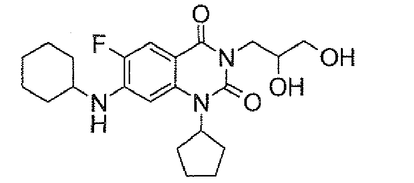 |
| 56 | 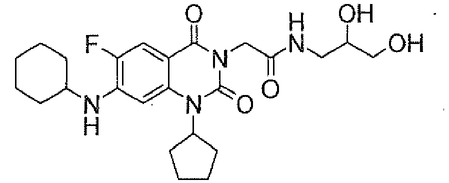 |
| 57 | 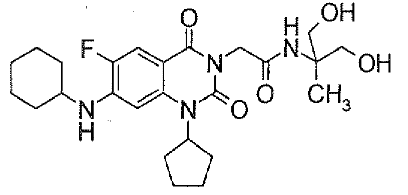 |

**[Table 16]**

| | |
|---|---|
| 58 | 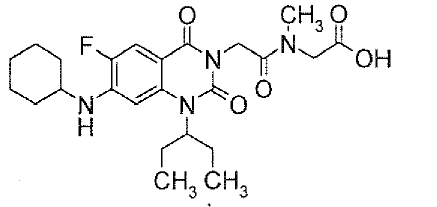 |
| 59 | 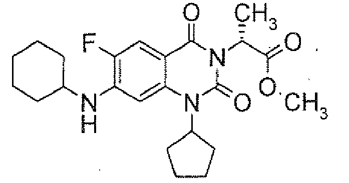 |
| 60 | 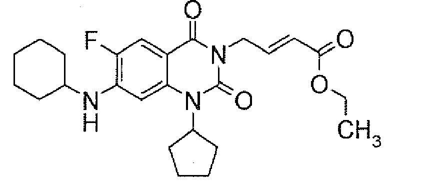 |
| 61 | 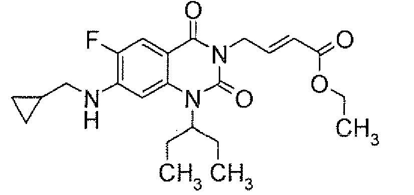 |
| 62 | 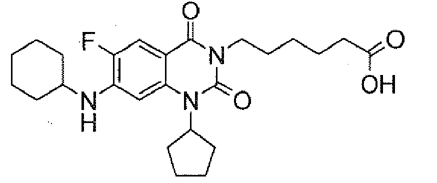 |
| 63 | 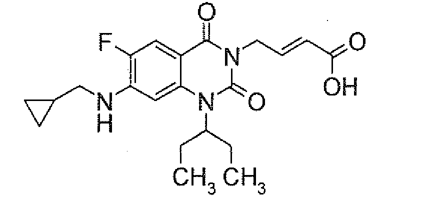 |

**[Table 17]**

| | |
|---|---|
| 64 | 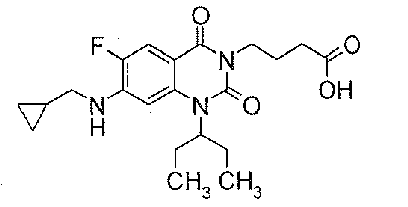 |
| 65 | 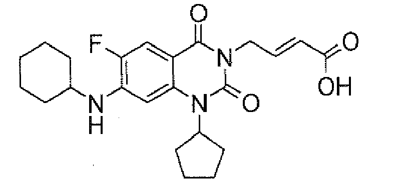 |
| 66 | 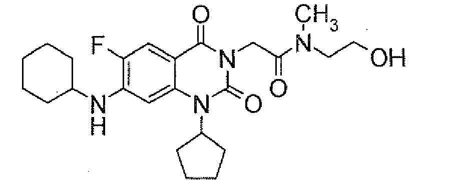 |
| 67 | 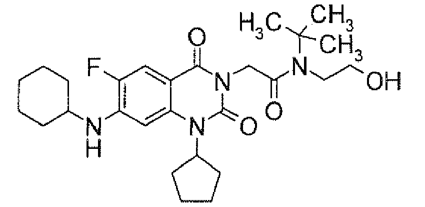 |
| 68 | 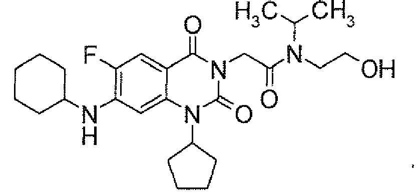 |
| 69 | 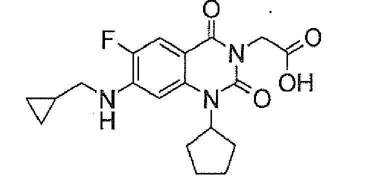 |

**[Table 18]**

| | |
|---|---|
| 70 | 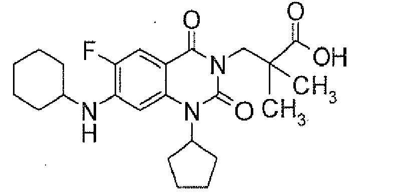 |
| 71 | 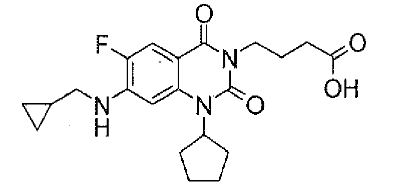 |
| 72 | 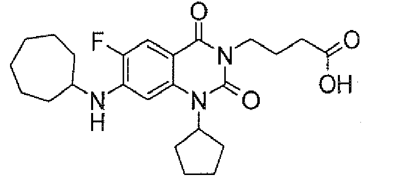 |
| 73 | 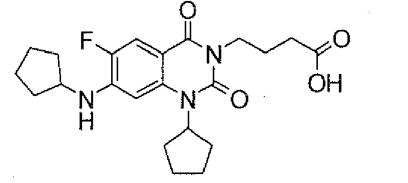 |
| 74 | 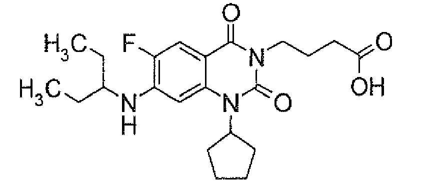 |
| 75 | 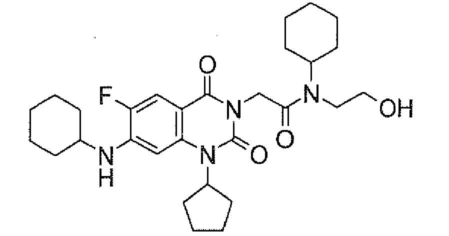 |

**[Table 19]**

| | |
|---|---|
| 76 | 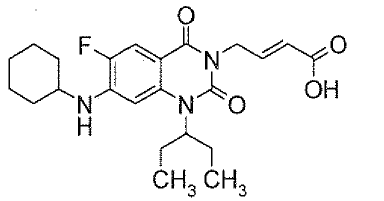 |
| 77 | 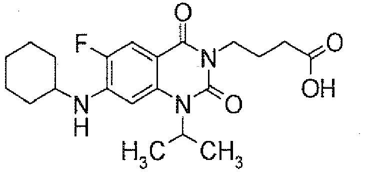 |
| 78 | 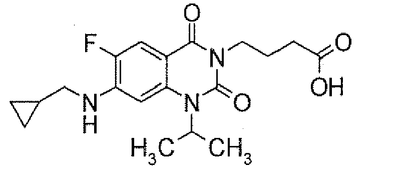 |
| 8 | 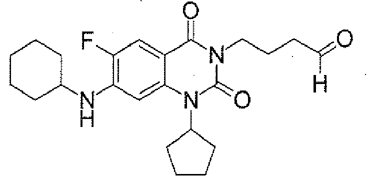 |
| 9 | 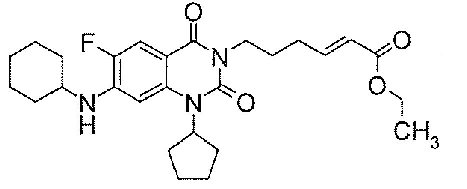 |
| 79 | 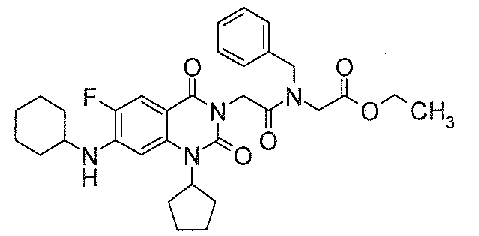 |

**[Table 20]**

| | |
|---|---|
| 80 | 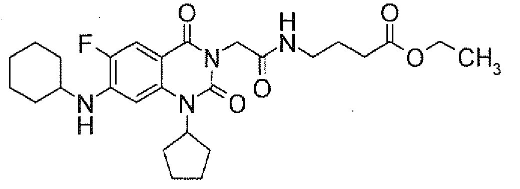 |
| 81 | 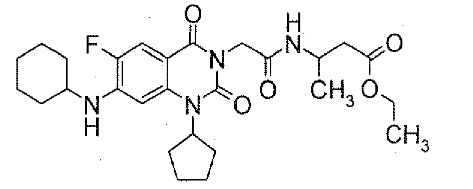 |
| 82 | 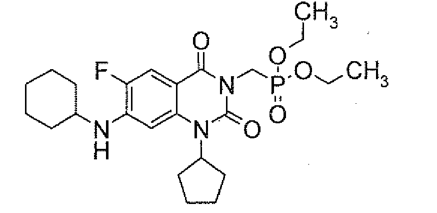 |
| 83 | 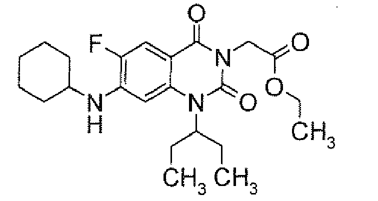 |
| 84 | 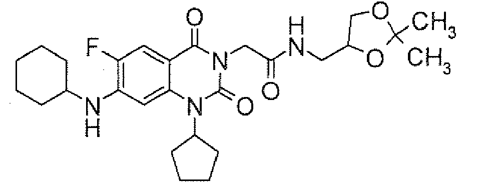 |
| 4 | 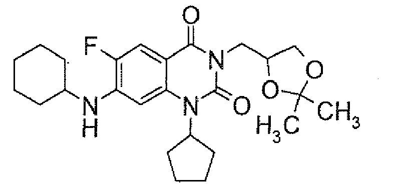 |

**[Table 21]**

| | |
|---|---|
| 85 | 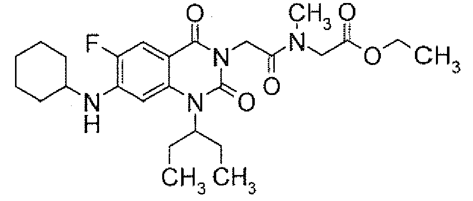 |
| 19 | 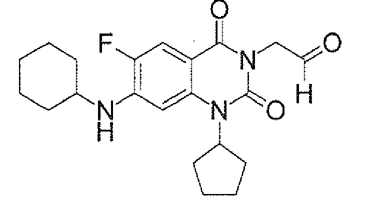 |
| 86 | 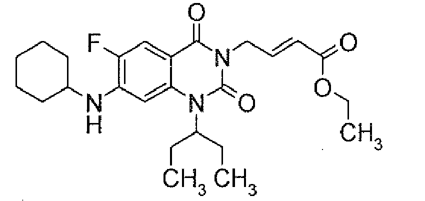 |
| 87 | 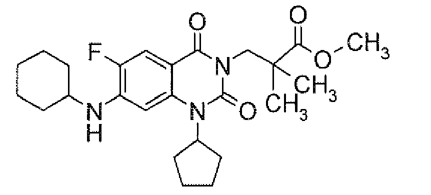 |
| 88 | 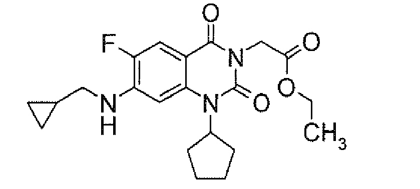 |
| 89 | 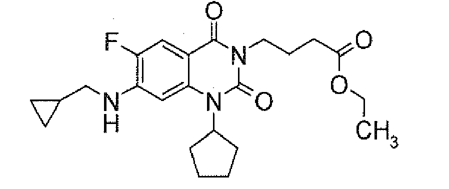 |

**[Table 22]**

| | |
|---|---|
| 90 | 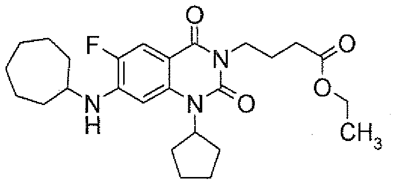 |
| 91 | 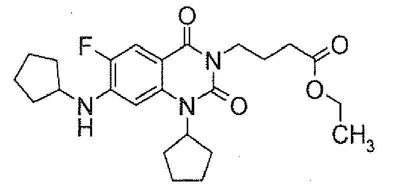 |
| 92 | 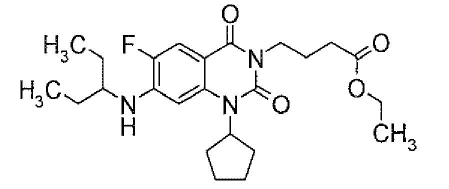 |
| 93 | 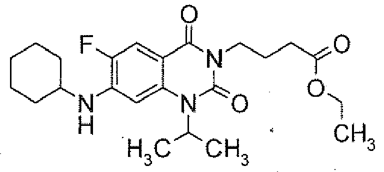 |
| 94 | 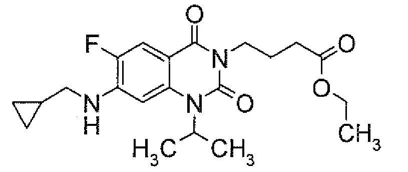 |
| 95 | 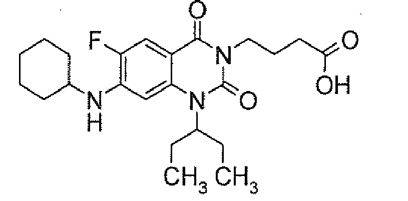 |
| 96 | 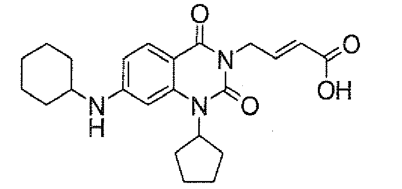 |

**[Table 23]**

| | |
|---|---|
| 97 | 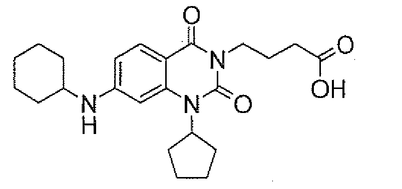 |
| 98 | 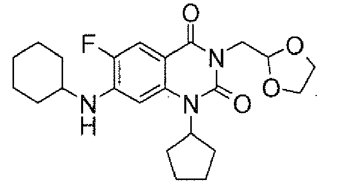 |
| 99 | 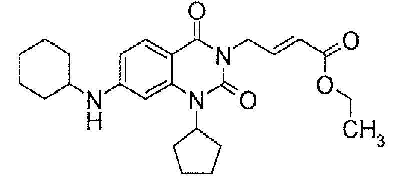 |
| 100 | 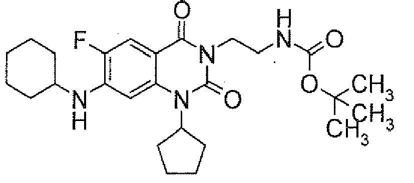 |
| 101 | 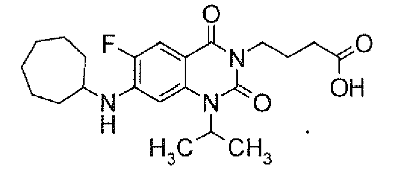 |
| 102 | 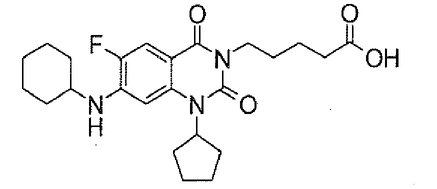 |
| 103 | 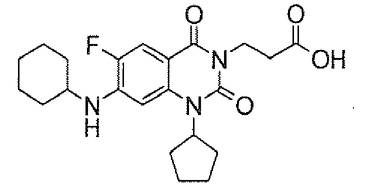 |

**[Table 24]**

| | |
|---|---|
| 104 | 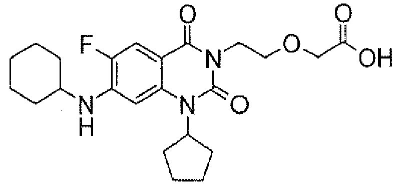 |
| 105 | 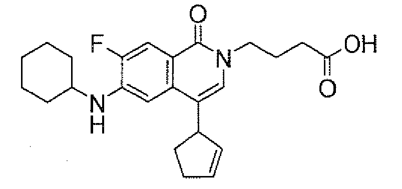 |
| 106 | 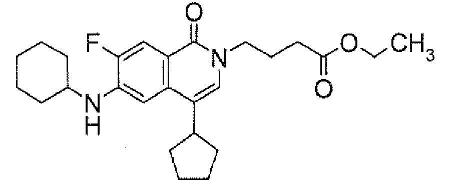 |
| 107 | 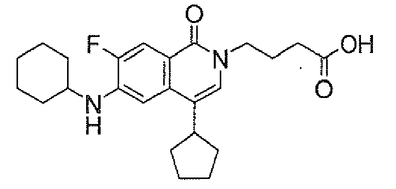 |
| 12 | 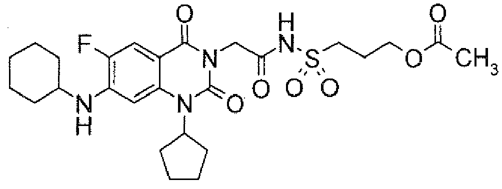 |
| 108 | 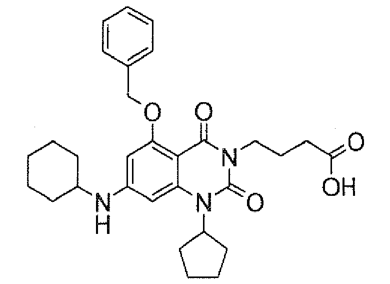 |

**[Table 25]**

| | |
|---|---|
| 109 | 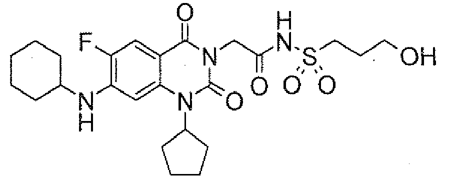 |
| 110 | 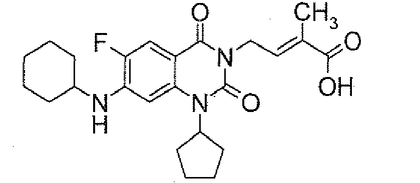 |
| 111 | 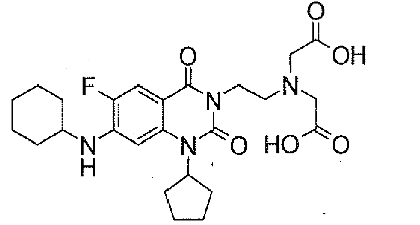 |
| 112 | 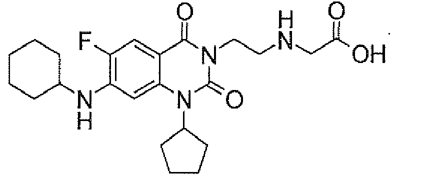 |
| 113 | 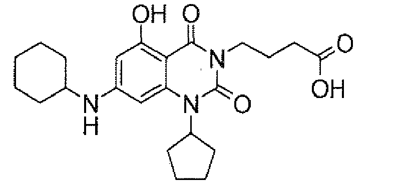 |
| 114 | 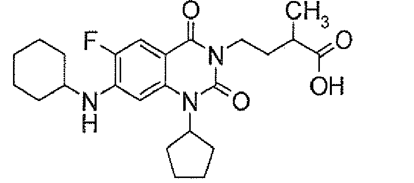 |

**[Table 26]**

| | |
|---|---|
| 18 | 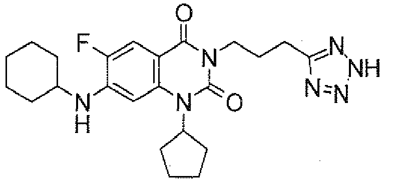 |
| 14 | 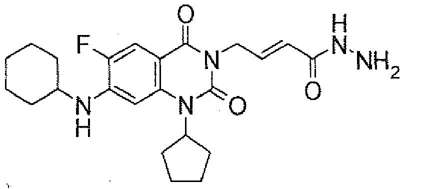 |
| 15 | 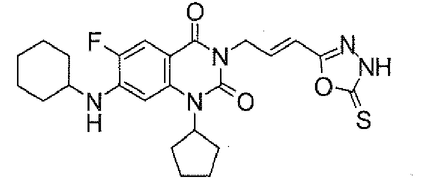 |
| 115 | 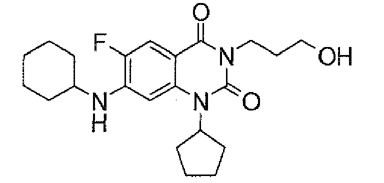 |
| 116 | 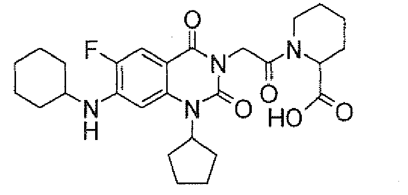 |
| 117 | 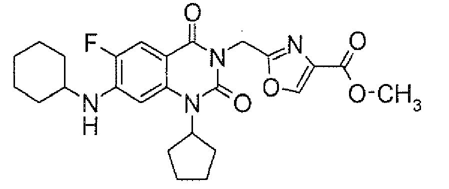 |

**[Table 27]**

| | |
|---|---|
| 118 | 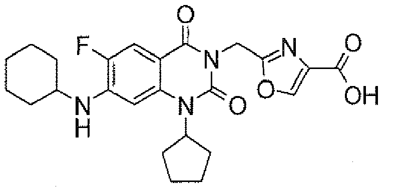 |
| 119 | 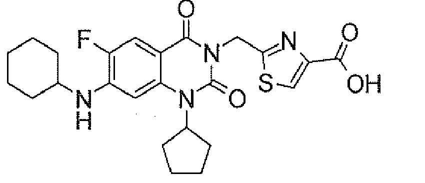 |
| 120 | 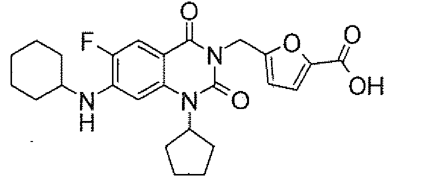 |
| 121 | 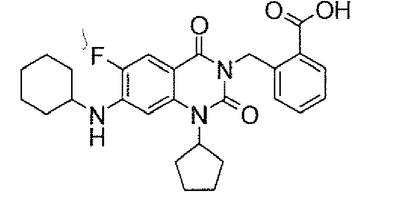 |
| 122 | 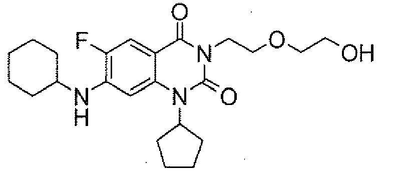 |
| 123 | 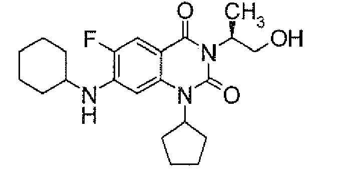 |
| 124 | 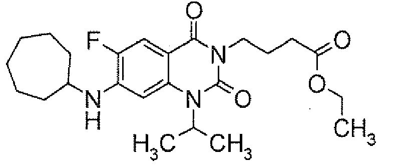 |

**[Table 28]**

| | |
|---|---|
| 125 | 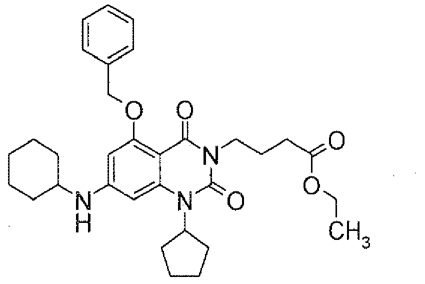 |
| 126 | 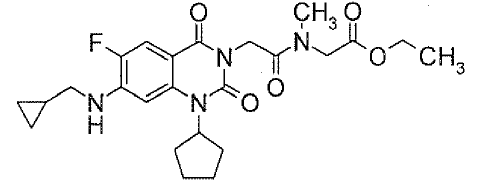 |
| 127 | 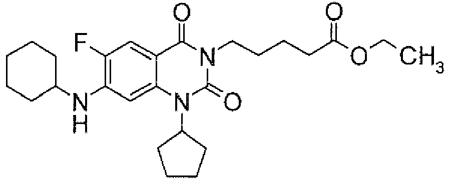 |
| 128 | 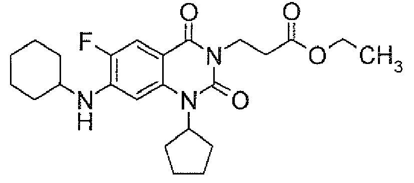 |
| 10 | 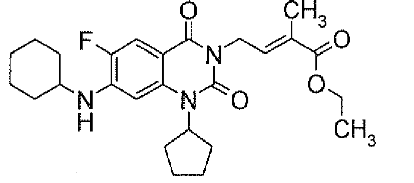 |
| 11 | 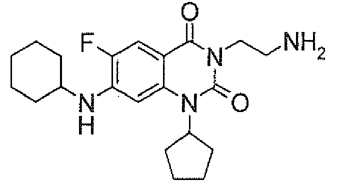 |

**[Table 29]**

| | |
|---|---|
| 129 | 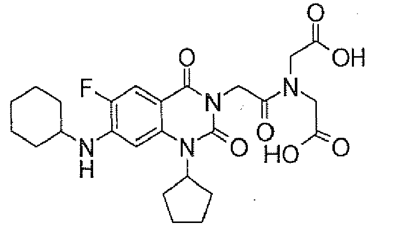 |
| 130 | 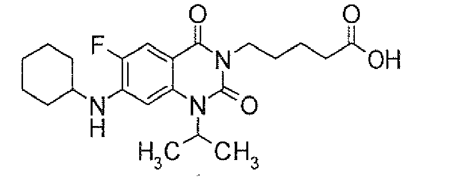 |
| 131 | 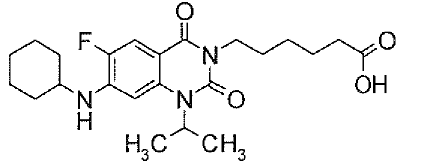 |
| 132 | 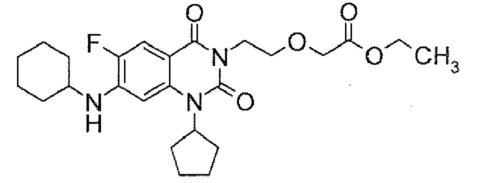 |
| 133 | 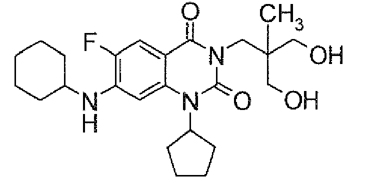 |
| 134 | 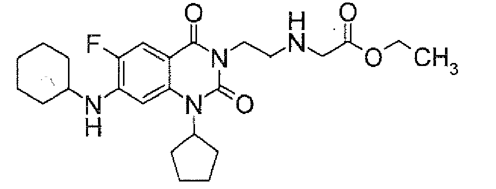 |

**[Table 30]**

| | |
|---|---|
| 135 | 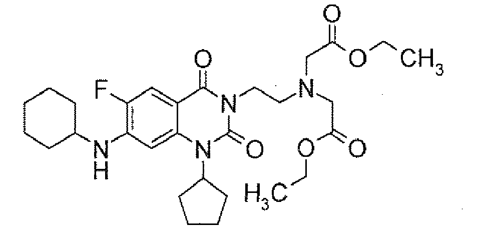 |
| 136 | 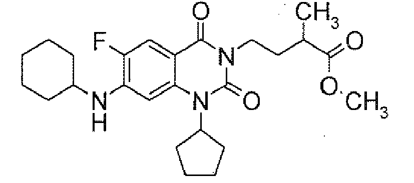 |
| 137 | 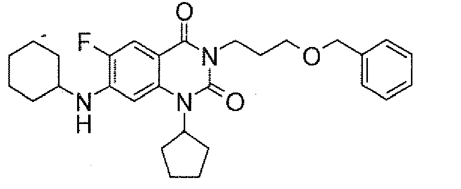 |
| 138 | 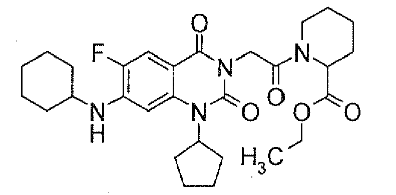 |
| 139 | 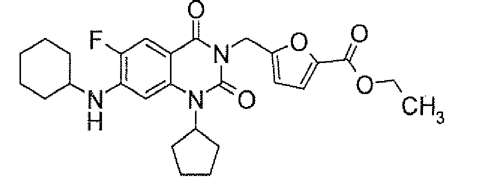 |
| 140 | 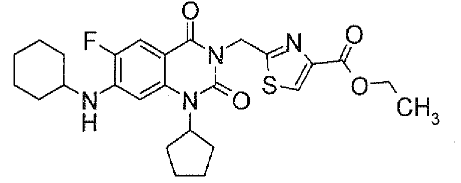 |

**[Table 31]**

| | |
|---|---|
| 141 | 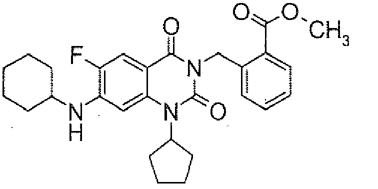 |
| 142 | 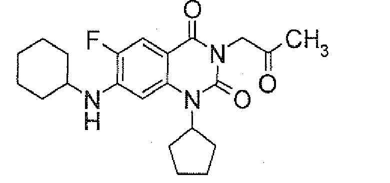 |
| 143 | 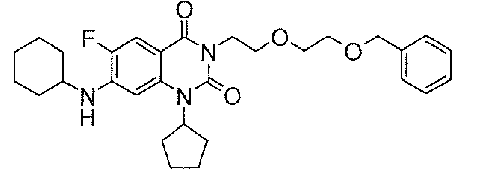 |
| 144 | 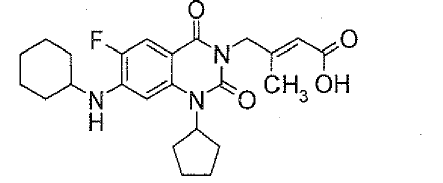 |
| 145 | 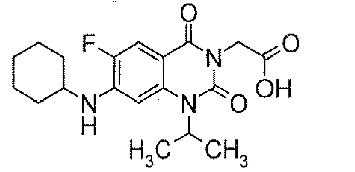 |
| 146 | 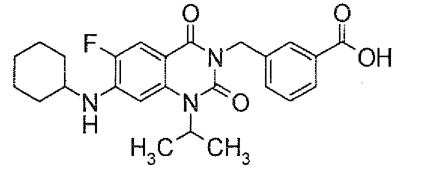 |
| 147 | 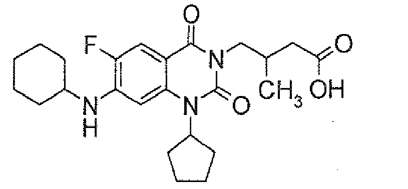 |

**[Table 32]**

| | |
|---|---|
| 148 | 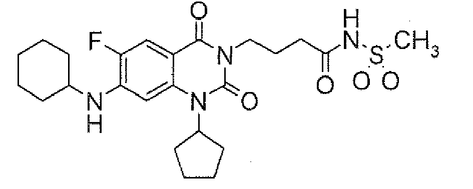 |
| 149 | 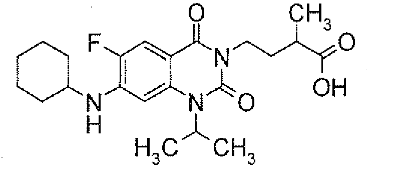 |
| 150 | 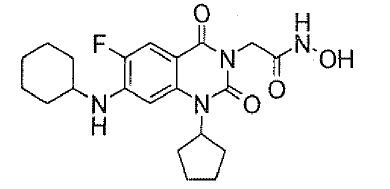 |
| 151 | 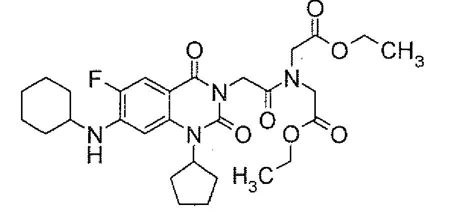 |
| 152 | 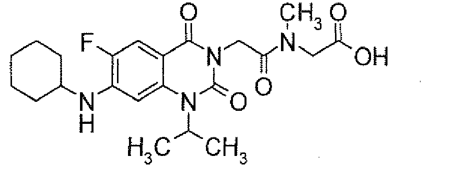 |
| 153 | 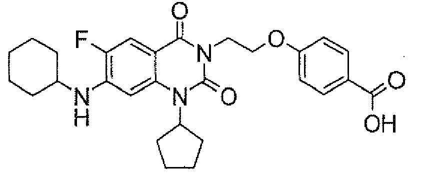 |
| 26 | 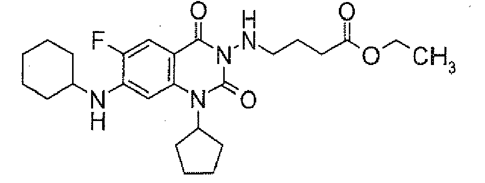 |

**[Table 33]**

| | |
|---|---|
| 154 | 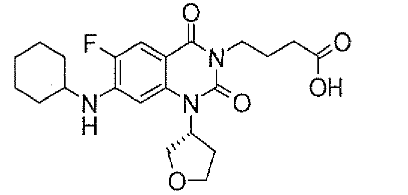 |
| 155 | 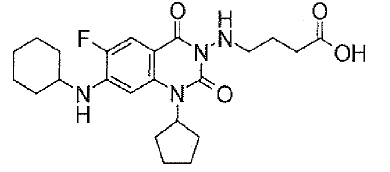 |
| 30 | 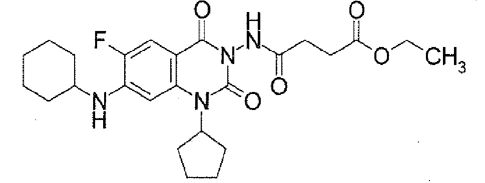 |
| 156 | 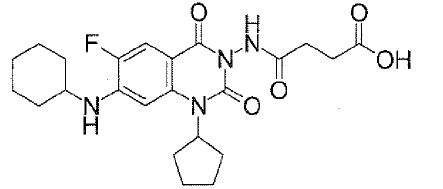 |
| 27 | 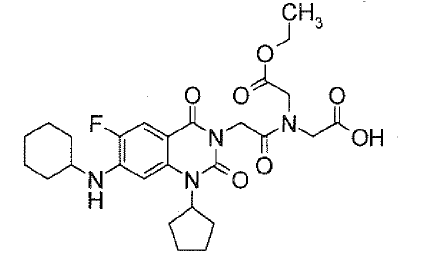 |
| 157 | 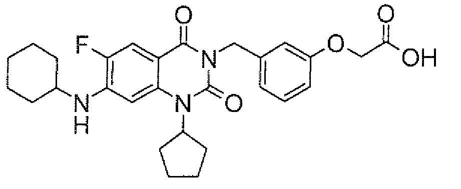 |

**[Table 34]**

| | |
|---|---|
| 158 | 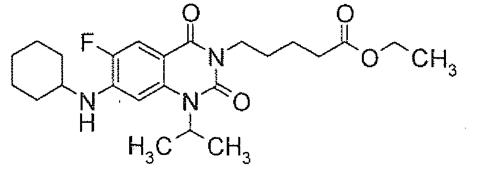 |
| 159 | 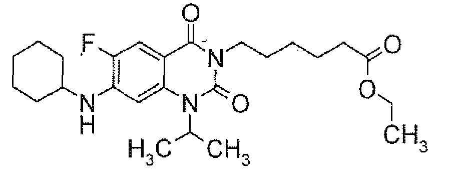 |
| 160 | 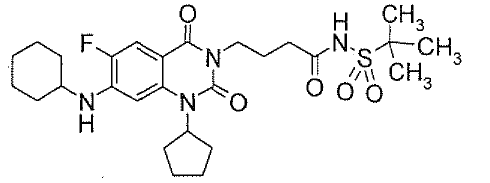 |
| 161 | 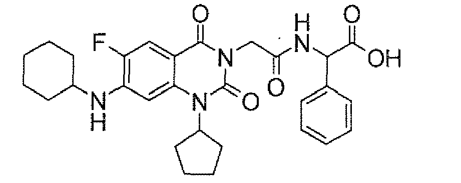 |
| 162 | 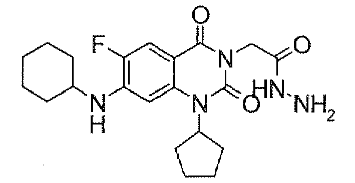 |
| 20 | 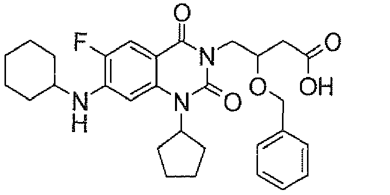 |
| 163 | 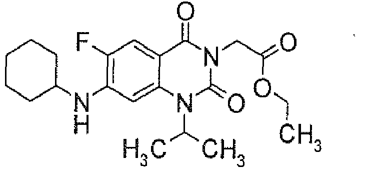 |

**[Table 35]**

| | |
|---|---|
| 164 | 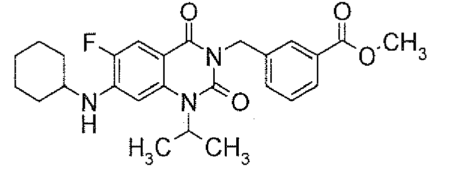 |
| 165 | 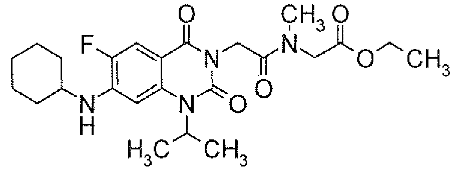 |
| 166 | 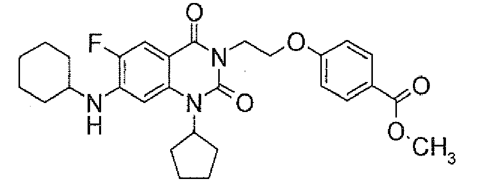 |
| 167 | 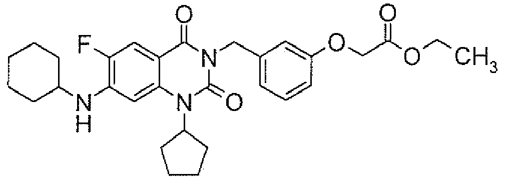 |
| 168 | 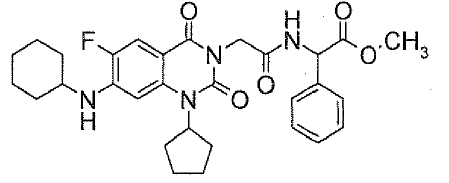 |
| 169 | 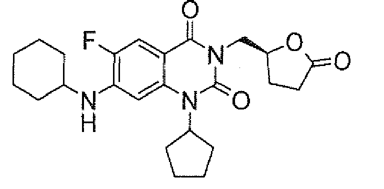 |
| 170 | 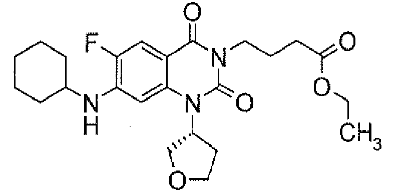 |

**[Table 36]**

| | |
|---|---|
| 171 | 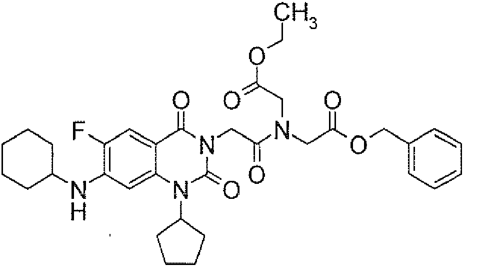 |
| 172 | 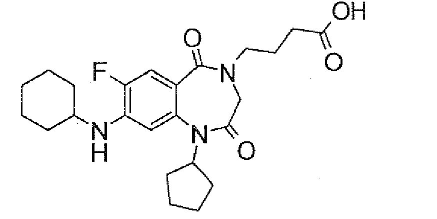 |
| 173 | 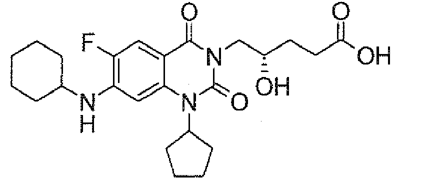 |
| 174 | 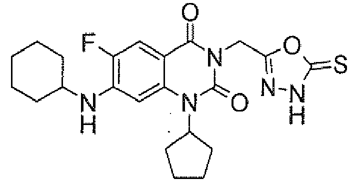 |
| 13 | 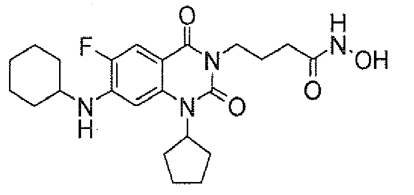 |
| 175 | 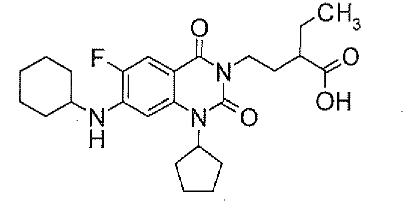 |

**[Table 37]**

| | |
|---|---|
| 176 | 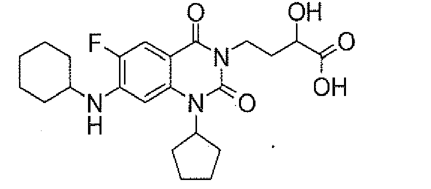 |
| 177 | 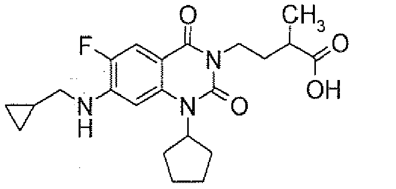 |
| 178 | 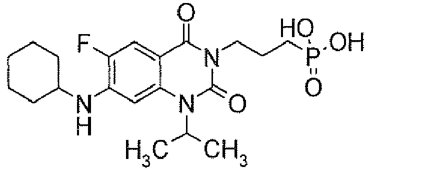 |
| 179 | 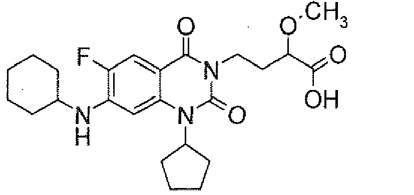 |
| 180 | 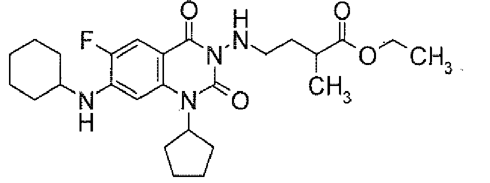 |
| 16 | 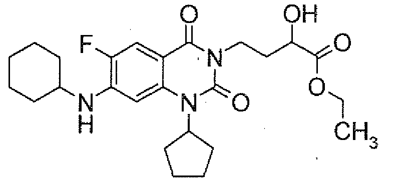 |

**[Table 38]**

| | |
|---|---|
| 181 | 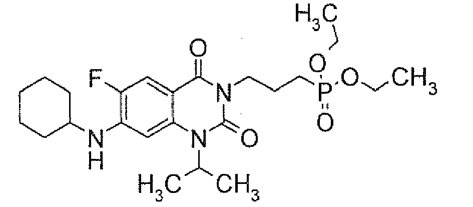 |
| 182 | 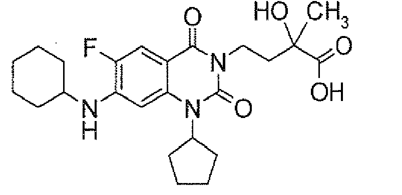 |
| 183 | 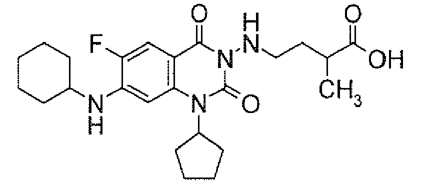 |
| 184 | 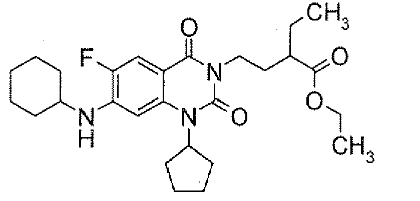 |
| 185 | 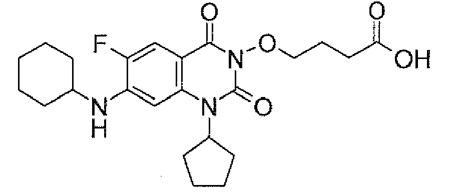 |
| 186 | 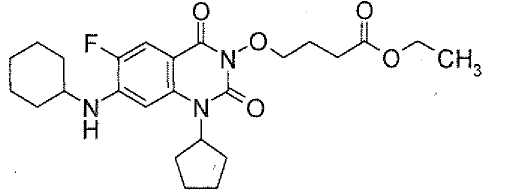 |

**[Table 39]**

| | |
|---|---|
| 17 | 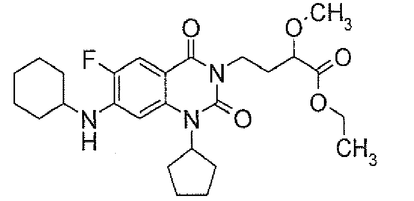 |
| 187 | 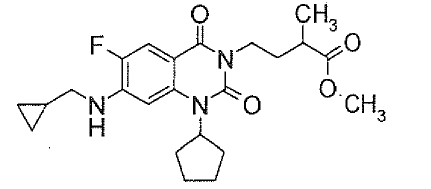 |
| 188 | 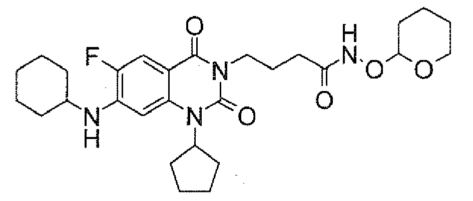 |
| 189 | 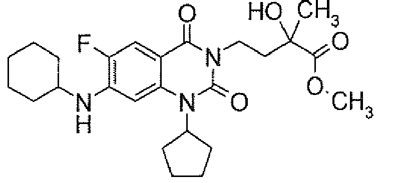 |
| 190 | 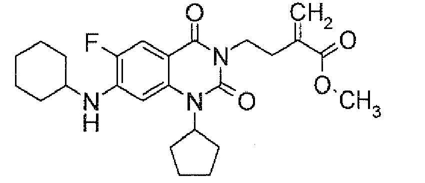 |
| 191 | 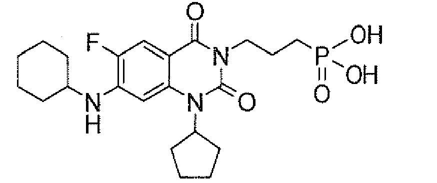 |

**[Table 40]**

| | |
|---|---|
| 192 | 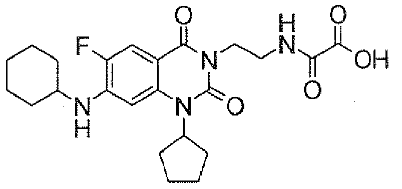 |
| 193 | 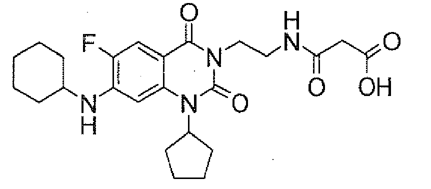 |
| 194 | 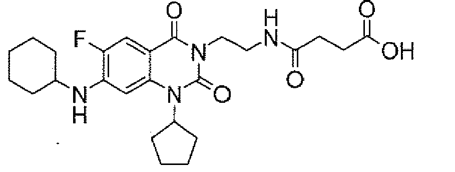 |
| 21 | 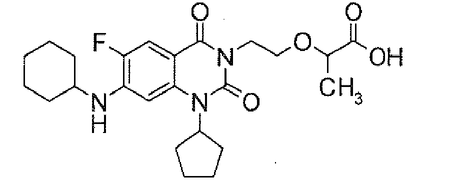 |
| 195 | 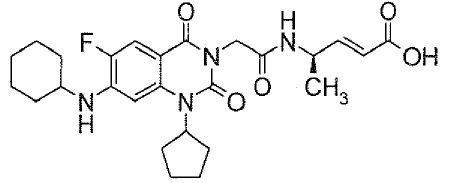 |
| 196 | 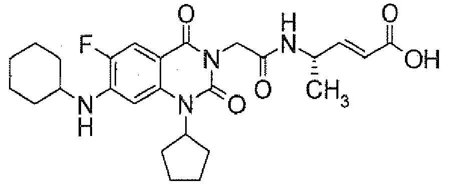 |
| 197 | 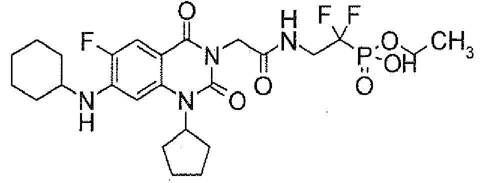 |

**[Table 41]**

| | |
|---|---|
| 198 | 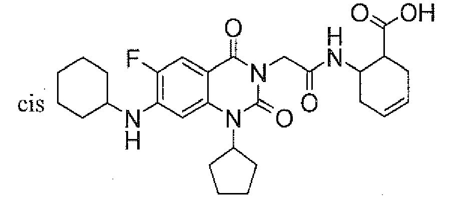 |
| 199 | 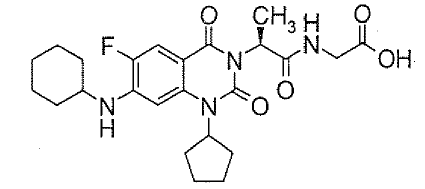 |
| 200 | 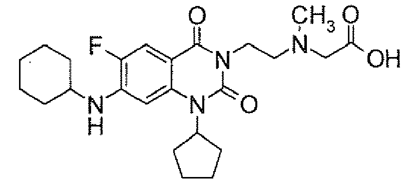 |
| 201 | 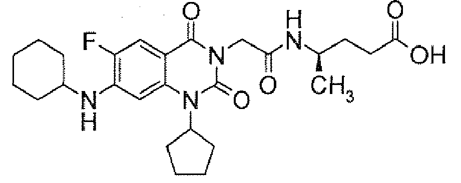 |
| 202 | 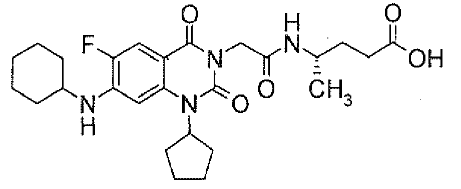 |
| 203 | 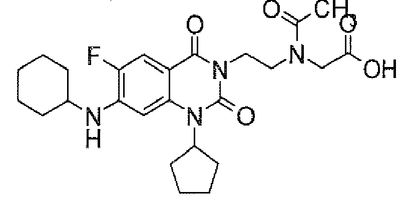 |

**[Table 42]**

| | |
|---|---|
| 204 | 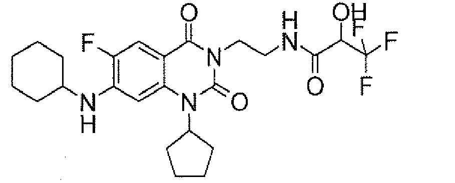 |
| 205 | 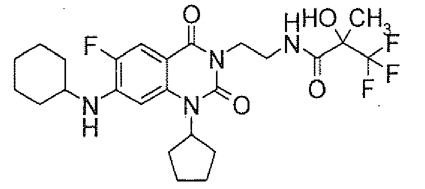 |
| 206 | 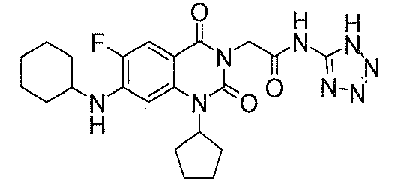 |
| 28 | 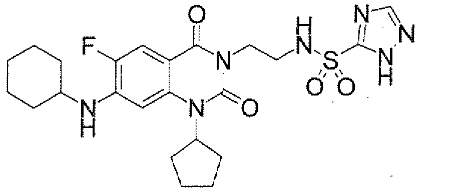 |
| 29 | 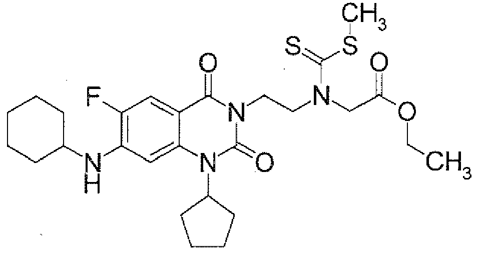 |
| 207 | 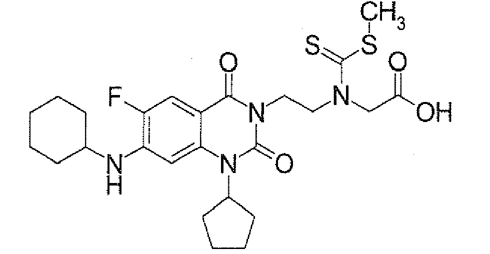 |

**[Table 43]**

| | |
|---|---|
| 24 | 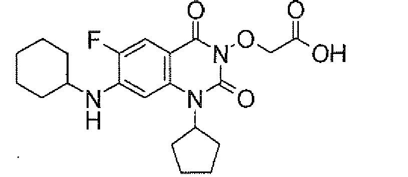 |
| 208 | 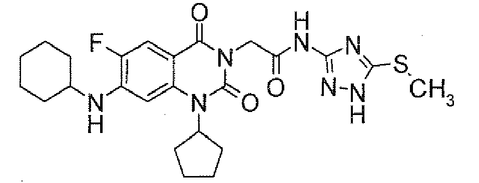 |
| 209 | 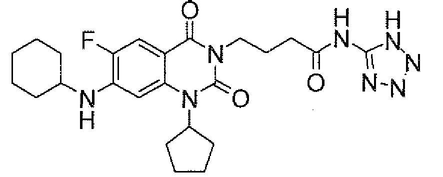 |
| 210 | 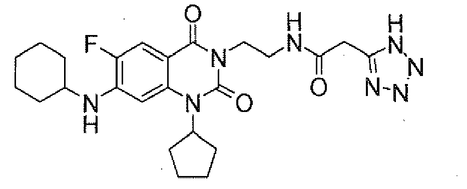 |
| 211 | 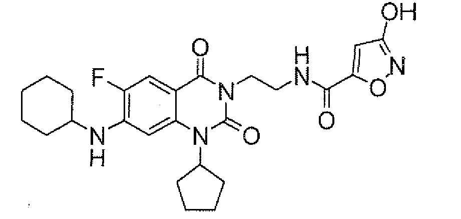 |
| 212 | 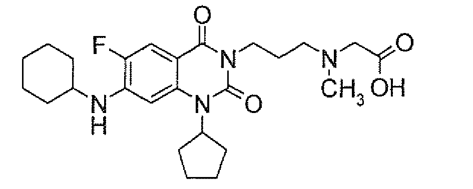 |

**[Table 44]**

| | |
|---|---|
| 25 | 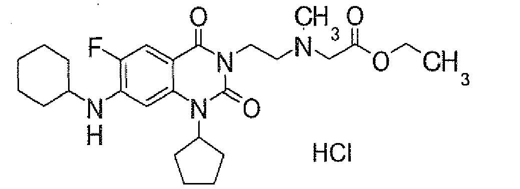 |
| 213 | 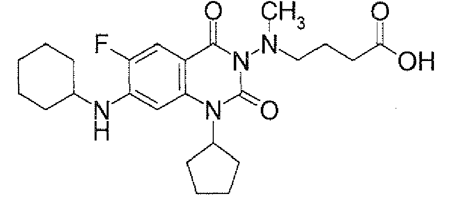 |
| 214 | 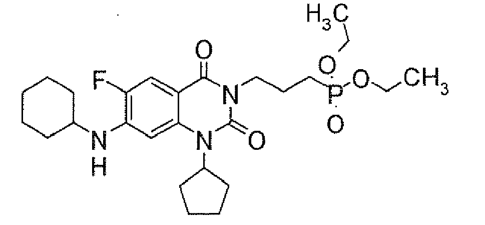 |
| 215 | 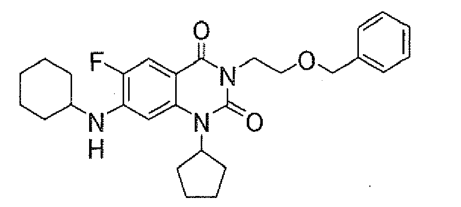 |
| 216 | 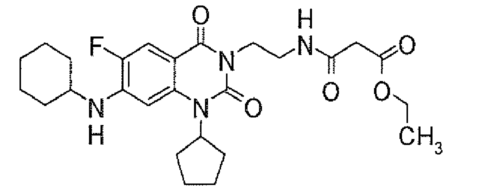 |
| 217 | 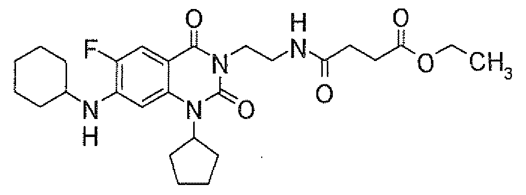 |

**[Table 45]**

| | |
|---|---|
| 23 | 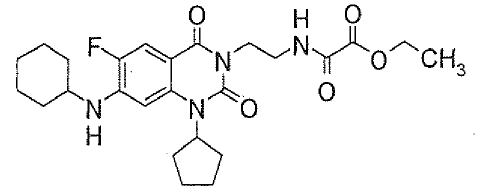 |
| 218 | 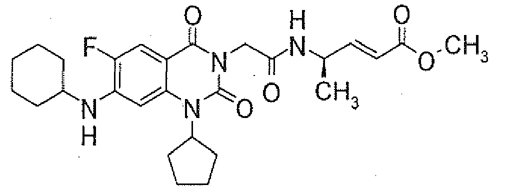 |
| 219 | 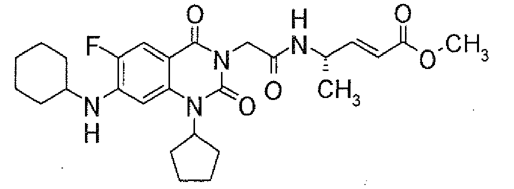 |
| 220 | 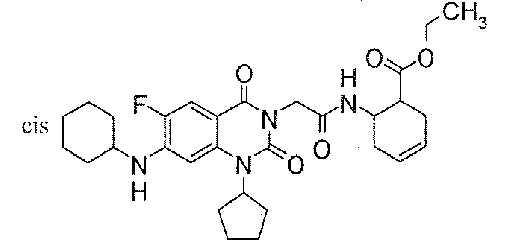 |
| 221 | 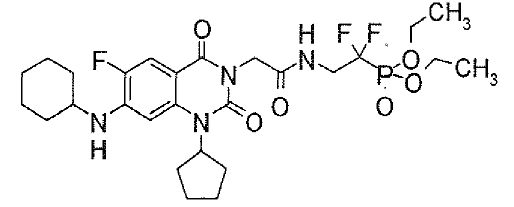 |
| 222 | 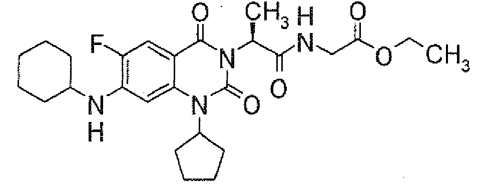 |

**[Table 46]**

| | |
|---|---|
| 223 | 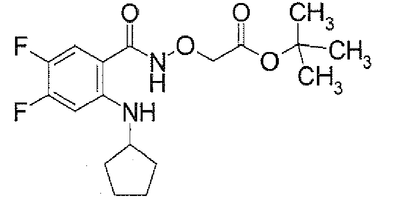 |
| 224 | 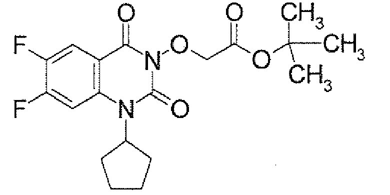 |
| 22 | 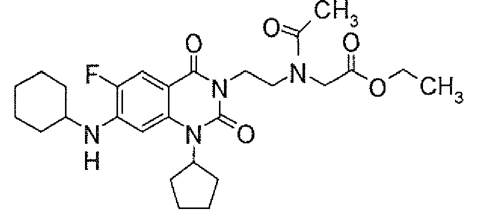 |
| 225 | 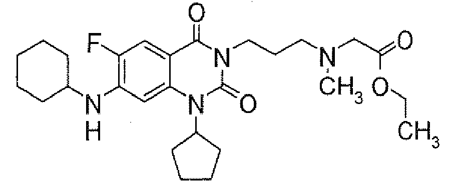 |
| 226 | 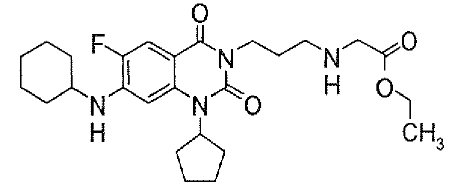 |
| 227 | 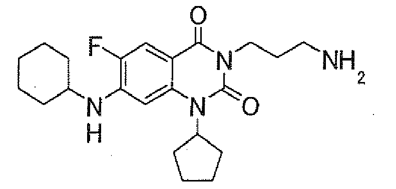 |

**[Table 47]**

| | |
|---|---|
| 228 | 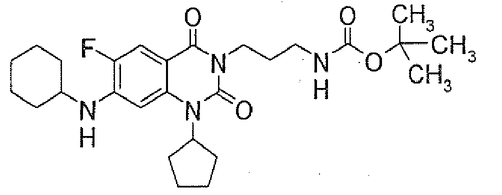 |
| 229 | 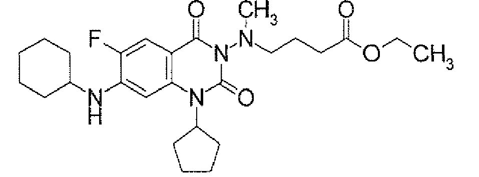 |
| 230 | 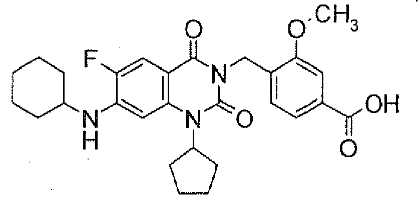 |
| 231 | 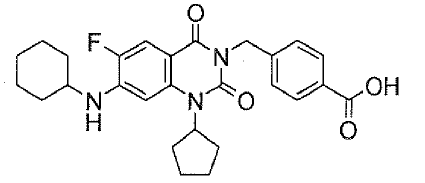 |
| 232 | 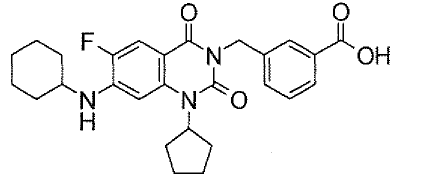 |
| 233 | 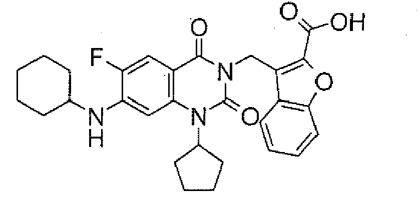 |

**[Table 48]**

| | |
|---|---|
| 234 | 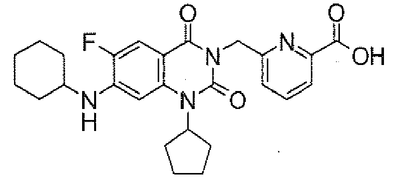 |
| 235 | 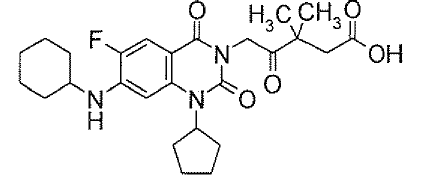 |
| 236 | 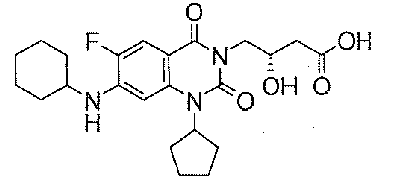 |
| 237 | 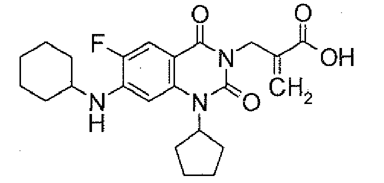 |
| 238 | 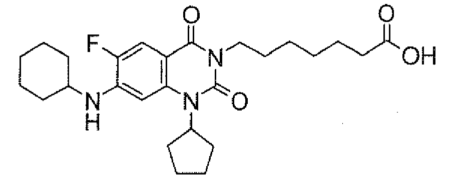 |
| 239 | 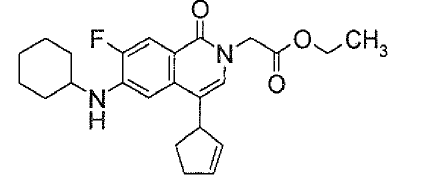 |
| 2 | 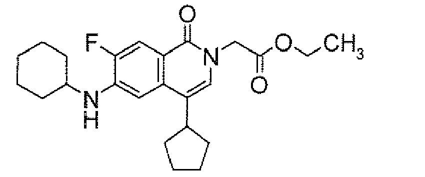 |

**[Table 49]**

| | |
|---|---|
| 3 | 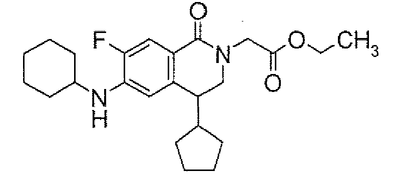 |
| 240 | 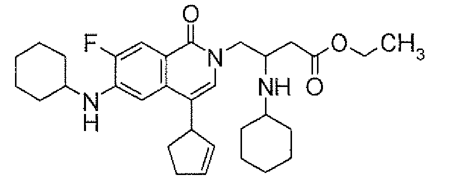 |
| 241 | 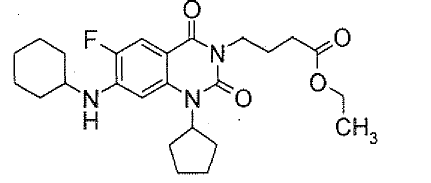 |
| 242 | 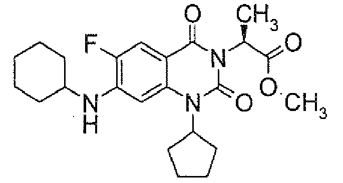 |
| 243 | 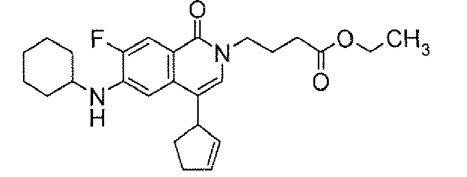 |
| 244 | 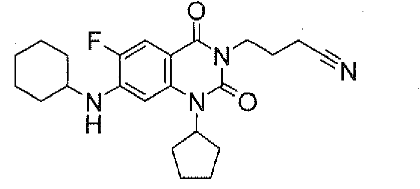 |
| 245 | 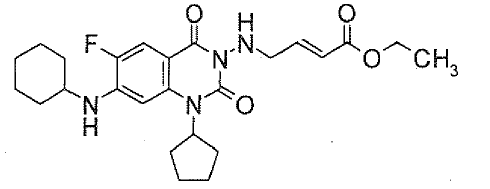 |

**[Table 50]**

| | |
|---|---|
| 246 | 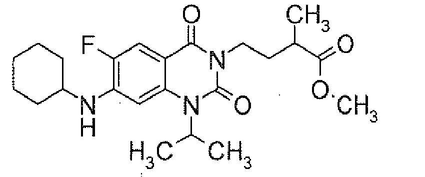 |
| 247 | 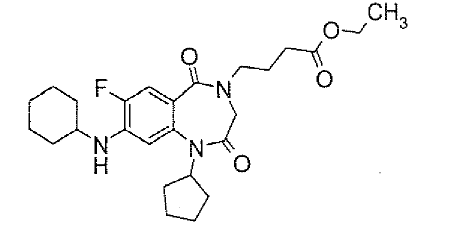 |
| 248 | 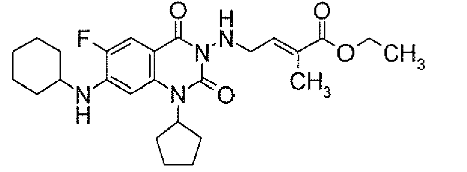 |
| 249 | 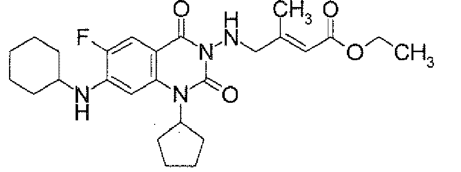 |
| 250 | 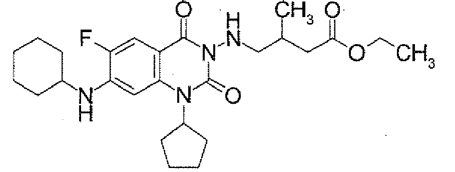 |
| 251 | 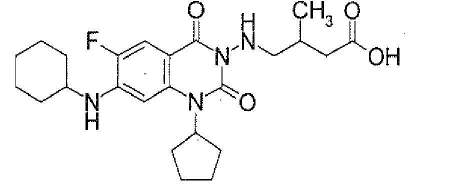 |
| 252 | 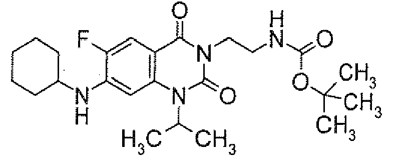 |

**[Table 51]**

| | |
|---|---|
| 253 | 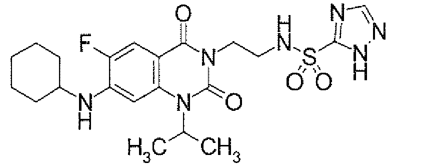 |
| 254 | 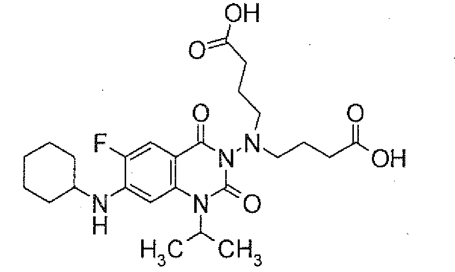 |
| 255 | 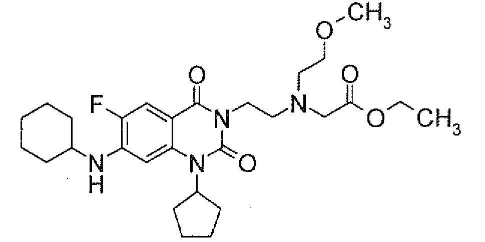 |
| 256 | 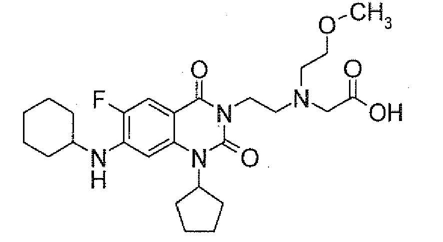 |
| 257 | 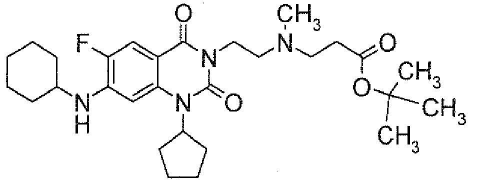 |
| 34 | 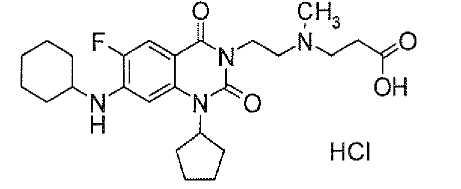 |

**[Table 52]**

| | |
|---|---|
| 36 | 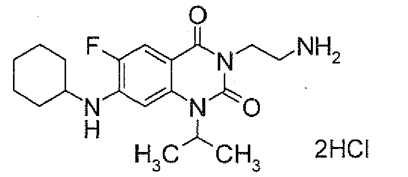 |
| 258 | 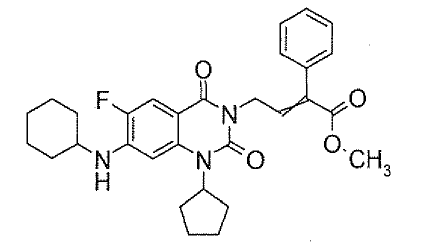 |
| 259 | 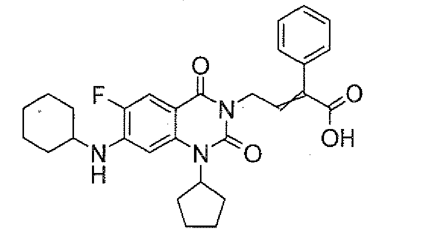 |
| 260 | 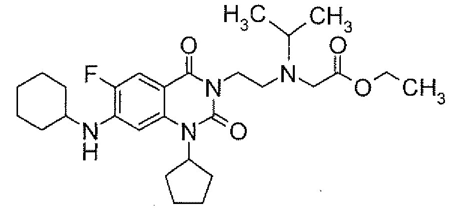 |
| 261 | 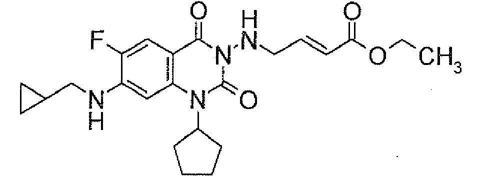 |
| 262 | 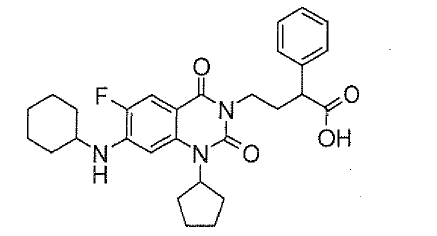 |

**[Table 53]**

| | |
|---|---|
| 35 | 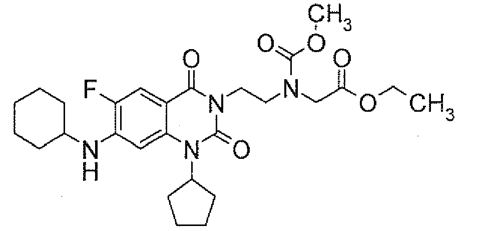 |
| 263 | 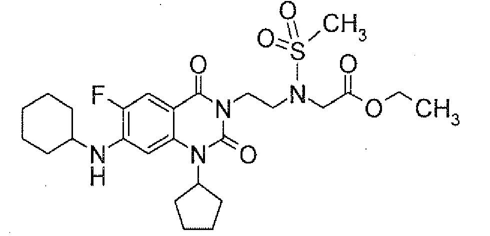 |
| 264 | 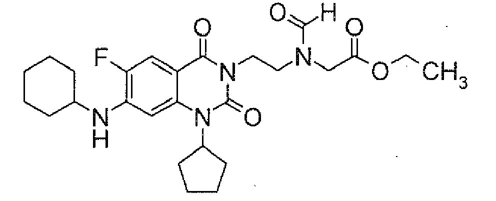 |
| 265 | 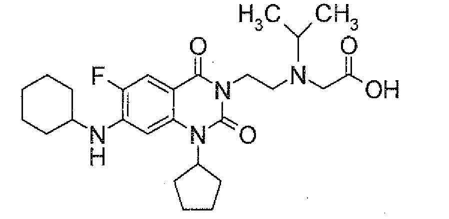 |
| 266 | 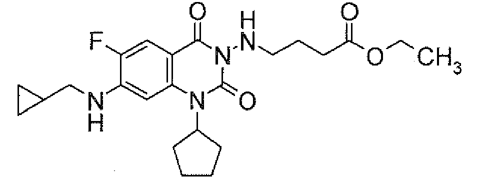 |
| 267 | 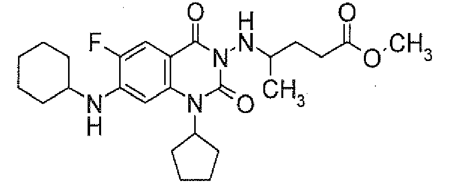 |

**[Table 54]**

| | |
|---|---|
| 268 | 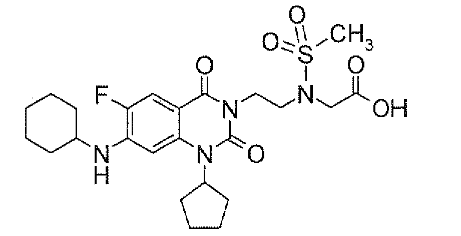 |
| 269 | 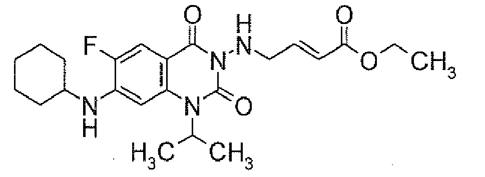 |
| 270 | 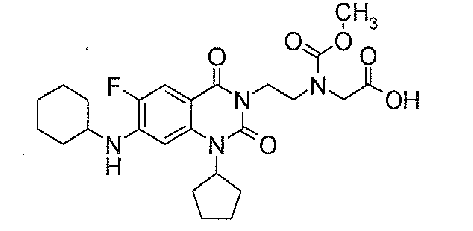 |
| 271 | 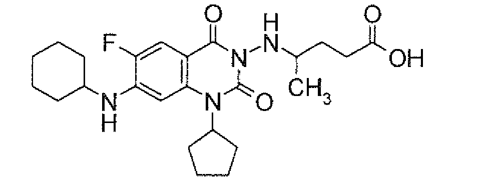 |
| 272 | 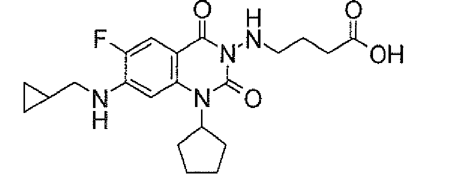 |
| 273 | 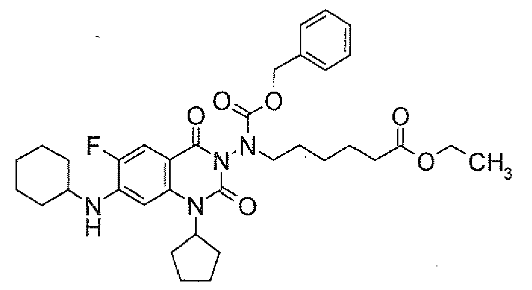 |

**[Table 55]**

| | |
|---|---|
| 274 | 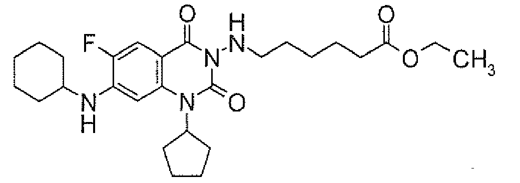 |
| 275 | 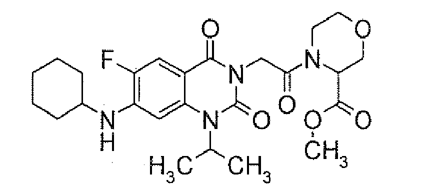 |
| 276 | 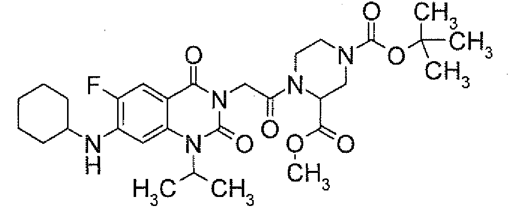 |
| 277 | 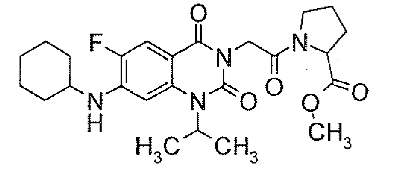 |
| 278 | 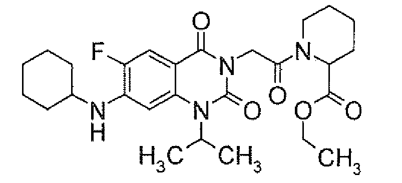 |
| 279 | 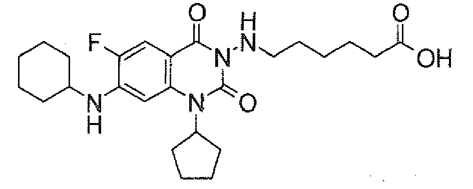 |
| 280 | 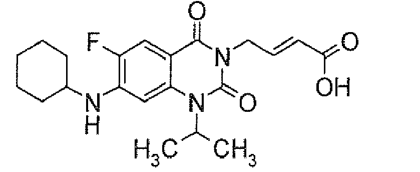 |

**[Table 56]**

| | |
|---|---|
| 281 | 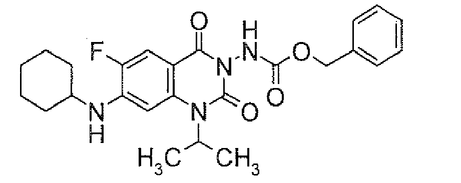 |
| 32 | 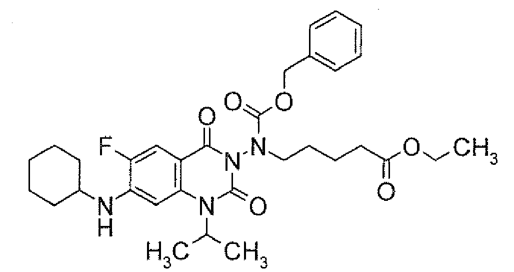 |
| 282 | 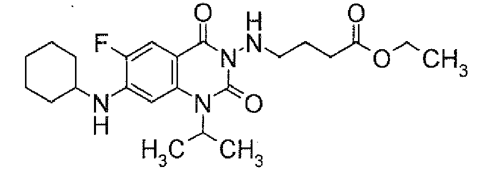 |
| 283 | 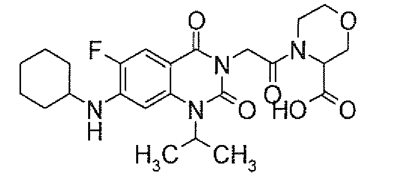 |
| 284 | 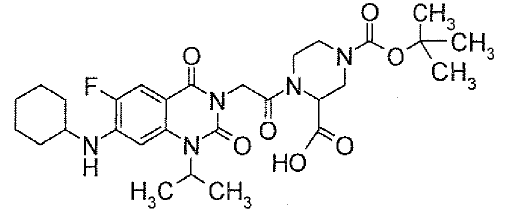 |
| 285 | 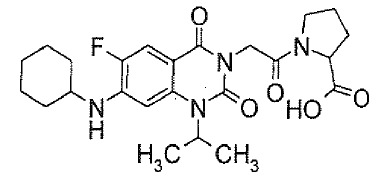 |

**[Table 57]**

| | |
|---|---|
| 286 | 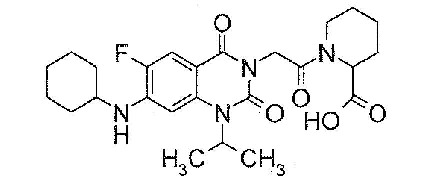 |
| 33 | 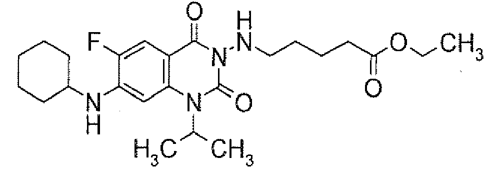 |
| 287 | 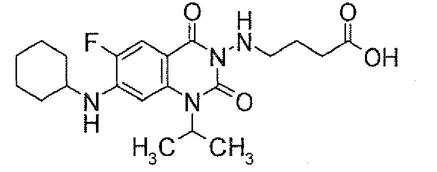 |
| 288 | 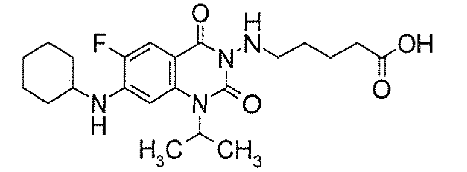 |
| 289 | 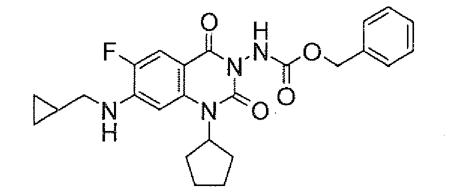 |
| 290 | 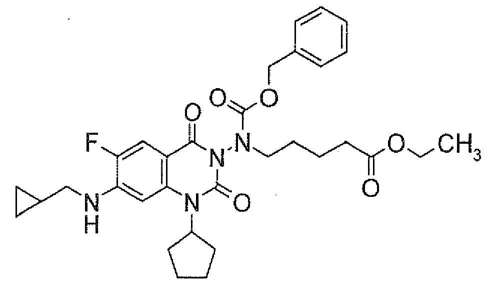 |

**[Table 58]**

| | |
|---|---|
| 291 | 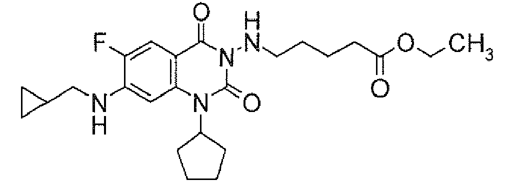 |
| 292 | 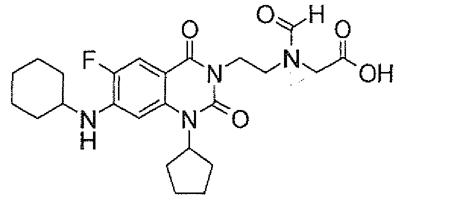 |
| 293 | 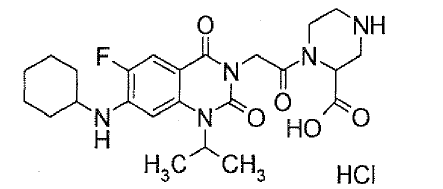 |
| 294 | 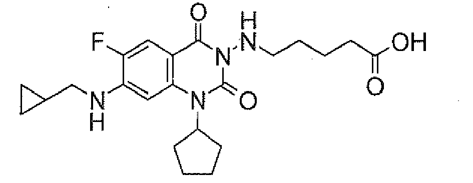 |
| 295 | 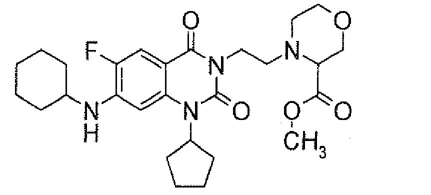 |
| 296 | 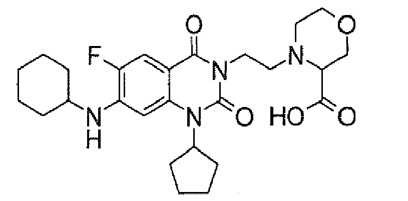 |

**[Table 59]**

| | |
|---|---|
| 297 | 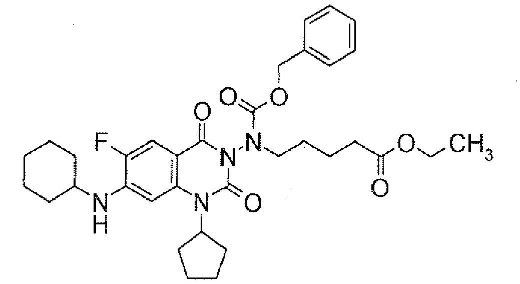 |
| 298 | 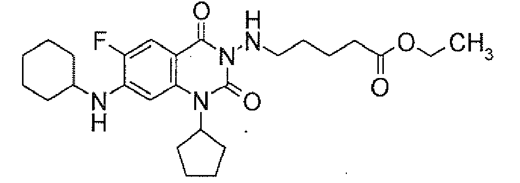 |
| 299 | 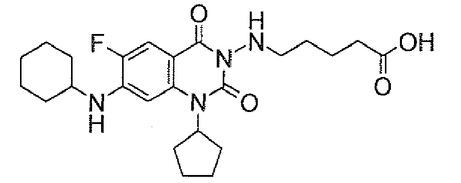 |
| 300 | 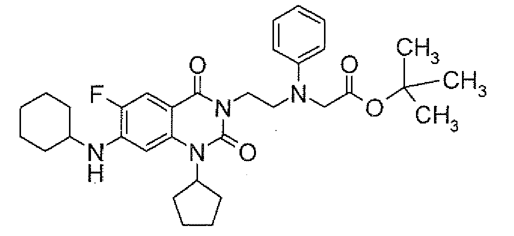 |
| 301 | 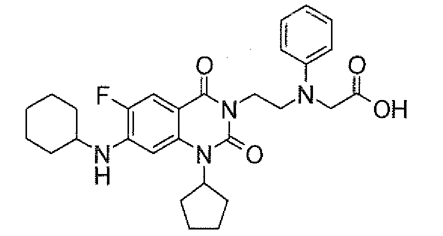 |

**[Table 60]**

| | |
|---|---|
| 302 | 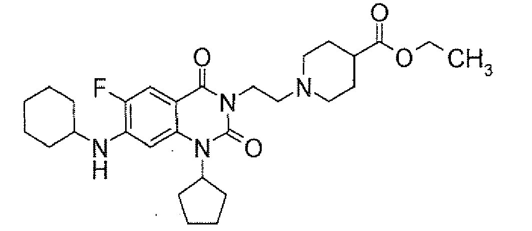 |
| 31 | 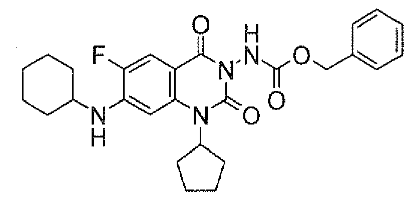 |
| 303 | 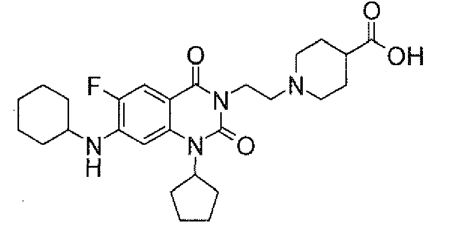 |
| 304 | 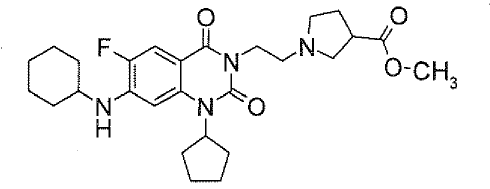 |
| 305 | 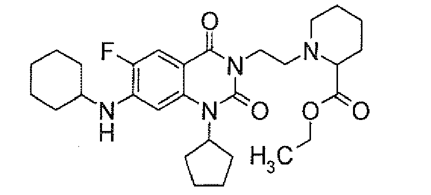 |
| 306 | 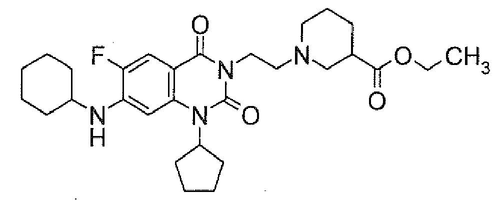 |

**[Table 61]**

| | |
|---|---|
| 307 | 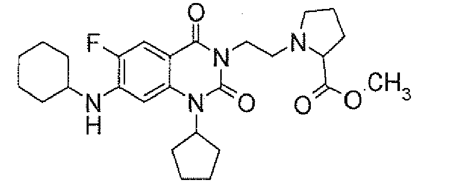 |
| 308 | 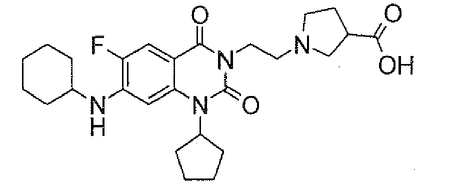 |
| 309 | 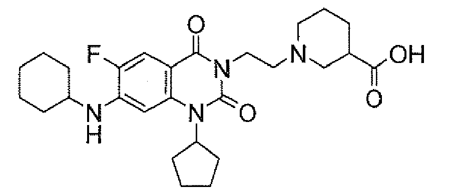 |
| 310 | 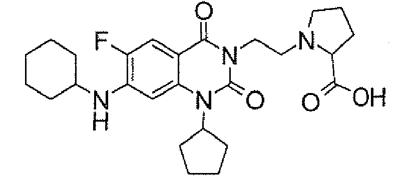 |
| 311 | 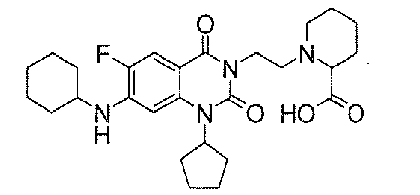 |
| 312 | 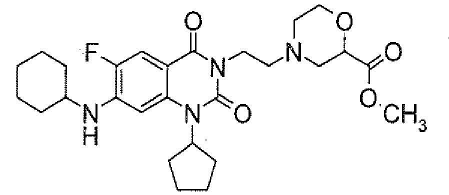 |

**[Table 62]**

| | |
|---|---|
| 313 | 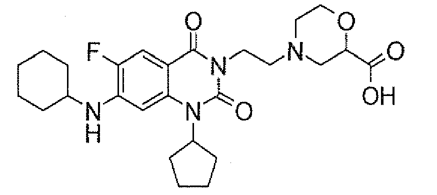 |
| 314 | 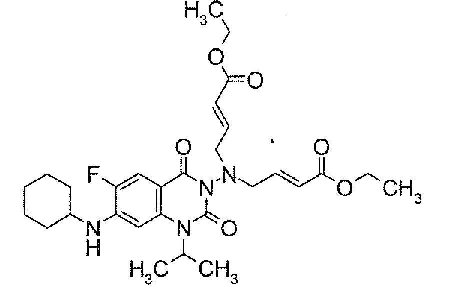 |
| 315 | 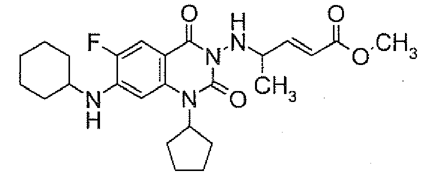 |
| 316 | 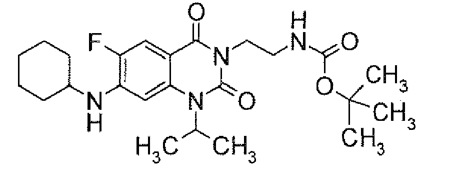 |

**[Table 63]**

| Ex | Syn | Data |
|---|---|---|
| 37 | 1 | FAB+: 385 |
| 38 | 1 | FAB+: 387 |
| 39 | 1 | FAB+: 389 |
| 1 | 1 | FAB+: 404 |
| 40 | P4 | FAB+: 432 |
| 41 | 1 | FAB+: 475 |
| 42 | 1 | FAB+: 461 NMR1: 1.10-1.25 (1H, m), 1.27-1.43 (4H, m), 1.60-1.81 (5H, m), 1.86-1.99 (6H,m), 2.01-2.13 (2H, m), 3.37-3.50 (1H, m), 3.77 (2H, d, J = 5.8 Hz), 4.53 (2H, s), 5.36 (1H, quintet, J = 8.7 Hz), 6.39 (1H, dd, J = 2.1, 8.0 Hz), 6.43 (1H, d, J = 7.1 Hz), 7.51 (1H, d, J = 11.6 Hz), 8.42 (1H, t, J = 5.7 Hz), 12.40-12.70 (1H, br) |
| 43 | P8 | FAB+: 503 |
| 44 | 1 | FAB+: 510 |
| 45 | P8 | FAB+: 489 |
| 46 | 1 | FAB+: 432 NMR1: 1.09-1.24 (1H, m), 1.27-1.43 (4H, m), 1.60-1.84 (7H, m), 1.86-2.00 (6H, m), 2.02-2.14 (2H, m), 2.23 (2H, t, J = 7.4 Hz), 3.36-3.47 (1H, m), 3.92 (2H, t, J = 6.9 Hz), 5.36 (1H, quintet, J = 8.7 Hz), 6.33 (1H, dd, J = 2.0, 8.0 Hz), 6.41 (1H, d, J = 7.1 Hz), 7.51 (1H, d, J=11.7 Hz), 12.02 (1H, brs) |
| 47 | 1 | FAB+: 418 |
| 48 | 1 | FAB+: 418 |
| 49 | P3, 1 | FAB+: 418 |
| 7 | 7 | FAB+: 390 |
| 50 | 1 | FAB+: 551 |
| 51 | 1 | FAB+: 489 |
| 52 | 1 | FAB+: 489 |
| 53 | P8 | ESI+: 491 |
| 54 | 1 | FAB+: 406 |
| 55 | 1 | ESI+:458 |
| 6 | 6 | ESI+:440 |
| 5 | 5 | FAB+: 420 |
| 56 | 5 | FAB+: 477 |
| 57 | P8 | FAB+: 491 |
| 58 | 1 | FAB+: 477 |
| 59 | P3 | FAB+: 432 |
| 60 | P3 | FAB+: 458 |
| 61 | P3 | FAB+: 432 |

**[Table 64]**

| | | |
|---|---|---|
| 62 | 26 | FAB+: 460 |
| 63 | 1 | FAB+: 404 |
| 64 | 26 | FAB+: 406 |
| 65 | 1 | FAB+: 430 NMR1: 1.10-1.22 (1H, m), 1.26-1.43 (4H, m), 1.60-1.82 (5H, m), 1.87-2.00 (6H, m), 2.02-2.14 (2H, m), 3.38-3.50 (1H, m), 4.64 (2H, d, J = 4.2 Hz), 5.31-5.44 (1H, m), 5.66 (1H, d, J = 15.9 Hz), 6.43 (2H, t, J = 6.7 Hz), 6.81 (1H, dt, Jd = 15.9 Hz, Jt = 4.9 Hz), 7.53 (1H, d, J = 11.7 Hz), 12.32 (1H, brs) |
| 66 | P8 | FAB+: 461 |
| 67 | P8 | FAB+: 503 |
| 68 | P8 | FAB+: 489 |
| 69 | 1 | FAB+: 376 NMR1: -0.04-0.03 (2H, m), 0.18-0.25 (2H, m), 0.77-0.89 (1H, m), 1.30-1.44 (2H, m), 1.57-1.70 (4H, m), 1.72-1.84 (2H, m), 2.89 (2H, t, J = 6.1 Hz), 4.25 (2H, s), 4.94 (1H, quintet, J = 8.4 Hz), 6.30 (1H, d, J = 7.1 Hz), 6.51-6.57 (1H, m), 7.23 (1H, d, J = 11.5 Hz), 12.62 (1H, brs) |
| 70 | 1 | FAB+: 446 |
| 71 | 1 | FAB+: 404 |
| 72 | 1 | FAB+: 446 |
| 73 | 1 | FAB+: 418 |
| 74 | 1 | FAB+: 420 |
| 75 | P8 | FAB+: 529 |
| 76 | 1 | FAB+: 432 |
| 77 | 1 | FAB+: 406 NMR1: 1.09-1.24 (1H, m), 1.26-1.46 (4H, m), 1.49 (6H, d, J = 6-8 Hz), 1.59-1.68 (1H, m), 1.72-1.83 (4H, m), 1.88-1.96 (2H, m), 2.22 (2H, t, J = 7.4 Hz), 3.43-3.55 (1H, m), 3.90 (2H, t, J = 6.9 Hz), 4.90-5.14 (1H, m), 6.30 (1H, d, J = 6.5 Hz), 6.54 (1H, d, J = 7.0 Hz), 7.50 (1H, d, J = 11.7 Hz), 12.00 (1H, brs) |
| 78 | 1 | FAB+: 378 |
| 8 | 8 | ESI+: 416 |
| 9 | 9 | ESI+: 486 |
| 79 | P8 | FAB+: 579 |
| 80 | P8 | FAB+: 517 |
| 81 | P8 | FAB+: 517 |
| 82 | P3 | FAB+: 496 |
| 83 | P4 | FAB+: 434 |
| 84 | P8 | ESI+: 517 |
| 4 | 4 | FAB+: 460 |
| 85 | P8 | ESI+: 505 |
| 19 | 19 | FAB+: 388 |

**[Table 65]**

| | | |
|---|---|---|
| 86 | P3 | FAB+: 460 |
| 87 | 4 | FAB+: 460 |
| 88 | P3 | FAB+: 404 |
| 89 | P3 | FAB+: 432 |
| 90 | P4 | FAB+: 474 |
| 91 | P4 | FAB+: 446 |
| 92 | P4 | FAB+: 448 |
| 93 | P4 | FAB+: 434 |
| 94 | P4 | FAB+: 406 |
| 95 | 26 | FAB+: 434 |
| 96 | 1 | ESI+; 412 |
| 97 | 26 | FAB+: 414 |
| 98 | P3 | FAB+: 432 |
| 99 | P3 | ESI+: 440 |
| 100 | P3 | FAB+: 489 |
| 101 | 1 | FAB+: 420 |
| 102 | 1 | FAB+: 446 |
| 103 | 1 | FAB+: 418 |
| 104 | I | FAB+: 448 |
| 105 | 1 | FAB+: 413 |
| 106 | 2 | FAB+: 443 |
| 107 | 1 | FAB+: 415 |
| 12 | 12 | FAB+: 567 |
| 108 | 1 | FAB+: 520 |
| 109 | 1 | FAB+: 525 |
| 110 | 1 | ESI+:444 |
| 111 | 1 | ESI+: 505 |
| 112 | 1 | FAB+: 447 |
| 113 | 27 | FAB+: 430 |
| 114 | 1 | FAB+: 446 NMR1: 1.10 (3H, d, J = 7.1 Hz), 1.12-1.43 (5H, m), 1.48-1.81 (6H, m), 1.83-1.99 (7H, m), 2.01-2.14 (2H, m), 2.27-2.39 (1H, m), 3.39-3.48 (1H, m), 3.84-3.99 (2H, m), 5.36 (1H, quintet, J = 8.7 Hz), 6.34 (1H, dd, J = 2.1, 8.0 Hz), 6.41 (1H, d, J = 7.1 Hz), 7.51 (1H, d, J = 11.7 Hz) |
| 18 | 18 | FAB+: 456 |
| 14 | 14 | FAB+: 444 |
| 15 | 15 | FAB+: 486 |
| 115 | 27 | FAB+: 404 |
| 116 | 1 | ESI+: 515 |
| 117 | P3 | FAB+: 485 |

**[Table 66]**

| | | |
|---|---|---|
| 118 | 1 | FAB+: 471 |
| 119 | 1 | FAB+: 487 |
| 120 | 1 | FAB+: 470 |
| 121 | 1 | FAB+: 480 |
| 122 | 27 | FAB+: 434 |
| 123 | 7 | FAB+: 404 |
| 124 | P4 | FAB+: 448 |
| 125 | P4 | ESI+: 548 |
| 126 | P8 | FAB+: 475 |
| 127 | P3 | FAB+: 474 |
| 128 | P3 | FAB+: 446 |
| 10 | 10 | FAB+: 472 |
| 11 | 11 | FAB+: 389 |
| 129 | 1 | FAB+: 519 |
| 130 | I | FAB+: 420 |
| 131 | 1 | FAB+: 434 |
| 132 | 4 | FAB+: 476 |
| 133 | 4,5 | ESI+: 448 |
| 134 | P3 | ESI+: 475 |
| 135 | P3 | ESI+: 561 |
| 136 | P3 | FAB+: 460 |
| 137 | P3 | FAB+: 494 |
| 138 | P8 | FAB+: 543 |
| 139 | P3 | FAB+: 498 |
| 140 | P3 | FAB+: 515 |
| 141 | P3 | FAB+: 494 |
| 142 | P3 | ESI+: 402 |
| 143 | 4 | FAB+: 524 |
| 144 | 1 | FAB+: 444 |
| 145 | 1 | FAB+: 378 |
| 146 | 1 | FAB+: 454 |
| 147 | 1 | FAB+: 446 |
| 148 | 14 | FAB+: 509 |
| 149 | 1 | FAB+: 420 |
| 150 | P8 | FAB+: 419 |
| 151 | P8 | FAB+: 575 |
| 152 | 1 | FAB+: 449 |
| 153 | 1 | FAB+: 510 |
| 26 | 26 | FAB+: 475 |
| 154 | 1 | FAB+: 434 |

**[Table 67]**

| | | |
|---|---|---|
| 155 | 1 | FAB+: 447 NMR1: 1.09-1.22 (1H, m), 1.25-1.43 (4H, m), 1.56-1.81 (7H, m), 1.86-2.00 (6H, m), 2.02-2.14 (2H, m), 2.35 (2H, t, J = 7.3 Hz), 2.80-2.88 (2H, m), 3.35-3.48 (1H, m), 5.34 (1H, quintet, J = 8.6 Hz), 5.72 (1H, brs), 6.36 (1H, d, J = 6.4 Hz), 6.42 (1H, d, J = 7.0 Hz), 7.52 (1H, d, J = 11.7 Hz), 11.99 (1H, brs) |
| 30 | 30 | ESI+: 489 |
| 156 | 1 | ESI+: 461 |
| 27 | 27 | FAB+: 547 |
| 157 | 1 | FAB+: 510 NMR1: 1.08-1.24 (1H, m), 1.27-1.43 (4H, m), 1.59-1.82 (5H, m), 1.87-1.98 (6H, m), 2.02-2.14 (2H, m), 3.37-3.47 (1H, m), 4.61 (2H, s), 5.04 (2H, s), 5.38 (1H, quintet, J = 8.7 Hz), 6.41 (1H, dd, J = 2.3, 8.2 Hz), 6.44 (1H, d, J = 7.2 Hz), 6.76 (1H, dd, J = 2.5, 8.1 Hz), 6.81 (1H, s), 6.84 (1H, d, J = 7.9 Hz), 7.20 (1H, t, J = 7.9 Hz), 7.54 (1H, d, J = 11.7 Hz), 12.97 (1H, brs) |
| 158 | P3 | FAB+: 448 |
| 159 | P3 | FAB+: 462 |
| 160 | 12 | FAB+: 551 |
| 161 | 1 | FAB+: 537 |
| 162 | P8 | FAB+: 418 |
| 20 | 20 | FAB+: 538 |
| 163 | P3 | FAB+: 406 |
| 164 | P3 | ESI+: 468 |
| 165 | P8 | FAB+: 477 |
| 166 | 4 | FAB+: 524 |
| 167 | 4 | FAB+: 538 |
| 168 | P8 | FAB+: 551 |
| 169 | 4 | FAB+: 444 |
| 170 | P3 | FAB+: 462 |
| 171 | P8 | FAB+: 637 |
| 172 | 1 | FAB+: 446 |
| 173 | 1 | FAB+: 462 |
| 174 | 15 | ESI-: 458 |
| 13 | 13 | FAB+: 447 |
| 175 | 1 | FAB+: 460 |
| 176 | 1 | FAB+: 448 |
| 177 | 1 | FAB+: 418 |
| 178 | 6 | FAB+: 442 |
| 179 | 1 | FAB+: 462 |
| 180 | 26 | FAB+: 489 |
| 16 | 16 | ESI+: 476 |

**[Table 68]**

| | | |
|---|---|---|
| 181 | P3 | FAB+: 498 |
| 182 | 1 | ESI+: 462 |
| 183 | 1 | FAB+: 461 |
| 184 | 4 | ESI+: 488 |
| 185 | 1 | ESI+: 448 NMR1: 1.10-1.43 (5H, m), 1.60-2.00 (13H, m), 2.02-2.14 (2H, m), 2.46 (2H, t, J = 7.3 Hz), 3.37-3.47 (1H, m), 4.05 (2H, t, J = 6.2 Hz), 5.32 (1H, quintet, J = 8.5 Hz), 6.41 (2H, d, J = 7.1 Hz), 7.52 (1H, d, J = 11.6 Hz), 12.09 (1H, s) |
| 186 | P4 | ESI+: 476 |
| 17 | 17 | ESI+: 490 |
| 187 | P3 | ESI+: 432 |
| 188 | 12 | ESI+: 531 |
| 189 | 16 | ESI+: 476 |
| 190 | 4 | FAB+: 458 |
| 191 | 6 | ESI+: 468 |
| 192 | 1 | ESI+: 461 |
| 193 | 1 | ESI+: 475 |
| 194 | 1 | ESI+: 489 |
| 21 | 21 | FAB+: 462 |
| 195 | 1 | FAB+: 501 |
| 196 | 1 | FAB+: 501 |
| 197 | 6 | ESI+: 575 |
| 198 | 1 | FAB+: 527 |
| 199 | 1 | ESI+: 475 |
| 200 | 1 | ESI+: 461 |
| 201 | 26 | FAB+: 503 |
| 202 | 26 | ESI+: 503 |
| 203 | 1 | ESI+: 489 |
| 204 | P8 | ESI+: 515 |
| 205 | P8 | ESI+: 529 |
| 206 | P8 | ESI-: 469 |
| 28 | 28 | ESI+: 520 |
| 29 | 29 | ESI+: 565 |
| 207 | 1 | ESI+: 537 |
| 24 | 24 | ESI+: 420 NMR1: 1.10-1.23 (1H,m), 1.26-1.43 (4H,m), 1.60-1.81 (5H, m), 1.86-2.00 (6H, m), 2.03-2.15 (2H, m), 3.39-3.50 (1H, m), 4.62 (2H, s), 5.30 (1H, quintet, J = 8.6 Hz), 6.41-6.48 (2H, m), 7.53 (1H, d, J = 11.5 Hz), 13.14 (1H, brs) |
| 208 | P8 | ESI+: 516 |
| 209 | P8 | FAB+: 499 |

**[Table 69]**

| | | |
|---|---|---|
| 210 | P8 | ESI+: 499 |
| 211 | P8 | ESI+: 500 |
| 212 | 1 | ESI+: 475 |
| 25 | 25 | ESI+: 489 |
| 213 | 1 | FAB+: 461 |
| 214 | P3 | FAB+: 524 |
| 215 | P3 | ESI+: 480 |
| 216 | P8 | ESI+: 503 |
| 217 | P8 | ESI+: 517 |
| 23 | 23 | ESI+: 489 |
| 218 | P8 | FAB+: 515 |
| 219 | P8 | FAB+: 515 |
| 220 | P8 | ESI+: 555 |
| 221 | P8 | ESI+: 603 |
| 222 | P8 | FAB+: 503 |
| 223 | P8 | ESI+: 371 |
| 224 | P9 | FAB+: 397 |
| 22 | 22 | FAB+: 517 |
| 225 | P3 | ESI+: 503 |
| 226 | P3 | ESI+: 489 |
| 227 | 11 | ESI+: 403 |
| 228 | 4 | ESI+: 503 |
| 229 | P3 | FAB+: 489 |
| 230 | P3,1 | ESI+: 510 |
| 231 | P3,1 | ESI+: 480 |
| 232 | P3,1 | ESI+: 480 |
| 233 | P3,1 | ESI+: 520 |
| 234 | P3,1 | ESI+: 481 |
| 235 | P3,1 | ESI+: 488 |
| 236 | P3,1 | ESI+:448 |
| 237 | P3,1 | ESI+: 430 |
| 238 | P3,1 | ESI+: 474 |
| 239 | P4 | FAB+: 413 |
| 2 | 2 | FAB+: 415 |
| 3 | 3 | FAB+: 417 |
| 240 | P4 | FAB+: 538 |
| 241 | P4 | FAB+: 460 |
| 242 | P3 | FAB+: 432 |
| 243 | P4 | FAB+: 441 |
| 244 | P3 | FAB+: 413 |

**[Table 70]**

| | | |
|---|---|---|
| 245 | P3 | FAB+: 473 |
| 246 | P3 | FAB+: 434 |
| 247 | P4 | FAB+: 474 |
| 248 | P3 | FAB+: 487 |
| 249 | P3 | FAB+: 487 |
| 250 | 26 | FAB+: 489 |
| 251 | 1 | FAB+: 461 |
| 252 | P3 | ESI+: 463 |
| 253 | 11,28 | ESI+: 494 |
| 254 | 26,1 | FAB+: 507 |
| 255 | P3 | ESI+: 533 |
| 256 | 1 | ESI+: 505 NMR1: 1.10-1.43 (5H, m), 1.60-1.81 (5H,m), 1.86-1.99 (6H, m), 2.02-2.14 (2H, m), 2.75-2.83 (4H, m), 3.13 (3H, s), 3.94 (2H, t, J = 6.7 Hz), 5.36 (1H, quintet, J = 8.6 Hz), 6.33 (1H, dd, J = 2.3, 8.1 Hz), 6.41 (1H, d, J = 7.1 Hz), 7.51 (1H, d, J = 11.7 Hz) |
| 257 | P7 | ESI+: 531 |
| 34 | 34 | ESI+: 475 |
| 36 | 36 | ESI+: 363 |
| 258 | P3 | ESI+: 520 |
| 259 | 1 | FAB+: 506 |
| 260 | P3 | ESI+: 517 |
| 261 | P3 | ESI+: 445 |
| 262 | 2 | ESI+: 508 |
| 35 | 35 | ESI+: 533 |
| 263 | 28 | ESI+: 553 |
| 264 | P8 | ESI+: 503 |
| 265 | 1 | ESI+: 489 |
| 266 | 26 | ESI+: 447 |
| 267 | 26 | FAB+: 475 |
| 268 | 1 | ESI+: 525 |
| 269 | P3 | ESI+: 447 |
| 270 | 1 | ESI+: 505 |
| 271 | 1 | FAB+: 461 |

**[Table 71]**

| | | |
|---|---|---|
| 272 | 1 | ESI+: 419 NMR1: 0.25-0.30 (2H, m), 0.46-0.52 (2H,m), 1.06-1.16 (1H, m), 1.56-1.72 (4H, m), 1.87-2.00 (4H, m), 2.03-2.15 (2H, m), 2.35 (2H, t, J = 7.3 Hz), 2.80-2.88 (2H, m), 3.16 (2H, t, J = 6.1 Hz), 5.21 (1H, quintet, J = 8.3 Hz), 5.68-5.76 (1H, m), 6.55 (1H, d, J = 7.0 Hz), 6.71-6.77 (1H, m), 7.52 (1H, d, J =11.6 Hz), 11.99 (1H, brs) |
| 273 | 32 | ESI+: 637 |
| 274 | 33 | East+: 503 |
| 275 | P8 | ESI+: 505 |
| 276 | P8 | ESI+: 604 |
| 277 | P8 | ESI+: 489 |
| 278 | P8 | ESI+: 517 |
| 279 | 1 | ESI+: 475 |
| 280 | P3,1 | FAB+: 404 |
| 281 | 31 | ESI+: 469 |
| 32 | 32 | ESI+: 597 |
| 282 | 26 | ESI+: 449 |
| 283 | 1 | ESI+: 491 |
| 284 | 1 | ESI+: 590 |
| 285 | 1 | ESI+: 475 |
| 286 | 1 | ESI+: 489 |
| 33 | 33 | ESI+: 463 |
| 287 | 1 | ESI+: 421 NMR1: 1.10-1.22 (1H, m), 1.25-1.47 (4H, m), 1.50 (6H, d, J = 6.8 Hz), 1.57-1.67 (3H, m), 1.71-1.79 (2H, m), 1.88-1.95 (2H, m), 2.35 (2H, t, J = 7.3 Hz), 2.80-2.88 (2H, m), 3.44-3.55 (1H, m), 4.95-5.07 (1H, m), 5.65-5.74 (1H, m), 6.30 (1H, d, J = 7.1 Hz), 6.55 (1H, d, J = 6.9 Hz), 7.50 (1H, d, J = 11.7 Hz), 11.97 (1H, brs) |
| 288 | 1 | ESI+; 435 |
| 289 | 31 | ESI+: 467 |
| 290 | 32 | ESI+: 595 |
| 291 | 33 | ESI+: 461 |
| 292 | 1 | ESI+: 475 |
| 293 | 36 | ESI+: 490 |

**[Table 72]**

| | | |
|---|---|---|
| 294 | 1 | ESI+: 433 NMR1: 0.24-0.30 (2H, m), 0.46-0.52 (2H, m), 1.06-1.14 (1H, m), 1.43 (2H, quintet, J = 7.1 Hz), 1.57 (2H, quintet, J = 7.5 Hz), 1.62-1.72 (2H, m), 1.87-1.98 (4H, m), 2.03-2.14 (2H, m), 2.23 (2H, t, J = 7.3 Hz), 2.78-2.86 (2H, m), 3.16 (2H, t, J = 6.0 Hz), 5.21 (1H, quintet, J = 8.3 Hz), 5.59-5.68 (1H, m), 6.55 (1H, d, J = 7.0 Hz), 6.69-6.75 (1H, m), 7.52 (1H, d, J = 11.6 Hz), 11.96 (1H, s) |
| 295 | P7 | ESI+: 517 |
| 296 | 1 | ESI+: 503 |
| 297 | 32 | ESI+: 623 |
| 298 | 33 | ESI+: 489 |
| 299 | 1 | ESI+: 461 |
| 300 | P3 | ESI+: 579 |
| 301 | 34 | ESI+: 523 |
| 302 | P7 | ESI+: 529 |
| 31 | 31 | ESI+: 495 |
| 303 | 1 | FAB+: 501 |
| 304 | P7 | ESI+: 501 |
| 305 | P7 | ESI+: 529 |
| 306 | P7 | ESI+: 529 |
| 307 | P7 | ESI+: 501 |
| 308 | 1 | ESI+: 487 |
| 309 | 1 | ESI+: 501 |
| 310 | 1 | ESI+: 487 |
| 311 | 1 | FAB+: 501 |
| 312 | P7 | ESI+: 517 |
| 313 | 1 | FAB+: 503 |
| 314 | P3 | FAB+: 559 |
| 315 | P3 | FAB+: 473 |
| 316 | P3 | ESI+: 463 |

**[Table 73]**

| No | Structure |
|---|---|
| 1 | 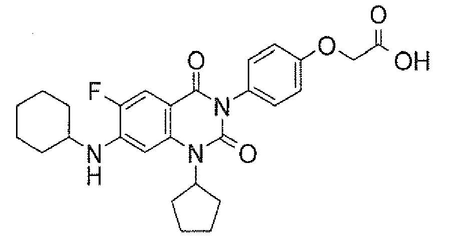 |
| 2 | 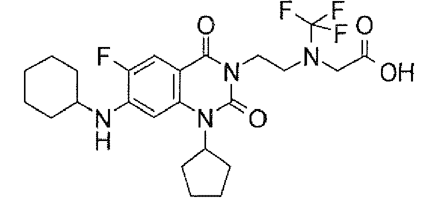 |
| 3 | 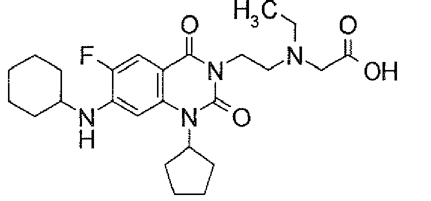 |
| 4 | 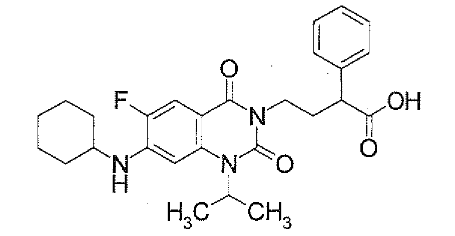 |
| 5 | 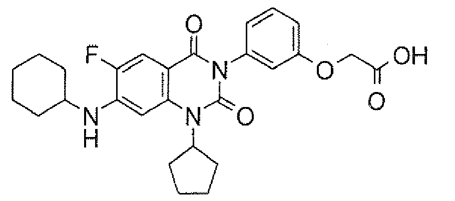 |

**[Table 74]**

| | |
|---|---|
| 6 | 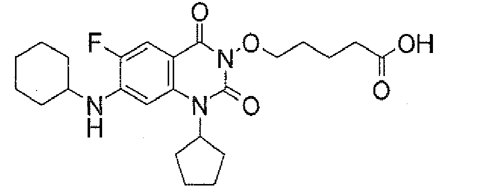 |
| 7 | 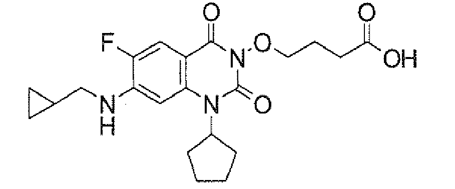 |
| 8 | 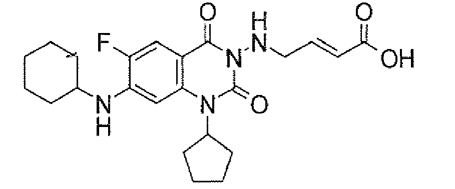 |
| 9 | 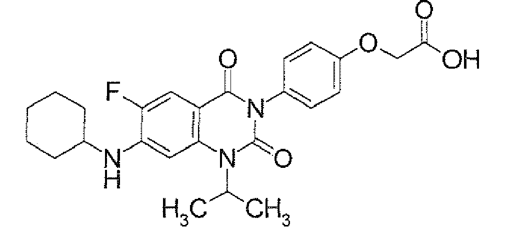 |
| 10 | 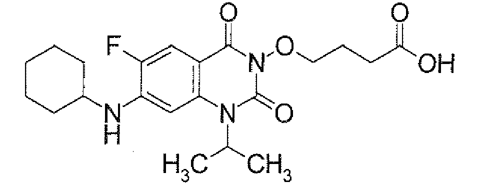 |
| 11 | 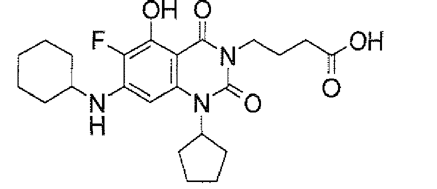 |

**[Table 75]**

| | |
|---|---|
| 12 | 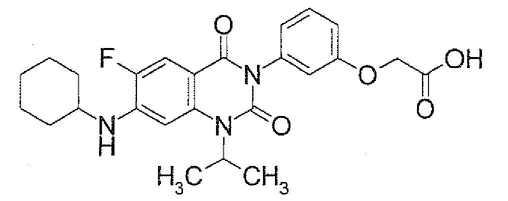 |
| 13 | 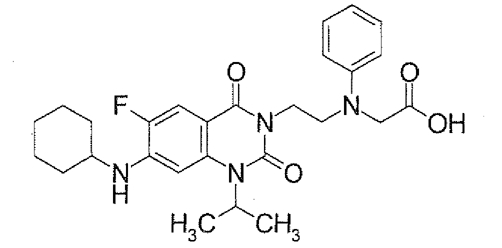 |
| 14 | 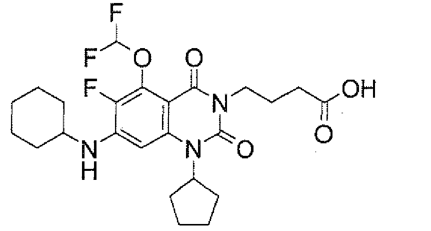 |

### Industrial Applicability

Since a compound of the present invention exhibits an excellent P2Y12 inhibitory action, it is useful as a medical drug, particularly, as a platelet aggregation inhibitor.

## Claims

1. A bicyclic heterocyclic compound presented by the formula (I) or a pharmaceutically acceptable salt thereof: 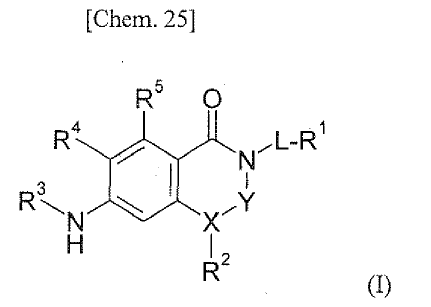 (wherein, symbols indicate the following meanings:
X: C(R⁶) or N;
Y: (i) CH(R⁷) when X is C(R⁶), and (ii) C(O) or *-C(O)-CH₂- when X is N, wherein * represents a bond to X;
R⁶ and R⁷ indicate H, or
R⁶ and R⁷ may form a bond together;
R¹: lower alkyl, halogeno-lower alkyl, lower alkylene-R¹⁰, lower alkenylene-R¹⁰, aryl, or a heterocyclic group, in which lower alkylene, lower alkenylene, aryl, and the heterocyclic group may be substituted;
L: a single bond, -O-, -N(R¹¹)-, -N(R¹¹)C(O)-*, or -N(R¹¹)C(O)O-*, wherein * represents a bond to R¹;
R¹⁰: -OR¹¹, -CN, -C(O)R¹¹, -CO₂R⁰, -CO₂-lower alkylene-aryl, -C(O)N(R¹¹)₂, -C(O)N(R⁰)-S(O)₂-R¹¹, -C(O)N(R⁰)-OR⁰, -C(O)N(R⁰)O-heterocyclic group, -C(O)N(R⁰)N(R⁰)₂, -N(R¹¹)₂, -N(R¹¹)C(O)R¹¹, -N(R¹¹)-CO₂R⁰, -N(R⁰)C(O)CO₂R⁰, -N(R¹¹)-S(O)₂-R¹¹, -N(R¹¹)C(S)S-R⁰, -P(O)(OR⁰)₂, aryl, or a heterocyclic group, in which aryl and the heterocyclic group may be substituted;
R⁰: the same with or different from each other, and -H or lower alkyl;
R¹¹: the same with or different from each other, and -H, lower alkyl, halogeno-lower alkyl, lower alkenyl, cycloalkyl, cycloalkenyl, aryl, heterocyclic group, lower alkylene-OR⁰, lower alkylene-CO₂R⁰, lower alkylene-CO₂-lower alkylene-aryl, lower alkylene-aryl, lower alkylene-heterocyclic group, lower alkylene-OC(O)R⁰, lower alkylene-P(O)(OR⁰)₂, lower alkylene-O-lower alkylene-aryl, lower alkenylene-OR⁰, lower alkenylene-CO₂R⁰, lower alkenylene-aryl, lower alkenylene-heterocyclic group, or lower alkenylene-P(O)(OR⁰)₂, in which lower alkylene, lower alkenylene, cycloalkyl, cycloalkenyl, aryl, and heterocyclic group may be substituted;
R²: lower alkyl, cycloalkyl, cycloalkenyl, or a heterocyclic group;
R³: lower alkyl, cycloalkyl, or lower alkylene-cycloalkyl;
R⁴: -H or halogen;
R⁵: -H, halogen, -OR⁰, -O-halogeno-lower alkyl, or -O-lower alkylene-aryl, wherein, N-(2,6-dichloro benzoyl)-4-[7-(ethylamino)-1-methyl-2,4-dioxo-1,4-dihydroquinazolin-3(2H)-yl]-L-phenylalanine and 3-(3-chlorophenyl)-7-(isobutylamino)-1-methylquinazoline-2,4(1H,3H)-dione are excluded).

2. The compound according to Claim 1, wherein X is N, and Y is C(O).

3. The compound according to Claim 2, wherein R³ is cycloalkyl or lower alkylene-cycloalkyl.

4. The compound according to Claim 3, wherein R⁴ is -F.

5. The compound according to Claim 4, wherein R⁵ is -H.

6. The compound according to Claim 5, wherein R² is lower alkyl or cycloalkyl.

7. The compound according to Claim 6, wherein L is a single bond, -O-, or -NH-.

8. The compound according to Claim 7,
wherein R¹ is lower alkylene-CO₂R⁰, lower alkenylene-CO₂R⁰, lower alkylene-N(R⁰)-lower alkylene-CO₂R⁰, lower alkylene-N(lower alkylene-OR⁰)-lower alkylene-CO₂R⁰, lower alkylene-C(O)N(R⁰)-lower alkylene-CO₂R⁰, or lower alkylene-(heterocyclic group substituted with -CO₂R⁰).

9. The compound according to Claim 1, wherein X is C(R⁶), and Y is CH(R⁷).

10. The compound according to Claim 1, wherein X is N, and Y is *-C(O)-CH₂-(wherein, * represents a bond to X).

11. The compound or a pharmaceutically acceptable salt thereof according to Claim 1, which is selected from the group consisting of 4-[7-(cyclohexylamino)-1-cyclopentyl-6-fluoro-2,4-dioxo-1,4-dihydroquinazolin-3(2H)-yl]butanoic acid; 4-[7-(cyclohexylamino)-1-cyclopentyl-6-fluoro-2,4-dioxo-1,4-dihydroquinazolin-3(2H)-yl]-2-methylbutanoic acid; 4-{[7-(cyclohexylamino)-1-cyclopentyl-6-fluoro-2,4-dioxo-1,4-dihydroquinazolin-3(2H)-yl]amino}butanoic acid; 4-{[7-(cyclohexylamino)-1-cyclopentyl-6-fluoro-2,4-dioxo-1,4-dihydroquinazolin-3(2H)-yl]oxy}butanoic acid; [{2-[7-(cyclohexylamino)-1-cyclopentyl-6-fluoro-2,4-dioxo-1,4-dihydroquinazolin-3(2H)-yl]ethyl}(2-methoxyethyl)amino]acetic acid; 4-({1-cyclopentyl-7-[(cyclopropylmethyl)amino]-6-fluoro-2,4-dioxo-1,4-dihydroquinazolin-3(2H)-yl} amino)butanoic acid; 4-{{[7-(cyclohexylamino)-6-fluoro-1-isopropyl-2,4-dioxo-1,4-dihydroquinazolin-3(2H)-yl)amino}butanoic acid; 5-({1-cyclopentyl-7-(cyclopropylmethyl)amino]-6-fluoro-2,4-dioxo-1,4-dihydroquinazolin-3(2H)-yl}amino)pentanoic acid; 1-{2-[7-(cyclohexylamino)-1-cyclopentyl-6-fluoro-2,4-dioxo-1,4-dihydroquinazolin-3(2H)-yl]ethyl}pipendine-3-carboxylic acid; (2E)-4-[7-(cyclohexylamino)-1-cyclopentyl-6-fluoro-2,4-dioxo-1,4-dihydroquinazolin-3(2H)-yl]-2-butenoic acid; and {[7-(cyclohexylamino)-1-cyclopentyl-6-fluoro-2,4-dioxo-1,4-dihydroquinazolin-3(2H)-yl]oxy} acetic acid.

12. A pharmaceutical composition comprising the compound or a pharmaceutically acceptable salt thereof according to Claim 1, and a pharmaceutically acceptable carrier.

13. The pharmaceutical composition according to Claim 12, which is a platelet aggregation inhibitor.

14. The pharmaceutical composition according to Claim 12, which is a P2Y12 inhibitor.

15. Use of the compound or a pharmaceutically acceptable salt thereof according to Claim 1 for the manufacture of a platelet aggregation inhibitor or a P2Y12 inhibitor.
